# EUROPEAN PATENT APPLICATION

(11) **EP 1 627 927 A2**
(43) Date of publication of application: **22.02.2006**
(21) Application number: 05024557.0
(22) Date of filing: 01.03.2002
(51) Int. Cl.: C12Q 1/68

(54) **Methods, systems and computer program products for determining the biological effect and/or activity of drugs, chemical substances and/or pharmaceutical compositions based on their effect on the methylation status of the DNA**

(30) Priority: 01.03.2001 US 272484 P
(62) Divisional of application: 02726125.4
(71) Applicant: Epigenomics AG, 10178 Berlin (DE)
(72) Inventor: The designation of the inventor has not yet been filed
(74) Representative: Krauss, Jan

(57) **Abstract**

This invention is related to methods, systems and computer program products for determining the biological effect and/or activity of drugs, chemical substances and/or pharmaceutical compositions using their effect on DNA-methylation as a marker for their biological effect(s). The invention is further related to the use of the inventive methods, systems and computer program products in obtaining new biologically active compounds which can be used as so-called "lead"-compounds for new and effective medicaments and treatment strategies of, in particular, human, diseases.

## Description

### FIELD OF THE INVENTION

This invention is related to methods, systems and computer program products for determining the biological effect and/or activity of drugs, chemical substances and/or pharmaceutical compositions using their effect on DNA-methylation as a marker for their biological effect(s). The invention is further related to the use of the inventive methods, systems and computer program products in obtaining new biologically active compounds which can be used as so-called "lead"-compounds for new and effective medicaments and treatment strategies of, in particular, human, diseases.

### BACKGROUND OF THE INVENTION

### 1. The constant need for new compounds and biologically active compounds for the development of new pharmaceutics and medicaments

The advance in medical research constantly leads to the discovery of yet unknown and complex diseases, for which new, specific and effective pharmaceuticals and treatments have to be developed. In a majority of such new cases, nothing is known about biological compounds which would/could be effective in treating such diseases.

In general, time plays an important role in these cases, since in most of the cases an effective drug/treatment has to be found very rapidly.

Furthermore, such developments currently involve very cost-intensive screening procedures until a particularly suited compound (often called "lead"-compound) is found which could then serve as a chemical basis for an effective treatment.

Another current development in the treatment of diseases is the so-called "personalized" treatment, in which an individually treatment schedule and/or pharmaceutical composition is applied to the individual patient. Since the treatment is directed or applied to a very limited scope and number of patients (i.e. only one patient) and diseases, such treatment again is very cost-intensive and therefore can only seldom be applied in an efficient manner.

Furthermore, problems arise with already known biological compounds in such a way that a) unwanted side effects are discovered, that limit the use of established pharmaceutics, and b) resistance can be found/are developed against major therapeutics (like in the case of antibiotic resistances) which limit the success of presently applied compounds.

In view of the above, there exists a constant need for new potential candidate compounds for the treatment of emerging new diseases, personalised medicine and, of course, alternative treatments for already known diseases. Furthermore, the need exists for a reliable, cost-effective, fast and automateable method for screening such new effective compounds.

### 2. Screening for new biologically active compounds using "combinatorial chemistry"

The method of combinatorial chemistry is described as a profound change in the strategies that biotechnology-based industries are deploying in the search for exploitable biology and to discover new products and develop new or improved processes. (see, for example, Bull AT, et al. "Search and discovery strategies for biotechnology: the paradigm shift." Microbiol Mol Biol Rev 2000 Sep;64(3):573-606)

In general, combinatorial chemistry involves screening of a specific (or a set of specific) compound with a vast number of potential biological candidate substances (for example, proteins) that might interact with the compound. Interacting partners are selected and used for further screening. Initially screened and isolated compounds can be used as "lead"-compounds for the development of biologically active compounds useful for treatment of diseases.

Other methods and devices for combinatorial chemistry are described in, for example, US 6,175,816 (Flavin, et al.; "Use of automated technology in chemical process research and development") US 6,045,755 (Lebl, et al.; "Apparatus and method for combinatorial chemistry synthesis") US 5,880,972 (Horlbeck; "Method and apparatus for generating and representing combinatorial chemistry libraries") and US 5,721,099 (Still, et al.; "Complex combinatorial chemical libraries encoded with tags").

WO 00/71742 describes the "marriage" of solid-state electronics and neuronal function to create a new high-throughput electrophysiological assay to determine a compound's acute and chronic effect on cellular function. Electronics, surface chemistry, biotechnology, and fundamental neuroscience are integrated to provide an assay where the reporter element is an array of electrically active cells. This innovative technology was applied to neurotoxicity, and to screening compounds from combinatorial chemistry, gene function analysis, and basic neuroscience applications. Further disclosed are algorithms to analyze the action potential peak shape differences to indicate the pathway(s) affected by the presence of a new drug or compound; from that, aspects of its function in that cell are deduced. This observation is said to be exploited to determine the functional category of biochemical action of an unknown compound.

WO 00/23458 describes templated combinatorial chemical libraries comprised of a plurality of bifunctional molecules having both a chemical compound and a nucleic acid tag that defines the structure of the chemical compound and directs its synthesis.

Logani S, et al. ("Actions of Ginkgo Biloba related to potential utility for the treatment of conditions involving cerebral hypoxia." Life Sci 2000 Aug 11;67(12):1389-96) describe the use of HTS (high-throughput screening) libraries for reevaluation of the pharmacologic properties of substances such as extract from the leaves of Ginkgo biloba Linne (form. Salisburia adiantifolia Sm.).

Although the method of combinatorial chemistry exhibits several advantages in comparison to conventional methods for screening for biologically effective compounds which are useful for the development of new medicaments, there are still several drawbacks associated with this method.

The screening of a combinatorial chemistry library involves a screening for a multitude of different possible reactions and/or interactions of the compounds to be analysed with the interacting partners. Therefore, the reaction conditions are assumed crucial for the result of the screening. In particular, a compound which shows an interaction with a target in such a combinatorial assay in vitro might exhibit completely different reaction conditions in vivo which makes prediction of an effective compound very difficult and unreliable. As a result, an interaction in an in vitro combinatorial chemistry screening assay can always only give a hint for a potential biological function of the screened compound in vivo.

As a result, combinatorial chemistry screening involves a necessary second step; once a potential target/lead compound has been identified/found, the biological effect still has to be confirmed/determined in an in vivo context. This makes compound identification using this method unpredictable, slow and costly.

### 3. Methylation pattern and diseases

### 3.1 State of the art in methylation analysis

The modification of the genomic base cytosine to 5'-methylcytosine represents the epigenetic parameter which to date is the most important one and has been best examined. Nevertheless, methods exist today to determine comprehensive genotypes of cells and individuals, but no comparable methods exist to date to generate and evaluate epigenotypic information on a large scale.

In principle, there are three methods that differ in principle for determining the 5-methyl state of a cytosine in the sequence context.

The first method is based in principle on the use of restriction endonucleases (RE), which are methylation-sensitive". REs are characterized in that they produce a cut in the DNA at a certain DNA sequence which is usually 4-8 bases long. The position of such cuts can be detected by gel electrophoresis, transfer to a membrane and hybridization. Methylation-sensitive means that certain bases within the recognition sequence must be unmethylated for the step to occur. The band pattern after a restriction cut and gel electrophoresis thus changes depending on the methylation pattern of the DNA. However, most CpG that can be methylated are outside of the recognition sequences of REs, and thus cannot be examined.

The sensitivity of this method is extremely low (Bird, A. P., Southern, E. M., J. Mol. Biol. 118, 27-47). A variant combines PCR with this method; an amplification by two primers located on both sides of the recognition sequence occurs after a cut only if the recognition sequence is in the methylated form. In this case, the sensitivity theoretically increases to a single molecule of the target sequence; however, only individual positions can be examined, at great cost (Shemer, R. et al., PNAS 93, 6371-6376).

The second variant is based on the partial chemical cleavage of whole DNA, using the model of a Maxam-Gilbert sequencing reaction, ligation of adaptors to the ends thus generated, amplification with generic primers, and separation by gel electrophoresis. Using this method, defined regions having a size of less than thousands of base pairs can be examined. However, the method is so complicated and unreliable that it is practically no longer used (Ward, C, et al., J. Biol. Chem. 265, 3030-3033).

A new method for the examination of DNA to determine the presence of 5-methylcytosine is based on the specific reaction of bisulfite with cytosine. The latter is converted under appropriate conditions into uracil, which, as far as base pairing is concerned, is equivalent to thymidine, and which also corresponds to another base. 5-Methylcytosine is not modified. As a result, the original DNA is converted in such a manner that methylcytosine, which originally could not be distinguished from cytosine by its hybridisation behaviour, now can be detected by "normal" molecular biological techniques. All of these techniques are based on base pairing, which can now be completely exploited. The state of the art, as far as sensitivity is concerned, is defined by a method which includes the DNA to be examined in an agarose matrix, intended to prevent the diffusion and renaturing of the DNA (bisulfite reacts only with single-stranded DNA) and to replace all precipitation and purification steps by rapid dialysis (Olek, A., et al., Nucl. Acids. Res. 24, 5064-5066). Using this method, individual cells can be examined, which illustrates the potential of the method. However, so far only individual regions up to approximately 3000 base pairs in length have been examined, and an overall examination of cells to identify thousands of possible methylation events is not possible. However, this method is not capable of reliably analyzing minute fragments from small sample quantities. In spite of protection against diffusion, such samples are lost through the matrix.

### 3.2 State of the art in the use of the bisulfite technique

To date, barring few exceptions, (for example, Zeschnigk, M. et al., Eur. J. Hum. Gen. 5, 94-98; Kubota, T. et al., Nat. Genet. 16, 16-17), the bisulfite technique is only used in research. However, short specific pieces of a known gene after bisulfite treatment are routinely amplified and either completely sequenced (Olek, A. and Walter, J., Nat. Genet. 17, 275-276) or the presence of individual cytosine positions is detected by a "primer extension reaction" (Gonzalgo, M. L. and Jones, P. A., Nucl. Acids. Res. 25, 2529-2531), or enzyme cut (Xiong, Z. and Laird, P. W., Nucl. Acids. Res. 25, 2532-2534). All these references are from the year 1997. The concept of using complex methylation patterns for correlation with phenotypic data pertaining to complex genetic diseases, much less via an evaluation algorithm such as, for example, a neural network, has, so far, gone unmentioned in the literature; moreover, it cannot be performed according to the methodologies of the state of the art.

### 3.3 State of the art with respect to methylation and the diagnosis of human diseases

In the past, modification of the methylation pattern was analysed in order to study and understand the genetic mechanisms which are involved in the outbreak or the progression of a disease. All this research was done in a piece-by-piece fashion by studying only one gene/chromosomal region at a time and no diagnosis/therapeutic treatment regimen was based on the methylation pattern modifications. In fact, the type of disease associated with the modification of the methylation pattern was known before methylation analysis was performed. Therefore, the following publications only indicate the widespread connection between modifications of the methylation patterns and human diseases. Modifications can include both hyper- or hypomethylation of selected sites of the DNA.

Disease associated with a modification of the methylation patterns are, for example:
- Leukemia (Aoki E et al. "Methylation status of the p15INK4B gene in hematopoietic progenitors and peripheral blood cells in myelodysplastic syndromes" Leukemia 2000 Apr;14(4):586-93; Nosaka K et al. "Increasing methylation of the CDKN2A gene is associated with the progression of adult T-cell leukemia" Cancer Res 2000 Feb 15;60(4):1043-8; Asimakopoulos FA et al. "ABL1 methylation is a distinct molecular event associated with clonal evolution of chronic myeloid leukemia" Blood 1999 Oct 1;94(7):2452-60; Fajkusova L. et al. "Detailed Mapping of Methylcytosine Positions at the CpG Island Surrounding the Pa Promoter at the bcr-abl Locus in CML Patients and in Two Cell Lines, K562 and BV173" Blood Cells Mol Dis 2000 Jun;26(3):193-204; Litz CE et al. "Methylation status of the major breakpoint cluster region in Philadelphia chromosome negative leukemias" Leukemia 1992 Jan;6(1):35-41)
- Head and neck cancer (Sanchez-Cespedes M et al. "Gene promoter hypermethylation in tumors and serum of head and neck cancer patients" Cancer Res 2000 Feb 15;60(4):892-5)
- Hodgkin's disease (Garcia JF et al. "Loss of p16 protein expression associated with methylation of the p16INK4A gene is a frequent finding in Hodgkin's disease" Lab Invest 1999 Dec;79(12):1453-9)
- Gastric cancer (Yanagisawa Y et al. "Methylation of the hMLH1 promoter in familial gastric cancer with microsatellite instability" Int J Cancer 2000 Jan 1;85(1):50-3)
- Prostate cancer (Rennie PS et al. "Epigenetic mechanisms for progression of prostate cancer" Cancer Metastasis Rev 1998-99;17(4):401-9)
- Renal cancer (Clifford SC et al. "Inactivation of the von Hippel-Lindau (VHL) tumor suppressor gene and allelic losses at chromosome arm 3p in primary renal cell carcinoma: evidence for a VHL-independent pathway in clear cell renal tumourigenesis" Genes Chromosomes Cancer 1998 Jul;22(3):200-9)
- Bladder cancer (Sardi I et al. "Molecular genetic alterations of c-myc oncogene in superficial and locally advanced bladder cancer" Eur Urol 1998;33(4):424-30)
- Breast cancer (Mancini DN et al. "CpG methylation within the 5' regulatory region of the BRCA1 gene is tumor specific and includes a putative CREB binding site" Oncogene 1998 Mar 5;16(9):1161-9; Zrihan-Licht S et al. "DNA methylation status of the MUC1 gene coding for a breast-cancer-associated protein" Int J Cancer 1995 Jul 28;62(3):245-51; Kass DH et al. "Examination of DNA methylation of chromosomal hot spots associated with breast cancer" Anticancer Res 1993 Sep-Oct;13(5A):1245-51)
- Burkitt's lymphoma (Tao Q et al. "Epstein-Barr virus (EBV) in endemic Burkitt's lymphoma: molecular analysis of primary tumor tissue" Blood 1998 Feb 15;91(4):1373-81)
- Wilms tumor (Kleymenova EV et al. "Identification of a tumor-specific methylation site in the Wilms tumor suppressor gene" Oncogene 1998 Feb 12;16(6):713-20)
- Prader-Willi/Angelman syndrome (Zeschnigh et al. "Imprinted segments in the human genome: different DNA methylation patterns in the Prader-Willi/Angelman syndrome region as determined by the genomic sequencing method" Human Mol. Genetics (1997) (6)3 pp 387-395; Fang P et al. "The spectrum of mutations in UBE3A causing Angelman syndrome" Hum Mol Genet 1999 Jan;8(1):129-35)
- ICF syndrome (Tuck-Muller et al. "CMDNA hypomethylation and unusual chromosome instability in cell lines from ICF syndrome patients" Cytogenet Cell Genet 2000;89(1-2):121-8)
- Dermatofibroma (Chen TC et al. "Dermatofibroma is a clonal proliferative disease" J Cutan Pathol 2000 Jan;27(1):36-9)
- Hypertension (Lee SD et al. "Monoclonal endothelial cell proliferation is present in primary but not secondary pulmonary hypertension" J Clin Invest 1998 Mar 1;101(5):927-34)
- Pediatric Neurobiology (Campos-Castello J et al. "The phenomenon of genomic "imprinting" and its implications in clinical neuropediatrics" Rev Neurol 1999 Jan 1-15;28(1):69-73)
- Autism (Klauck SM et al. "Molecular genetic analysis of the FMR-1 gene in a large collection of autistic patients" Hum Genet 1997 Aug;100(2):224-9)
- Ulcerative colitis (Gloria L et al. "DNA hypomethylation and proliferative activity are increased in the rectal mucosa of patients with long-standing ulcerative colitis" Cancer 1996 Dec 1;78(11):2300-6)
- Fragile X syndrome (Hornstra IK et al. "High resolution methylation analysis of the FMR1 gene trinucleotide repeat region in fragile X syndrome" Hum Mol Genet 1993 Oct;2(10):1659-65)
- Huntington's disease (Ferluga J et al. "Possible organ and age-related epigenetic factors in Huntington's disease and colorectal carcinoma" Med Hypotheses 1989 May;29(1):51-4)

All the above-cited documents are hereby incorporated by reference.

Furthermore, it is known that the methylation pattern of methylation sensitive sites of other genes that are associated with other diseases is modified during the acute or non-acute phases of these diseases. Those genes are depicted in the listing of genes that is enclosed in this application and are associated with, for example, diseases related to angiogenesis, apoptosis, behavior, disorders of the cell cycle, cell signalling, developmental disorders, diseases related with DNA adducts, DNA damage, disorders in DNA replication, gene regulation, diseases related to immunological disorders, disturbances of the metabolism, metastasis, diseases related to miscellaneous clinical syndromes, pharmacological conditions, diseases related to a disturbed signal transduction, disturbed transcription, and tumour suppression/oncogene related diseases.

### SUMMARY OF THE INVENTION

In view of the foregoing, an object of the invention is to provide methods, systems and computer program products for determining the biological effect and/or activity of drugs, chemical substances and/or pharmaceutical compositions using their effect on DNA-methylation as a marker for their biological effect(s).

A further object of the invention is to provide the inventive systems, methods and computer program products for their use in determining a drug, chemical substance and/or pharmaceutical composition that is biologically effective and/or active.

A further object of the invention is to provide biologically effective and/or active drugs, chemical substances and/or pharmaceutical compositions, which can be obtained using a method according to the invention.

A further object of the invention is to provide the biologically effective and/or active drug, chemical substance and/or pharmaceutical composition which is obtained using a method according to the invention for the use in a treatment of a disease and/or medical condition.

A further object of the invention is to provide systems, methods and computer program products for performing any of the inventive methods.

A further object of the invention is to provide a treatment of a disease and/or medical condition, based on a biologically effective and/or active drug, chemical substance and/or pharmaceutical composition according to the invention.

This object is solved according to the present invention by providing a method for determining the biological effect and/or activity of at least one drug, chemical substance and/or pharmaceutical composition, which comprises the steps of:
(a) obtaining a biological sample A containing DNA from at least one individual, tissue, cell or other biological material containing DNA, which was exposed to said at least one drug, chemical substance and/or pharmaceutical composition;
(b) obtaining a biological sample B containing DNA from at least one individual, tissue, cell or other biological material containing DNA, which was not exposed to said at least one drug, chemical substance or pharmaceutical composition;
(c) analysing the level of cytosine methylation at chosen sites of the DNA contained in the samples A and B;
(d) selecting the sites which are differentially methylated between the DNA in samples A and B,
   whereby a knowledge base is generated; and
(e) concluding from the said knowledge base on the biological effect and/or activity of said at least one drug, chemical substance or pharmaceutical composition.

The present invention uses the modifications in the methylation pattern of the DNA for screening of biologically effective substances. In general, the invention uses the fact that the biological effect of a potentially biologically effective drug, chemical substance or pharmaceutical composition results in a modification of the DNA-methylation pattern of a cell or biological test system upon contact with the drug, chemical substance or pharmaceutical composition. Analysis of the modification of the pattern allows a direct conclusion about the biological effect of the drug, chemical substance or pharmaceutical composition in vivo and its potential application in the treatment of diseases or medical conditions.

The invention has several advantages in comparison to other screening methods, in particular combinatorial chemistry. First, the reaction conditions of the drug, chemical substance or pharmaceutical composition with the biological test system can be controlled in a very reliable manner. Modifications can be applied in a highly reproducible way due to the simplicity of the assay procedure and conditions.

Second, the analysis of the methylation pattern of the DNA allows screening of the in vivo effect of the substance in a one-step procedure using one controllable reaction (namely, the bisulfite treatment in order to look at the methylation status) instead of millions of unknown interactions between drugs, chemical substances or pharmaceutical compositions compounds and compounds of the cell or biological test system.

Thirdly, screening for potential lead-compounds becomes less time consuming and less costly, since the complete screening and analysis procedure can be automated.

Fourth, the inventive method allows the inclusion of personal data into the selection/analysis procedure which allows for a personalised screening of drugs, chemical substances or pharmaceutical compositions.

Other preferred embodiments of the invention will become apparent to the person skilled in the art after reading the features of the dependent claims.

In one embodiment of the method according to the invention, the biological sample is obtained by means of a biopsy, by means of an operation on an individual, by means of a dissection, derived from a preserved biological sample, collected from body fluid(s) and/or collected directly from the environment. In general, the only prerequisite for such a biological sample is to contain DNA which can be used directly or indirectly for the methylation analysis.

In another embodiment of the method according to the invention, the biological sample comprises a eucaryotic and/or procaryotic cell line, a biopsy sample, blood, sputum, faeces, urine, cerebral liquid, tissue embedded in paraffin, tissue derived from eyes, intestine, brain, heart, prostata, kidney, lung, breast or liver, histological samples or a combination thereof.

A preferred method according to the invention is characterised in that the biological sample is obtained from biological material of healthy and/or diseased individuals. Such diseases include all diseases and/or medical conditions which involve a modification of the DNA methylation of the cell and include, for example, unwanted side effects of medicaments, cancers, metastasis, dysfunctions, damages or diseases of the central nervous system (CNS), aggressive symptoms or behavioural disorders, clinical, psychological and social consequences of brain injuries, psychotic disorders and disorders of the personality, dementia and/or associates syndromes, cardiovascular diseases, malfunctions or damages, diseases, malfunctions or damages of the gastrointestine, diseases, malfunctions or damages of the respiratory system, injury, inflammation, infection, immunity and/or reconvalescence, diseases, malfunctions or damages as consequences of modifications in the developmental process, diseases, malfunctions or damages of the skin, muscles, connective tissue or bones, endocrine or metabolic diseases, malfunctions or damages, headache, and sexual malfunctions or combinations thereof, leukemia, head and neck cancer, Hodgkin's disease, gastric cancer, prostate cancer, renal cancer, bladder cancer, breast cancer, Burkitt's lymphoma, Wilms tumor, Prader-Willi/Angelman syndrome, ICF syndrome, dermatofibroma, hypertension, pediatric neurobiological diseases, autism, ulcerative colitis, fragile X syndrome, and Huntington's disease.

In a further preferred method according to the invention, the biological samples A and B are obtained from the identical individual, tissue, cell or other biological material.

In a further preferred method according to the invention, the biological samples A and B are taken before, during and/or after onset of a treatment with said drug, chemical substance or pharmaceutical composition. This allows the use of the inventive method to monitor and/or modify an already employed treatment regimen and to screen for unwanted side effects of the initially employed drugs, chemical substances or pharmaceutical compositions which leads to a strictly "personalised" medicament and/or treatment.

An even more preferred method according to the invention further comprises the step of isolating DNA from the said samples before analysing the level of cytosine methylation at chosen sites in said isolated DNA. This facilitates the reliability and the handling of the DNA in the further analyses procedures. Nevertheless, the inventive method can be successfully be performed even without any purification of the DNA. The isolation of the DNA for performing the inventive method can be characterised in that the isolation of said DNA contained in said biological sample comprises isolating subcellular compartments, organelles, macromolecular structures and multiprotein complexes, partial or complete preparation of the DNA and/or partial digestion of the material with an enzyme selected from proteases, RNAses and/or DNAses or combinations thereof. The pre-isolation of only parts of the cells, like organelles or the like, allows a pre-selection of the genes to be analysed. Other methods mentioned above can limit the amount of cellular debris which could interfere with the further analysis.

In another embodiment of the method according to the invention, the analysis of the level of cytosine methylation comprises chemical treatment with bisulphite, hydrogen sulphite or disulphite, polymerase chain reaction (PCR), hybridisation analyses, sequencing, mass spectrometry and fluorescent, enzymatic, radioactive, dye and/or antibody labelling. In general, all methods for the analysis of the methylation statuses at selected sites of the DNA can be employed. Such methods are known to the skilled artisan and are described in, for example, Dahl et al., "Analysis of in vivo methylation." Methods Mol Biol 2000;130:47-57; Zhou Y. et al., "Use of a single sequencing termination reaction to distinguish between cytosine and 5-methylcytosine in bisulfite-modified DNA." Biotechniques 1997 May;22(5):850-4; Yoder JA et al. "Genetic analysis of genomic methylation patterns in plants and mammals." C Biol Chem 1996 Oct;377(10):605-10 and others.

Another preferred method according to the invention is characterised in that all potentially methylated sites of the DNA are analysed. Such sites usually include all so-called "CpG"-islands on a given DNA sequence and are readily detectable by the person skilled in the art.

Preferably, the level of at least two cytosine methylation sites is analysed in parallel, in order to test the potential effect of the drug, chemical substance or pharmaceutical composition on more than one methylation site. Preferably, the level of at least 100 cytosine methylation sites is analysed in parallel. The analysis of a multitude of sites in parallel allows for both an effective screening and a statistically highly relevant result of the method.

In general, the effect of the drug, chemical substance or pharmaceutical composition to be analysed on the biological cell, tissue or other biological system results in a modification of the expression of the genes of the respective cell, tissue or other biological system. Nevertheless, it is further preferred to analyse methylation sites that are located in methylation relevant regions of the DNA other than the genes themselves and which comprise complete genes and/or promoters, introns, first exons and/or enhancers. From the analysis of the methylation sites which are relevant for the expression of a certain gene, but not localised inside the sequence of the gene itself, the effect of the site for the expression of the gene can be readily extrapolated by the person skilled in the art.

For example, such methylation sites are located in methylation relevant regions of genes related with unwanted side effects of medicaments, cancers, metastasis, dysfunctions, damages or diseases of the central nerval system (CNS), aggressive symptoms or behavioural disorders, clinical, psychological and social consequences of brain injuries, psychotic disorders and disorders of the personality, dementia and/or associates syndromes, cardiovascular diseases, malfunctions or damages, diseases, malfunctions or damages of the gastrointestine, diseases, malfunctions or damages of the respiratory system, injury, inflammation, infection, immunity and/or reconvalescence, diseases, malfunctions or damages as consequences of modifications in the developmental process, diseases, malfunctions or damages of the skin, muscles, connective tissue or bones, endocrine or metabolic diseases, malfunctions or damages, headache, and sexual malfunctions or combinations thereof. Examples for genes, that are related to diseases related to angiogenesis, apoptosis, behavior, disorders of the cell cycle, cell signalling, developmental disorders, diseases related with DNA adducts, DNA damage, disorders in DNA replication, gene regulation, diseases related to immunological disorders, disturbances of the metabolism, metastasis, diseases related to miscellaneous clinical syndromes, medical and pharmacological conditions, diseases related to a disturbed signal transduction, disturbed transcription, and tumour suppression/oncogene related diseases are depicted in the listing enclose in this application. An effect of the drug, chemical substance or pharmaceutical composition to be analysed on the methylation statuses of sites which are relevant for the expression for gene(s) known to be related with these diseases would allow one to directly connect the tested drug, chemical substance or pharmaceutical composition with an effect on those genes and therefore allow the identification of possibly valuable new lead compounds as well as therapeutically important compounds.

Particularly preferred is a method according to the invention which is characterised in that the methylation sites are located in methylation relevant regions of genes related with leukemia, head and neck cancer, Hodgkin's disease, gastric cancer, prostate cancer, renal cancer, bladder cancer, breast cancer, Burkitt's lymphoma, Wilms tumor, Prader-Willi/Angelman syndrome, ICF syndrome, dermatofibroma, hypertension, pediatric neurobiological diseases, autism, ulcerative colitis, fragile X syndrome, and Huntington's disease.

For the use of the inventive method for personalised medicine, it is preferred that the analysed methylation sites are disease specific and/or personalised. This means, that a selection of sites is performed before the methylation analysis which allows a search which "looks" for an effect of a drug, chemical substance or pharmaceutical composition that is specifically suited (designed) for the individual need of the patient.

A further preferred method according to the invention is characterised in that the selection is based on the result of at least two individual rows of analyses. This will reduce the statistical error for the value of the methylation sensitivity of a selected site with an only limited increase of the costs for the analysis. In another preferred method according to the invention, the selection is performed in such a way to give a knowledge base comprising only one set of selected sites. Thus, the knowledge base will comprise only "on" and "off' type of data which allows for a very simple decision between different effects of different drugs, chemical substances or pharmaceutical compositions. In yet another embodiment of the inventive method, the selection is performed in such a way to result in a knowledge base comprising different classes of selected sites. Such classes can be referred to as "quality classes" which allows for a much more differentiated analysis of the effect of the drugs, chemical substances or pharmaceutical compositions to be analysed. The term "quality classes" as used herein comprises all different possibilities of grouping the different sites. Such groupings could, for example, include different importance for the selected sites for the analysis of the biological effect as well as statistical preciseness and/or quality of the analysis data of the selected site.

In a preferred method according to the invention, the selection is at least partially performed automatically by means of a suited automate, e.g. a computer device. Such device would be equipped with the necessary software for the analysis of the methylation sites and could be connected to an inter- or intranet, be part of a neural network or the like. The necessary data/information for the analyses can be present on the system directly or at a remote source, to which the device is directly or indirectly connected, for example via the internet.

In a preferred method according to the invention, at least two sites are selected in parallel. More preferably, at least 100 sites are selected in parallel. For the calculation of the results of the selection and the conclusion, all or only a part of the sites of the knowledge base can be used. In another embodiment of the method according to the invention, additional information about the biological sample is used for the conclusion. This additional information can comprise personal patient data, disease specific data, prior treatment data and/or additional methylation specific data.

According to another aspect of the method of the present invention, the conclusion is based on the result of at least two individual rows of analyses. This provides for an internal control run of the data which is used for the conclusion and increases the preciseness of the results. Preferably, the conclusion is performed by a computer system. Such device would be equipped with the necessary software for the conclusion and could be connected to an inter-or intranet, be part of a neural network or the like. The necessary data/information for the conclusion can be present on the system directly or at a remote source, to which the device is directly or indirectly connected, for example via the internet.

Another embodiment of the method according to the invention is characterised in that steps a) to d) are repeated. Repeating the method of the invention suits several different purposes. First, as mentioned above, the statistical quality of the of the resulting data increases. Second, an internal control can be provided, whether the biological sample was taken correctly and resembles e.g. the tissue of interest. Third, the inventive method can be repeated after a certain time after taking the first sample in order to provide for a monitoring of the effect of the drug(s), chemical substance(s) or pharmaceutical composition(s) to be analysed over time.

This information could be included in the results of the analysis in order to provide a more precise picture of the biological effect of the drug, chemical substance or pharmaceutical composition to be analysed. With the commonly used method of combinatorial chemistry, this aspect can not be analysed since this method uses a "dead" system for the analysis of the compounds.

In one embodiment, the method of the invention is characterised in that the identical biological sample, different biological samples or a combination thereof is used in steps a) and/or b). In another embodiment the method of the invention is characterised in that steps c) to d) are repeated. The number of repeating "cycles" of the invention can vary depending on the individual case, e.g. depending on the quality of the sample to be analyse. One possibility would be to repeat the method of the invention for at least 5 to 50 times.

Preferably, such method according to the invention is characterised in that the method is at least partially performed by means of a suited automate, for example a robot and/or a computer system. The inventive method can be conveniently automated and/or computerized and respective devices and programs are readily known to the person skilled in the art.

A still further object of the invention is the use of the inventive method for determining at least one drug, chemical substance and/or pharmaceutical composition that is biologically effective and/or active.

Preferred is a use, wherein said at least one drug, chemical substance and/or pharmaceutical composition is biologically effective and/or active in the treatment of unwanted side effects of medicaments, cancers, metastasis, dysfunctions, damages or diseases of the central nerval system (CNS), aggressive symptoms or behavioural disorders, clinical, psychological and social consequences of brain injuries, psychotic disorders and disorders of the personality, dementia and/or associates syndromes, cardiovascular diseases, malfunctions or damages, diseases, malfunctions or damages of the gastrointestine, diseases, malfunctions or damages of the respiratory system, injury, inflammation, infection, immunity and/or reconvalescence, diseases, malfunctions or damages as consequences of modifications in the developmental process, diseases, malfunctions or damages of the skin, muscles, connective tissue or bones, endocrine or metabolic diseases, malfunctions or damages, headache, and sexual malfunctions or combinations thereof.

Even more preferred is a use, wherein said at least one drug, chemical substance and/or pharmaceutical composition is biologically effective and/or active in the treatment of leukemia, head and neck cancer, Hodgkin's disease, gastric cancer, prostate cancer, renal cancer, bladder cancer, breast cancer, Burkitt's lymphoma, Wilms tumor, Prader-Willi/Angelman syndrome, ICF syndrome, dermatofibroma, hypertension, pediatric neurobiological diseases, autism, ulcerative colitis, fragile X syndrome, and Huntington's disease.

Another aspect of the invention is a biologically effective and/or active drug, chemical substance and/or pharmaceutical composition which is obtained according to a method according to the invention. Accordingly, another aspect of the invention is, to use such a biologically effective and/or active drug, chemical substance and/or pharmaceutical composition according for the treatment of a disease and/or medical condition.

Preferably, such disease and/or medical condition is related to unwanted side effects of medicaments, cancers, metastasis, dysfunctions, damages or diseases of the central nerval system (CNS), aggressive symptoms or behavioural disorders, clinical, psychological and social consequences of brain injuries, psychotic disorders and disorders of the personality, dementia and/or associates syndromes, cardiovascular diseases, malfunctions or damages, diseases, malfunctions or damages of the gastrointestine, diseases, malfunctions or damages of the respiratory system, injury, inflammation, infection, immunity and/or reconvalescence, diseases, malfunctions or damages as consequences of modifications in the developmental process, diseases, malfunctions or damages of the skin, muscles, connective tissue or bones, endocrine or metabolic diseases, malfunctions or damages, headache, and sexual malfunctions or combinations thereof. Other diseases would be diseases related to angiogenesis, apoptosis, behavior, disorders of the cell cycle, cell signalling, developmental disorders, diseases related with DNA adducts, DNA damage, disorders in DNA replication, gene regulation, diseases related to immunological disorders, disturbances of the metabolism, metastasis, diseases related to miscellaneous clinical syndromes, medical and pharmacological conditions, diseases related to a disturbed signal transduction, disturbed transcription, and tumour suppression/oncogene related diseases.

Most preferably, said disease and/or medical condition is leukemia, head and neck cancer, Hodgkin's disease, gastric cancer, prostate cancer, renal cancer, bladder cancer, breast cancer, Burkitt's lymphoma, Wilms tumor, Prader-Willi/Angelman syndrome, ICF syndrome, dermatofibroma, hypertension, pediatric neurobiological diseases, autism, ulcerative colitis, fragile X syndrome, and/or Huntington's disease.

Another aspect of the invention is related to a method for the treatment of a disease and/or medical condition, which comprises: a) providing at least one biologically effective and/or active drug, chemical substance and/or pharmaceutical composition obtained according to the invention; and b) installing a treatment for the disease and/or medical condition comprising application of the at least one biologically effective and/or active drug, chemical substance and/or pharmaceutical composition to the patient in need. Preferred in this context is a method according to the invention wherein said specific treatment is a disease specific and/or personalised. Such personalised treatment cannot reasonably be achieved with methods of treatment according to the state of the art as present.

Particularly preferred is the use of the inventive method for the treatment of unwanted side effects of medicaments, cancers, dysfunctions, damages or diseases of the central nerval system (CNS), aggressive symptoms or behavioural disorders, clinical, psychological and social consequences of brain injuries, psychotic disorders and disorders of the personality, dementia and/or associates syndromes, cardiovascular diseases, malfunctions or damages, diseases, malfunctions or damages of the gastrointestine, diseases, malfunctions or damages of the respiratory system, injury, inflammation, infection, immunity and/or reconvalescence, diseases, malfunctions or damages as consequences of modifications in the developmental process, diseases, malfunctions or damages of the skin, muscles, connective tissue or bones, endocrine or metabolic diseases, malfunctions or damages, headache, and sexual malfunctions or combinations thereof. Furthermore, the inventive method can be used for the treatment of diseases and/or conditions which are related to the genes as depicted in the listing of genes enclosed in this application, namely genes related to angiogenesis, apoptosis, behavior, disorders of the cell cycle, cell signalling, developmental disorders, diseases related with DNA adducts, DNA damage, disorders in DNA replication, gene regulation, diseases related to immunological disorders, disturbances of the metabolism, metastasis, diseases related to miscellaneous clinical syndromes, medical and pharmacological conditions, diseases related to a disturbed signal transduction, disturbed transcription, and tumour suppression/oncogene related diseases.

Even more preferred is the use of the method according to the invention for the treatment of leukemia, head and neck cancer, Hodgkin's disease, gastric cancer, prostate cancer, renal cancer, bladder cancer, breast cancer, Burkitt's lymphoma, Wilms tumor, Prader-Willi/Angelman syndrome, ICF syndrome, dermatofibroma, hypertension, pediatric neurobiological diseases, autism, ulcerative colitis, fragile X syndrome, and Huntington's disease.

The invention shall now be explained in more detail by the following examples without limiting the scope of the concept of the invention.

The invention shall now be explained in more detail by the following examples without limiting the scope of the concept of the invention.

### EXAMPLES

### Example 1:

### Determination of the biological effect of Tumour Necrosis Factor (TNF)

The colon cancer cell line HAT-29P218 was treated with 10 ng/ml TNF-alpha 1 and 9 ng/ml TGF-beta 1 for 10 days. The media was exchanged after each treatment of 48 h and followed by supplementation TGF-alpha 1 and TGF-beta 1 at the indicated concentrations.

After 10 days, the cells of the cytokine treated and the untreated control cell line cultures were collected by centrifugation and the chromosomal DNA was prepared using QIAamp DNA Mini Kit as recommended by the manufacturer (Quiagen, Hilden, Germany).

Subsequently, the chromosomal DNAs were bisulphite treated as published, for example, according to Olek et al. (ref?**) 6 different multiplex PCR reactions were performed on the 3 bisulphite DNA samples (untreated control, TGF-alpha 1 and TGF-beta 1 treated samples) using Cy5-labelled primer. The products of the methylation specific PCR reactions performed on the same DNA samples were combined. These complex mixtures of 64 PCR products derived from the three DNA samples were comparatively hybridised onto oligo micro arrays representing 256 CpG and the methylation statuses of the CpGs were analysed (see, for example, WO 99/28498). Comparison of three individual hybridisation assays of 2 complex PCRs independently performed on the 3 samples showed that the methylation status of CpGs of the c-myc and the p16 genes were significantly changed by TNF-alpha 1 and TNF-beta 1, respectively.

### Example 2

### Screening of a peptide library

A peptide library was prepared in a 96-well culture plate which contained overlapping peptide fragments derived from the peptide sequence of human serum albumine (HSA). As positive controls, each of vasoactive intestinal peptide (VIP) and pituitary adenylate cyclase-activating polypeptide (PACAP)was added in one of the wells. Both peptides are known to protect T cells from activation-induced cell death through down-regulation of Fas ligand (FasL) (Delgado M et al. "Vasoactive Intestinal Peptide and Pituitary Adenylate Cyclase-Activating Polypeptide Inhibit Expression of Fas Ligand in Activated T Lymphocytes by Regulating c-Myc, NF-kappaB, NF-AT, and Early Growth Factors 2/3" J Immunol 2001 Jan 15;166(2):1028-1040).

A T-cell line (example?) was incubated together with the peptides and VIP or PACAP. After 10 days, the cells of the VIP/PACAP and HSA peptide treated as well as untreated control cell line cultures were collected by centrifugation and the chromosomal DNA was prepared using QIAamp DNA Mini Kit as recommended by the manufacturer (Quiagen, Hilden, Germany).

Subsequently, the chromosomal DNAs were bisulphite treated as published, for example, according to Olek et al. 6 different multiplex PCR reactions were performed on the 3 bisulphite DNA samples (untreated control, HSA and VIP/PACAP treated samples) using Cy5-labelled primer. The products of the methylation specific PCR reactions performed on the same DNA samples were combined. These complex mixtures of 64 PCR products derived from the three DNA samples were comparatively hybridised onto oligo micro arrays representing 256 CpG and the methylation statuses of the CpGs were analysed according to a method described in WO 99/28498. Comparison of three individual hybridisation assays of 2 complex PCRs independently performed on the 3 samples showed that the methylation status of CpGs of the Fas-L gene was significantly changed by VIP and PACAP, respectively whereas no changes were seen with HSA derived peptides.

### Example 3

### Screening of a fractionated plant crude extract

In order to analyse the anti-metastatic effect of Celosia argentea seed extracts (CAE), which have traditionally been used as a therapeutic drug for eye and hepatic diseases in China and Japan a water extract of the seeds was prepared. Hayakawa Y et al. ("Anti-metastatic and immunomodulating properties of the water extract from Celosia argentea seeds Biol Pharm Bull 1998 Nov;21(11):1154-9") report that the anti-metastatic effect of CAE is based on its immunomodulating properties including induction of cytokines such as IL-12, IL-2 and IFN-gamma leading to a Th1 dominant immune state and activating macrophages to the tumoricidal state.

In order to prove this, macrophages were incubated with the CEA water extract which was added to culture media. After 10 days, the cells of the extract treated and the untreated control cell line cultures were collected by centrifugation and the chromosomal DNA was prepared using QIAamp DNA Mini Kit as recommended by the manufacturer (Quiagen, Hilden, Germany).

Subsequently, the chromosomal DNAs were bisulphite treated as published, for example, according to Olek et al. 6 different multiplex PCR reactions were performed on the 3 bisulphite DNA samples (untreated control and CEA treated samples) using Cy5-labelled primer. The products of the methylation specific PCR reactions performed on the same DNA samples were combined. These complex mixtures of 64 PCR products derived from the three DNA samples were comparatively hybridised onto oligo micro arrays representing 256 CpG and the methylation statuses of the CpGs were analysed (Olek et al. WO 99/28498). Comparison of three individual hybridisation assays of 2 complex PCRs independently performed on the 2 samples showed that the methylation status of CpGs of the IL-12, IL-2 and IFN-gamma genes were significantly changed by CEA water extract.

### Listing of genes for panels

### Angiogenesis

ADM (Adrenomedullin); ANG (Angiogenin, ribonuclease, RNase A family, 5); ANGPT1 (Angiopoietin 1); ANGPT2 (Angiopoietin 2); ANGPT3 (Angiopoietin 3); ANGPT4 (Angiopoietin 4); ANPEP (Alanyl (membrane) aminopeptidase (aminopeptidase N, aminopeptidase M, microsomal aminopeptidase, CD13, p150)); ARNT (Aryl hydrocarbon receptor nuclear translocator); BDK (Bradykinin); BDKRB2 (Bradykinin receptor B2); BTN (Butyrophilin); CD14 (CD14 antigen); CD19 (CD19 antigen); CD1D (CD1D antigen, d polypeptide); CD2 (CD2 antigen (p50), sheep red blood cell receptor); CD20 (CD20 antigen); CD22 (CD22 antigen); CD33 (CD33 antigen (gp67); CD34 (CD34 antigen); CD37 (CD37 antigen); CD38 (CD38 antigen (p45)); CD39 (CD39 antigen); CD4 (CD4 antigen (p55)); CD47 (CD47 antigen (Rh-related antigen, integrin-associated signal transducer); CD48 (CD48 antigen (B-cell membrane protein); CD53 (CD53 antigen); CD59 (CD59 antigen p18-20 (antigen identified by monoclonal antibodies 16.3A5, EJ16, EJ30, EL32 and G344); CD63 (CD63 antigen (melanoma 1 antigen); CD68 (CD68 antigen); CD7 (CD7 antigen (p41)); CD72 (CD72 antigen); CD8A (CD8 antigen, alpha polypeptide (p32)); CD9 (CD9 antigen (p24)); CD97 (CD97 antigen); CDW52 (CDW52 antigen (CAMPATH-1 antigen)); COL4A2 (Collagen, type IV, alpha 2); COL4A4 (Collagen, type IV, alpha 4); DAF (Decay accelerating factor for complement (CD55, Cromer blood group system)); DPP4 (Dipeptidylpeptidase IV (CD26, adenosine deaminase complexing protein 2)); EGF (Epidermal growth factor); EGFR (Epidermal growth factor receptor (avian erythroblastic leukemia viral (v-erb-b) oncogene homolog)); EMAPII (Endothelial monocyte-activating polypeptide); EMAPL (Echinoderm microtubule-associated protein-like); ENG (Endoglin (Osler-Rendu-Weber syndrome 1)); F3 ( (Coagulation factor III (thromboplastin, tissue factor)); F8C (Coagulation factor VIIIc, procoagulant component (hemophilia A)); FAP (Fibroblast activation protein, alpha); FGF1 (Fibroblast growth factor 1 (acidic)); FGF2 (Fibroblast growth factor 2 (basic)); FGFR1 (Fibroblast growth factor receptor 1 (fins-related tyrosine kinase 2, Pfeiffer syndrome)); FGFR2 (Fibroblast growth factor receptor 2 (bacteria-expressed kinase, keratinocyte growth factor receptor, craniofacial dysostosis 1, Crouzon syndrome, Pfeiffer syndrome, Jackson-Weiss syndrome)); FIGF (C-fos induced growth factor (vascular endothelial growth factor D)); FLT1 (Fms-related tyrosine kinase 1 (vascular endothelial growth factor/vascular permeability factor receptor)); FLT4 (Fms-related tyrosine kinase 4); FN1 (Fibronectin 1); GRB2 (Growth factor receptor-bound protein 2); HGF (Hepatocyte growth factor (hepapoietin A; scatter factor)); HIF1A (Hypoxia-inducible factor 1, alpha subunit (basic helix-loop-helix transcription factor)); IGF 1 (Insulin-like growth factor 1 (somatomedin C)); IGF2 (Insulin-like growth factor 2 (somatomedin A)); ITGBL1 (Integrin, beta-like 1 (with EGF-like repeat domains)); JAG1 (Jagged1 (Alagille syndrome)); JAG2 (Jagged 2); KAI1 (Kangai 1 (suppression of tumorigenicity 6, prostate); CD82 antigen (R2 leukocyte antigen, antigen detected by monoclonal and antibody IA4)); KDR (Kinase insert domain receptor (a type III receptor tyrosine kinase)); LAG3 (Lymphocyte-activation gene 3); LAMA1 (Laminin, alpha 1); LAMA2 (Laminin, alpha 2 (merosin, congenital muscular dystrophy)); LAMA4 (Laminin, alpha 4); LAMA5 (Laminin, alpha 5); LY64 (Lymphocyte antigen 64 (mouse) homolog, radioprotective, 105kD); LYZ (Lysozyme (renal amyloidosis)); MDU1 (Antigen identified by monoclonal antibodies 4F2, TRA1.10, TROP4, and T43); MET (Met proto-oncogene (hepatocyte growth factor receptor)); MIC2 (Antigen identified by monoclonal antibodies 12E7, F21 and 013); MICA (MHC class I polypeptide-related sequence A); MME (Membrane metallo-endopeptidase (neutral endopeptidase, enkephalinase, CALLA, CD 10)); MMP1 (Matrix metalloproteinase 1 (interstitial collagenase)); MMP10 (Matrix metalloproteinase 10 (stromelysin 2)); MMP2 (Matrix metalloproteinase 2 (gelatinase A, 72kD gelatinase, 72kD type IV collagenase)); MMP9 (Matrix metalloproteinase 9 (gelatinase B, 92kD gelatinase, 92kD type IV collagenase)); NEO1 (Neogenin (chicken) homolog 1); NOS2A (Nitric oxide synthase 2A (inducible, hepatocytes)); NOS3 (Nitric oxide synthase 3 (endothelial cell)); NRP1 (Neuropilin 1); NRP2 (Neuropilin 2); PAI1 (Plasminogen activator inhibitor, type I); PAI2 (Plasminogen activator inhibitor, type II (arginine-serpin)); PDNP1 (Phosphodiesterase I/nucleotide pyrophosphatase 1 (homologous to mouse Ly-41 antigen)); PF4 (Platelet factor 4); PF4V1 (Platelet factor 4 variant 1); PLAU (Plasminogen activator, urokinase); PLAUR (Plasminogen activator, urokinase receptor); PLG (Plasminogen); PRKCA (Protein kinase C, alpha); PRKCB 1 (Protein kinase C, beta 1); PRKM1 (Protein kinase, mitogen-activated 1 (MAP kinase 1; p40, p41)); PRKM10 (Protein kinase mitogen-activated 10 (MAP kinase)); PRKM3 (Protein kinase, mitogen-activated 3 (MAP kinase 3; p44)); PTAFR (Platelet-activating factor receptor); PTCH (Patched (Drosophila) homolog); PTK2 (PTK2 protein tyrosine kinase 2); PTK2B (Protein tyrosine kinase 2 beta); RHO (Rhodopsin (retinitis pigmentosa 4, autosomal dominant)); RTN1 (Reticulon 1); SDC1 (Syndecan 1); SDC2 (Syndecan 2 (heparan sulfate proteoglycan 1, cell surface-associated, fibroglycan)); SDC4 (Syndecan 4 (amphiglycan, ryudocan)); SELPLG (Selectin P ligand); SIAT1 (Sialyltransferase 1 (beta-galactoside alpha-2,6-sialytransferase)); SLAM (Signaling lymphocytic activation molecule); SOS1 (Son of sevenless (Drosophila) homolog 1); SST (Somatostatin); SSTR1 (Somatostatin receptor 1); SSTR2 (Somatostatin receptor 2); SSTR3 (Somatostatin receptor 3); SSTR4 (Somatostatin receptor 4); SSTR5 (Somatostatin receptor 5); ST2 (Suppression of tumorigenicity 2); STAT1 (Signal transducer and activator of transcription 1, 91kD); TEK (TEK tyrosine kinase, endothelial); TFPI (Tissue factor pathway inhibitor (lipoprotein-associated coagulation inhibitor)); TGFB 1 (Transforming growth factor, beta 1); TGFB 1 (Transforming growth factor, beta 1); TGFB1 (Transforming growth factor, beta 1); TGFBR1 (Transforming growth factor, beta receptor I (activin A receptor type II-like kinase, 53kD)); TGFBR1 (Transforming growth factor, beta receptor I (activin A receptor type II-like kinase, 53kD)); TGFBR1 (Transforming growth factor, beta receptor I (activin A receptor type II-like kinase, 53kD)); TGFBR2 (Transforming growth factor, beta receptor II (70-80kD)); THBS 1 (Thrombospondin 1); THBS1 (Thrombospondin 1); THBS2 (Thrombospondin 2); THBS4 (Thrombospondin 4); TIE (Tyrosine kinase with immunoglobulin and epidermal growth factor homology domains); TIEG (TGFB inducible early growth response); TIEG2 (TGFB inducible early growth response 2); TIMP1 (Tissue inhibitor of metalloproteinase 1 (erythroid potentiating activity, collagenase inhibitor)); TIMP2 (Tissue inhibitor of metalloproteinase 2); TM4SF2 (Transmembrane 4 superfamily member 2); TM4SF3 (Transmembrane 4 superfamily member 3); TNF (Tumor necrosis factor (TNF superfamily, member 2)); TNFRSF1A (Tumor necrosis factor receptor superfamily, member 1A (); TNFSF12 (Tumor necrosis factor (ligand) superfamily, member 12); TNFSF4 (Tumor necrosis factor (ligand) superfamily, member 4 (tax-transcriptionally activated glycoprotein 1, 34kD)); TNFSF5 (Tumor necrosis factor (ligand) superfamily, member 5); TNFSF7 (Tumor necrosis factor (ligand) superfamily, member 7); VCAM1 (Vascular cell adhesion molecule 1); VEGF (Vascular endothelial growth factor); VEGFB (Vascular endothelial growth factor B); VEGFC (Vascular endothelial growth factor C); VTN (Vitronectin (serum spreading factor, somatomedin B, complement S-protein)); and VWF (Von Willebrand factor).

### Apoptosis

APAF1 (Apoptotic protease activating factor); API1 (Apoptosis inhibitor 1); API2 (Apoptosis inhibitor 2); API3 (Apoptosis inhibitor 3); API4 (Apoptosis inhibitor 4 (survivin)); BAD (BCL2-antagonist of cell death); BAD (BCL2-antagonist of cell death); BAG4 (BCL2-associated athanogene 4); BAK1 (BCL2-antagonist/killer 1); BAX (BCL2-associated X protein); BCL10 (B-cell CLL/lymphoma 10); BCL2 (B-cell CLL/lymphoma 2); BCL2A1 (BCL2-related protein A1); BCL2L1 (BCL2-like 1); BCL2L2 (BCL2-like 2); BNIP1 (BCL2/adenovirus E1B 19kD-interacting protein 1); BNIP2 (BCL2/adenovirus E1B 19kD-interacting protein 2); BNIP3 (BCL2/adenovirus E1B 19kD-interacting protein 3); CASP1 (Caspase 1, apoptosis-related cysteine protease (interleukin 1, beta, convertase) ); CASP10 (Caspase 10, apoptosis-related cysteine protease); CASP2 (Caspase 2, apoptosis-related cysteine protease (neural precursor cell expressed, developmentally down-regulated 2)); CASP3 (Caspase 3, apoptosis-related cysteine protease); CASP4 (Caspase 4, apoptosis-related cysteine protease); CASP5 (Caspase 5, apoptosis-related cysteine protease); CASP6 (Caspase 6, apoptosis-related cysteine protease); CASP7 (Caspase 7, apoptosis-related cysteine protease); CASP8 (Caspase 8, apoptosis-related cysteine protease); CASP9 (Caspase 9, apoptosis-related cysteine protease); DAP (Death-associated protein); DAP3 (Death associated protein 3); DAPK3 (Death-associated protein kinase 3); DAXX (Death-associated protein 6); DFFA (DNA fragmentation factor, 45 kD, alpha subunit); MCL1 (Myeloid cell leukemia sequence 1 (BCL2-related)); MDM4 (Mouse double minute 4, human homolog of; p53-binding protein); MYD88 (Myeloid differentiation primary response gene (88)); NAIP (Neuronal apoptosis inhibitory protein); PAWR (PRKC, apoptosis, WT1, regulator); PDCD1 (Programmed cell death 1); PDCD2 (Programmed cell death 2); PSEN2 (Presenilin 2 (Alzheimer disease 4)); REQ (Requiem, apoptosis response zinc finger gene); SFRP5 (Secreted frizzled-related protein 5); STK17A (Serine/threonine kinase 17a (apoptosis-inducing)); STK17B (Serine/threonine kinase 17b (apoptosis-inducing)); TNFAIP1 (Tumor necrosis factor, alpha-induced protein 1 (endothelial)); TNFRSF6 (Tumor necrosis factor receptor superfamily, member); and TP53BP2 (Tumor protein p53-binding protein).

### Behavior

ADRA1A (Adrenergic, alpha-1A-, receptor); ADRA1C (Adrenergic, alpha-1C-, receptor); ADRA2A (Adrenergic, alpha-2A-, receptor); ADRA2B (Adrenergic, alpha-2B-, receptor); ADRA2C (Adrenergic, alpha-2C-, receptor); ADRB1 (Adrenergic, beta-1-, receptor); ADRB2 (Adrenergic, beta-2-, receptor, surface); ADRB3 (Adrenergic, beta-3-, receptor); ADRBK1 (Adrenergic, beta, receptor kinase 1); ADRBK2 (Adrenergic, beta, receptor kinase 2); COMT (Catechol-O-methyltransferase); DBH (Dopamine beta-hydroxylase (dopamine beta-monooxygenase)); DDC (Dopa decarboxylase (aromatic L-amino acid decarboxylase)); DRD1 (Dopamine receptor D1); DRD2 (Dopamine receptor D2); DRD3 (Dopamine receptor D3); DRD4 (Dopamine receptor D4); DRD5 (Dopamine receptor D5); MAOA (Monoamine oxidase A); MAOB (Monoamine oxidase B); OPRM1 (Opioid receptor, mu 1); PNMT (Phenylethanolamine N-methyltransferase); and TH (Tyrosine hydroxylase)

### Cell cycle

ABL2 (V-abl Abelson murine leukemia viral oncogene homolog 2 (arg, Abelson-related gene)); ACP1 (Acid phosphatase 1, soluble); ACP2 (Acid phosphatase 2, lysosomal); ACP5 (Acid phosphatase 5, tartrate resistant); ACPP (Acid phosphatase, prostate); ACVR1B (Activin A receptor, type IB); ADK (Adenosine kinase); ADRBK1 (Adrenergic, beta, receptor kinase 1); AK1 (Adenylate kinase 1); AK2 (Adenylate kinase 2); AK3 (Adenylate kinase 3); AKT1 (V-akt murine thymoma viral oncogene homolog 1); AKT2 (V-akt murine thymoma viral oncogene homolog 2); ALPI (Alkaline phosphatase, intestinal); ALPL (Alkaline phosphatase, liver/bone/kidney); ALPP (Alkaline phosphatase, placental (Regan isozyme)); ARAF1 (V-raf murine sarcoma 3611 viral oncogene homolog 1); BLK (B lymphoid tyrosine kinase); BMPR2 (Bone morphogenetic protein receptor, type II (serine/threonine kinase)); BMX (BMX non-receptor tyrosine kinase); BRAF (V-raf murine sarcoma viral oncogene homolog B1); BTK (Bruton agammaglobulinemia tyrosine kinase); CAK (Cell adhesion kinase); CALM1 (Calmodulin 1 (phosphorylase kinase, delta)); CAMK4 (Calcium/calmodulin-dependent protein kinase IV); CBFA2T1 (Core-binding factor, runt domain, alpha subunit 2; translocated to, 1; cyclin D-related); CBFA2T2 (Core-binding factor, runt domain, alpha subunit 2; translocated to, 2); CBFA2T3 (Core-binding factor, runt domain, alpha subunit 2; translocated to, 3); CCNA2 (Cyclin A2); CCNB 1 (Cyclin B1); CCND1 (Cyclin D1 (PRAD1: parathyroid adenomatosis 1)); CCND2 (Cyclin D2); CCND3 (Cyclin D3); CCNE1 (Cyclin E1); CCNE2 (Cyclin E2); CCNF (Cyclin F); CCNH (Cyclin H); CCNK (Cyclin K); CD48 (CD48 antigen (B-cell membrane protein)); CD69 (CD69 antigen (p60, early T-cell activation antigen)); CDC10 (Cell division cycle 10 (homologous to CDC 10 of S. cerevisiae)); CDC18L (CDC 18 (cell division cycle 18, S.pombe, homolog)-like); CDC2 (Cell division cycle 2, G1 to S and G2 to M); CDC25A (Cell division cycle 25A); CDC25A (Cell division cycle 25A); CDC25C (Cell division cycle 25C); CDC25C (Cell division cycle 25C); CDC27 (Cell division cycle 27); CDC2L1 (Cell division cycle 2-like 1 (PITSLRE proteins)); CDC42 (Cell division cycle 42 (GTP-binding protein, 25kD)); CDC7L1 (CDC7 (cell division cycle 7, S. cerevisiae, homolog)-like 1); CDK2 (Cyclin-dependent kinase 2); CDK2 (Cyclin-dependent kinase 2); CDK5 (Cyclin-dependent kinase 5); CDK6 (Cyclin-dependent kinase 6); CDK6 (Cyclin-dependent kinase 6); CDK7 (Cyclin-dependent kinase 7 (homolog of Xenopus MO15 cdk-activating kinase)); CDK7 (Cyclin-dependent kinase 7 (homolog of Xenopus MO15 cdk-activating kinase)); CDK8 (Cyclin-dependent kinase 8); CDK8 (Cyclin-dependent kinase 8); CDKN1B (Cyclin-dependent kinase inhibitor 1B (p27, Kip1)); CDKN1C (Cyclin-dependent kinase inhibitor 1C (p57, Kip2)); CDKN2B (Cyclin-dependent kinase inhibitor 2B (p15, inhibits CDK4)); CHC 1 (Chromosome condensation 1); CHEK1 (CHK1 (checkpoint, S.pombe) homolog); CHES1 (Checkpoint suppressor 1); CHUK (Conserved helix-loop-helix ubiquitous kinase); CKS1 (CDC28 protein kinase 1); CKS2 (CDC28 protein kinase 2); CLK2 (CDC-like kinase 2); CLK3 (CDC-like kinase 3); CNK (Cytokine-inducible kinase); COT (Cot (cancer Osaka thyroid) oncogene); CSNK1A1 (Casein kinase 1, alpha 1); CSNK1D (Casein kinase 1, delta); CSNK1E (Casein kinase 1, epsilon); CSNK2A2 (Casein kinase 2, alpha prime polypeptide); CSNK2B (Casein kinase 2, beta polypeptide); DENR (Density-regulated protein); DGKA (Diacylglycerol kinase, alpha (80kD)); DGKG (Diacylglycerol kinase, gamma (90kD)); DGKQ (Diacylglycerol kinase, theta (110kD)); DMPK (Dystrophia myotonica-protein kinase); DUSP3 (Dual specificity phosphatase 3 (vaccinia virus phosphatase VH1-related)); DUSP4 (Dual specificity phosphatase 4); DYRK1 (Dual-specificity tyrosine-(Y)-phosphorylation regulated kinase 1); E2F2 (E2F transcription factor 2); E2F5 (E2F transcription factor 5, p130-binding); EFNA1 (Ephrin-A1); EFNA5 (Ephrin-A5); EFNB1 (Ephrin-B1); EFNB2 (Ephrin-B2); EGR1 (Early growth response 1); EPHA2 (EphA2); EPHA4 (EphA4); EPHA5 (EphA5); EPHA7 (EphA7); EPHB3 (EphB3); EPHB4 (EphB4); EPHB6 (EphB6); ERBB3 (V-erb-b2 avian erythroblastic leukemia viral oncogene homolog 3); FAP (Fibroblast activation protein, alpha); FBL (Fibrillarin); FES (Feline sarcoma (Snyder-Theilen) viral (v-fes)/Fujinami avian sarcoma (PRCII) viral (v-fps) oncogene homolog); FGFR1 (Fibroblast growth factor receptor 1 (fms-related tyrosine kinase 2, Pfeiffer syndrome)); FGFR2 (Fibroblast growth factor receptor 2 (bacteria-expressed kinase, keratinocyte growth factor receptor, craniofacial dysostosis 1, Crouzon syndrome, Pfeiffer syndrome, Jackson-Weiss syndrome)); FGR (Gardner-Rasheed feline sarcoma viral (v-fgr) oncogene homolog); FLT1 (Fms-related tyrosine kinase 1 (vascular endothelial growth factor/vascular permeability factor receptor)); FLT3LG (Fms-related tyrosine kinase 3 ligand); FLT4 (Fms-related tyrosine kinase 4); FRK (Fyn-related kinase); GAS2 (Growth arrest-specific 2); GLA (Galactosidase, alpha); GPRK6 (G protein-coupled receptor kinase 6); GSPT1 (G1 to S phase transition 1); HMR (Hormone receptor (growth factor-inducible nuclear protein N10)); HUS 1 (HUS1 (S. pombe) checkpoint homolog); IFI56 (Interferon-induced protein 56); IGFBP1 (Insulin-like growth factor binding protein 1); ILK (Integrin-linked kinase); INP10 (Interferon (gamma)-induced cell line; protein 10 from); INPP5D (Inositol polyphosphate-5-phosphatase, 145kD); ISG20 (Interferon stimulated gene (20kD)); JAK1 (Janus kinase 1 (a protein tyrosine kinase)); JAK3 (Janus kinase 3 (a protein tyrosine kinase, leukocyte)); KDR (Kinase insert domain receptor (a type III receptor tyrosine kinase)); LCAT (Lecithin-cholesterol acyltransferase); LIMK1 (LIM domain kinase 1); LIMK2 (LIM domain kinase 2); LRP1 (Low density lipoprotein-related protein 1 (alpha-2-macroglobulin receptor)); LTK (Leukocyte tyrosine kinase); LY6E (Lymphocyte antigen 6 complex, locus E); MAD1L1 (MAD1 (mitotic arrest deficient, yeast, homolog)-like 1); MAD2L1 (MAD2 (mitotic arrest deficient, yeast, homolog)-like 1); MATK (Megakaryocyte-associated tyrosine kinase); MCL1 (Myeloid cell leukemia sequence 1 (BCL2-related)); MCM4 (Minichromosome maintenance deficient (S. cerevisiae) 4); MEKK3 (MAP/ERK kinase kinase 3); MEKK5 (MAP/ERK kinase kinase 5); MKI67 (Antigen identified by monoclonal antibody Ki-67); MST1R (Macrophage stimulating 1 receptor (c-met-related tyrosine kinase)); NCK1 (NCK adaptor protein 1); NEK3 (NIMA (never in mitosis gene a)-related kinase); NME1 (Non-metastatic cells 1, protein (NM23A) expressed in); NME2 (Non-metastatic cells 2, protein (NM23B) expressed in); NOS2A (Nitric oxide synthase 2A (inducible, hepatocytes)); NPM1 (Nucleophosmin (nucleolar phosphoprotein B23, numatrin)); NTRK3 (Neurotrophic tyrosine kinase, receptor, type 3); OAS1 (2',5'-oligoadenylate synthetase 1); PA2G4 (Proliferation-associated 2G4, 38kD); PCNA (Proliferating cell nuclear antigen); PCTK3 (PCTAIRE protein kinase 3); PDGFRB (Platelet-derived growth factor receptor, beta polypeptide); PDK2 (Pyruvate dehydrogenase kinase, isoenzyme 2); PDK4 (Pyruvate dehydrogenase kinase, isoenzyme 4); PDPK1 (3-phosphoinositide dependent protein kinase-1); PHKA2 (Phosphorylase kinase, alpha 2 (liver), glycogen storage disease IX); PHKG2 (Phosphorylase kinase, gamma 2 (testis)); PIK3CA (Phosphoinositide-3-kinase, catalytic, alpha polypeptide); PIK3CG (Phosphoinositide-3-kinase, catalytic, gamma polypeptide); PIM1 (Pim-1 oncogene); POLB (Polymerase (DNA directed), beta); POLD1 (Polymerase (DNA directed), delta 1, catalytic subunit (125kD)); PPP1CA (Protein phosphatase 1, catalytic subunit, alpha isoform); PPP1CB (Protein phosphatase 1, catalytic subunit, beta isoform); PPP1CC (Protein phosphatase 1, catalytic subunit, gamma isoform); PPP1R3 (Protein phosphatase 1, regulatory (inhibitor) subunit 3 (glycogen and sarcoplasmic reticulum binding subunit, skeletal muscle)); PPP2CA (Protein phosphatase 2 (formerly 2A), catalytic subunit, alpha isoform); PPP2R1B (Protein phosphatase 2 (formerly 2A), regulatory subunit A (PR 65), beta isoform); PPP2R2A (Protein phosphatase 2 (formerly 2A), regulatory subunit B (PR 52), alpha isoform); PPP2R3 (Protein phosphatase 2 (formerly 2A), regulatory subunit B" (PR 72), alpha isoform and (PR 130), beta isoform); PPP2R4 (Protein phosphatase 2A, regulatory subunit B' (PR 53)); PPP2R5A (Protein phosphatase 2, regulatory subunit B (B56), alpha isoform); PPP2R5B (Protein phosphatase 2, regulatory subunit B (B56), beta isoform); PPP2R5D (Protein phosphatase 2, regulatory subunit B (B56), delta isoform); PPP2R5E (Protein phosphatase 2, regulatory subunit B (B56), epsilon isoform); PPP3CA (Protein phosphatase 3 (formerly 2B), catalytic subunit, alpha isoform (calcineurin A alpha)); PPP3CB (Protein phosphatase 3 (formerly 2B), catalytic subunit, beta isoform (calcineurin A beta)); PPP4C (Protein phosphatase 4 (formerly X), catalytic subunit); PRKAA2 (Protein kinase, AMP-activated, alpha 2 catalytic subunit); PRKACA (Protein kinase, cAMP-dependent, catalytic, alpha); PRKACB (Protein kinase, cAMP-dependent, catalytic, beta); PRKACG (Protein kinase, cAMP-dependent, catalytic, gamma); PRKAG1 (Protein kinase, AMP-activated, gamma 1 non-catalytic subunit); PRKAR1A (Protein kinase, cAMP-dependent, regulatory, type I, alpha (tissue specific extinguisher 1)); PRKAR1B (Protein kinase, cAMP-dependent, regulatory, type I, beta); PRKAR2B (Protein kinase, cAMP-dependent, regulatory, type II, beta); PRKCA (Protein kinase C, alpha); PRKCB1 (Protein kinase C, beta 1); PRKCG (Protein kinase C, gamma); PRKCI (Protein kinase C, iota); PRKCL1 (Protein kinase C-like 1); PRKCL2 (Protein kinase C-like 2); PRKCM (Protein kinase C, mu); PRKCQ (Protein kinase C, theta); PRKCZ (Protein kinase C, zeta); PRKG1 (Protein kinase, cGMP-dependent, type I); PRKG2 (Protein kinase, cGMP-dependent, type II); PRKM1 (Protein kinase, mitogen-activated 1 (MAP kinase 1; p40, p41)); PRKM 10 (Protein kinase mitogen-activated 10 (MAP kinase)); PRKM11 (Protein kinase mitogen- activated 11); PRKM 13 (Protein kinase mitogen- activated 13); PRKM3 (Protein kinase, mitogen-activated 3 (MAP kinase 3; p44)); PRKM6 (Protein kinase, mitogen-activated 6 (extracellular signal-regulated kinase, p97)); PRKM7 (Protein kinase mitogen-activated 7 (MAP kinase)); PRKM8 (Protein kinase mitogen-activated 8 (MAP kinase)); PRKM9 (Protein kinase mitogen-activated 9 (MAP kinase)); PRKMK2 (Protein kinase, mitogen-activated, kinase 2, p45 (MAP kinase kinase 2)); PRKMK3 (Protein kinase, mitogen-activated, kinase 3 (MAP kinase kinase 3)); PRKMK5 (Protein kinase, mitogen-activated, kinase 5 (MAP kinase kinase 5)); PRKMK7 (Protein kinase, mitogen-activated, kinase 7 (MAP kinase kinase 7)); PRKR (Protein kinase, interferon-inducible double stranded RNA dependent); PTEN (Phosphatase and tensin homolog (mutated in multiple advanced cancers 1)); PTK7 (PTK7 protein tyrosine kinase 7); PTK9 (Protein tyrosine kinase 9); PTPN1 (Protein tyrosine phosphatase, non-receptor type 1); PTPN12 (Protein tyrosine phosphatase, non-receptor type 12); PTPN13 (Protein tyrosine phosphatase, non-receptor type 13 (APO-1/CD95 (Fas)-associated phosphatase)); PTPN2 (Protein tyrosine phosphatase, non-receptor type 2); PTPN3 (Protein tyrosine phosphatase, non-receptor type 3); PTPN4 (Protein tyrosine phosphatase, non-receptor type 4 (megakaryocyte)); PTPN6 (Protein tyrosine phosphatase, non-receptor type 6); PTPN7 (Protein tyrosine phosphatase, non-receptor type 7); PTPN9 (Protein tyrosine phosphatase, non-receptor type 9); PTPRA (Protein tyrosine phosphatase, receptor type, alpha polypeptide); PTPRB (Protein tyrosine phosphatase, receptor type, beta polypeptide); PTPRC (Protein tyrosine phosphatase, receptor type, c polypeptide); PTPRD (Protein tyrosine phosphatase, receptor type, D); PTPRF (Protein tyrosine phosphatase, receptor type, F); PTPRG (Protein tyrosine phosphatase, receptor type, gamma polypeptide); PTPRH (Protein tyrosine phosphatase, receptor type, H); PTPRK (Protein tyrosine phosphatase, receptor type, K); PTPRM (Protein tyrosine phosphatase, receptor type, M); PTPRN (Protein tyrosine phosphatase, receptor type, N); PTPRN2 (Protein tyrosine phosphatase, receptor type, N polypeptide 2); PTX3 (Pentaxin-related gene, rapidly induced by IL-1 beta); RAB8IP (Rab8 interacting protein (GC kinase)); RAB8IP (Rab8 interacting protein (GC kinase)); RAD9 (RAD9 (S. pombe) homolog); RAD9 (RAD9 (S. pombe) homolog); RBL1 (Retinoblastoma-like 1 (p107)); RET (Ret proto-oncogene (multiple endocrine neoplasia MEN2A, MEN2B and medullary thyroid carcinoma 1, Hirschsprung disease)); RHOK (Rhodopsin kinase); ROCK1 (Rho-associated, coiled-coil containing protein kinase 1); RPS6KA2 (Ribosomal protein S6 kinase, 90kD, polypeptide 2); RPS6KB1 (Ribosomal protein S6 kinase, 70kD, polypeptide 1); RYK (RYK receptor-like tyrosine kinase); SAPK3 (Stress-activated protein kinase 3); SERK1 (SAPK/Erk kinase 1); SRC (V-src avian sarcoma (Schmidt-Ruppin A-2) viral oncogene homolog); SRF (Serum response factor (c-fos serum response element-binding transcription factor)); SRPK2 (SFRS protein kinase 2); STK11 (Serine/threonine kinase 11 (Peutz-Jeghers syndrome)); STK2 (Serine/threonine kinase 2); STK3 (Serine/threonine kinase 3 (Ste20, yeast homolog)); STK4 (Serine/threonine kinase 4); SYK (Spleen tyrosine kinase); TCEB1L (Transcription elongation factor B (SIII), polypeptide 1-like); TESK1 (Testis-specific kinase 1); TFDP2 (Transcription factor Dp-2 (E2F dimerization partner 2)); TGFBI (Transforming growth factor, beta-induced, 68kD); TIEG (TGFB inducible early growth response); TK2 (Thymidine kinase 2, mitochondrial); TNFAIP1 (Tumor necrosis factor, alpha-induced protein 1 (endothelial)); TNFSF5 (Tumor necrosis factor (ligand) superfamily, member 5); TS546 (Temperature sensitivity complementation, cell cycle specific,); TTK (TTK protein kinase); TXK (TXK tyrosine kinase); TYK2 (Tyrosine kinase 2); TYR03 (TYRO3 protein tyrosine kinase); VRK2 (Vaccinia related kinase 2); ZAP70 (Zeta-chain (TCR) associated protein kinase (70 kD)); and ZPK (Zipper (leucine) protein kinase).

### Cell signaling

ABL1 (V-abl Abelson murine leukemia viral oncogene homolog 1); ABL2 (V-abl Abelson murine leukemia viral oncogene homolog 2 (arg, Abelson-related gene)); ABR (Active BCR-related gene); ACP1 (Acid phosphatase 1, soluble); ACP2 (Acid phosphatase 2, lysosomal); ACP5 (Acid phosphatase 5, tartrate resistant); ACPP (Acid phosphatase, prostate); ACTG1 (Actin, gamma 1); ACVR1B (Activin A receptor, type IB); ADD3 (Adducin 3 (gamma)); ADK (Adenosine kinase); ADRBK1 (Adrenergic, beta, receptor kinase 1); AIF1 (Allograft inflammatory factor 1); AK1 (Adenylate kinase 1); AK2 (Adenylate kinase 2); AK3 (Adenylate kinase 3); AKT1 (V-akt murine thymoma viral oncogene homolog 1); AKT2 (V-akt murine thymoma viral oncogene homolog 2); ALCAM (Activated leucocyte cell adhesion molecule); ALOX12 (Arachidonate 12-lipoxygenase); ALOX5 (Arachidonate 5-lipoxygenase); ALPI (Alkaline phosphatase, intestinal); ALPL (Alkaline phosphatase, liver/bone/kidney); ALPP (Alkaline phosphatase, placental (Regan isozyme)); ANK1 (Ankyrin 1, erythrocytic); ANT2 (Adenine nucleotide translocator 2 (fibroblast)); ARAF1 (V-raf murine sarcoma 3611 viral oncogene homolog 1); ARHA (Ras homolog gene family, member A); ARHC (Ras homolog gene family, member C); ARHGDIB (Rho GDP dissociation inhibitor (GDI) beta); BLK (B lymphoid tyrosine kinase); BMPR2 (Bone morphogenetic protein receptor, type II (serine/threonine kinase)); BMX (BMX non-receptor tyrosine kinase); BRAF (V-raf murine sarcoma viral oncogene homolog B1); BTK (Bruton agammaglobulinemia tyrosine kinase); CAK (Cell adhesion kinase); CALM1 (Calmodulin 1 (phosphorylase kinase, delta)); CAMK4 (Calcium/calmodulin-dependent protein kinase IV); CBLB (Cas-Br-M (murine) ectropic retroviral transforming sequence b); CD44 (CD44 antigen (homing function and Indian blood group system)); CD47 (CD47 antigen (Rh-related antigen, integrin-associated signal transducer)); CD48 (CD48 antigen (B-cell membrane protein)); CD58 (CD58 antigen, (lymphocyte function-associated antigen 3)); CD69 (CD69 antigen (p60, early T-cell activation antigen)); CDC25A (Cell division cycle 25A); CDC25C (Cell division cycle 25C); CDC2L1 (Cell division cycle 2-like 1 (PITSLRE proteins)); CDC42 (Cell division cycle 42 (GTP-binding protein, 25kD)); CDC42 (Cell division cycle 42 (GTP-binding protein, 25kD)); CDC7L1 (CDC7 (cell division cycle 7, S. cerevisiae, homolog)-like 1); CDH13 (Cadherin 13, H-cadherin (heart)); CDH17 (Cadherin 17, LI cadherin (liver-intestine)); CDH2 (Cadherin 2, N-cadherin (neuronal)); CDH4 (Cadherin 4, R-cadherin (retinal)); CDH5 (Cadherin 5, VE-cadherin (vascular epithelium)); CDK2 (Cyclin-dependent kinase 2); CDK5 (Cyclin-dependent kinase 5); CDK6 (Cyclin-dependent kinase 6); CDK7 (Cyclin-dependent kinase 7 (homolog of Xenopus MO15 cdk-activating kinase)); CDK8 (Cyclin-dependent kinase 8); CDKN2B (Cyclin-dependent kinase inhibitor 2B (p15, inhibits CDK4)); CHEK1 (CHK1 (checkpoint, S.pombe) homolog); CHUK (Conserved helix-loop-helix ubiquitous kinase); CKS1 (CDC28 protein kinase 1); CKS2 (CDC28 protein kinase 2); CLGN (Calmegin); CLK2 (CDC-like kinase 2); CLK3 (CDC-like kinase 3); CLTB (Clathrin, light polypeptide (Lcb)); CNK (Cytokine-inducible kinase); COT (Cot (cancer Osaka thyroid) oncogene); CSNK1A1 (Casein kinase 1, alpha 1); CSNK1D (Casein kinase 1, delta); CSNK1E (Casein kinase 1, epsilon); CSNK2A2 (Casein kinase 2, alpha prime polypeptide); CSNK2B (Casein kinase 2, beta polypeptide); CTNNA1 (Catenin (cadherin-associated protein), alpha 1 (102kD)); CTNNB1 (Catenin (cadherin-associated protein), beta 1 (88kD)); CTNND2 (Catenin (cadherin-associated protein), delta 2 (neural plakophilin-related arm-repeat protein)); DAPK1 (Death-associated protein kinase 1); DGKA (Diacylglycerol kinase, alpha (80kD)); DGKG (Diacylglycerol kinase, gamma (90kD)); DGKQ (Diacylglycerol kinase, theta (110kD)); DMPK (Dystrophia myotonica-protein kinase); DUSP3 (Dual specificity phosphatase 3 (vaccinia virus phosphatase VH1-related)); DUSP4 (Dual specificity phosphatase 4); DVL3 (Dishevelled 3 (homologous to Drosophila dsh)); DYRK1 (Dual-specificity tyrosine-(Y)-phosphorylation regulated kinase 1); EFNA1 (Ephrin-A1); EFNA5 (Ephrin-A5); EFNB1 (Ephrin-B1); EFNB2 (Ephrin-B2); EGR1 (Early growth response 1); EGR3 (Early growth response 3); EGR4 (Early growth response 4); EPHA2 (EphA2); EPHA4 (EphA4); EPHA5 (EphA5); EPHA7 (EphA7); EPHB3 (EphB3); EPHB4 (EphB4); EPHB6 (EphB6); ERBB3 (V-erb-b2 avian erythroblastic leukemia viral oncogene homolog 3); FAP (Fibroblast activation protein, alpha); FAT (FAT tumor suppressor (Drosophila) homolog); FBL (Fibrillarin); FES (Feline sarcoma (Snyder-Theilen) viral (v-fes)/Fujinami avian sarcoma (PRCII) viral (v-fps) oncogene homolog); FGFR1 (Fibroblast growth factor receptor 1 (fms-related tyrosine kinase 2, Pfeiffer syndrome)); FGFR2 (Fibroblast growth factor receptor 2 (bacteria-expressed kinase, keratinocyte growth factor receptor, craniofacial dysostosis 1, Crouzon syndrome, Pfeiffer syndrome, Jackson-Weiss syndrome)); FGR (Gardner-Rasheed feline sarcoma viral (v-fgr) oncogene homolog); FLNA (Filamin A, alpha (actin-binding protein-280)); FLT1 (Fms-related tyrosine kinase 1 (vascular endothelial growth factor/vascular permeability factor receptor)); FLT3LG (Fms-related tyrosine kinase 3 ligand); FLT4 (Fms-related tyrosine kinase 4); FN1 (Fibronectin 1); FRK (Fyn-related kinase); FYB (FYN-binding protein (FYB-120/130)); G1P3 (Interferon, alpha-inducible protein (clone IFI-6-16)); GBP1 (Guanylate binding protein 1, interferon-inducible, 67kD); GBP2 (Guanylate binding protein 2, interferon-inducible); GJB 1 (Gap junction protein, beta 1, 32kD (connexin 32, Charcot-Marie-Tooth neuropathy, X-linked)); GLA (Galactosidase, alpha); GNAI1 (Guanine nucleotide binding protein (G protein), alpha inhibiting activity polypeptide 1); GNG10 (Guanine nucleotide binding protein 10); GPRK6 (G protein-coupled receptor kinase 6); GRB2 (Growth factor receptor-bound protein 2); HMMR (Hyaluronan-mediated motility receptor (RHAMM)); HMR (Hormone receptor (growth factor-inducible nuclear protein N10)); ICAM1 (Intercellular adhesion molecule 1 (CD54), human rhinovirus receptor); ICAM2 (Intercellular adhesion molecule 2); ICAM3 (Intercellular adhesion molecule 3); IFI16 (Interferon, gamma-inducible protein 16); IFI56 (Interferon-induced protein 56); IFIT4 (Interferon-induced protein with tetratricopeptide repeats 4); IGFBP 1 (Insulin-like growth factor binding protein 1); ILK (Integrin-linked kinase); INP10 (Interferon (gamma)-induced cell line; protein 10 from); INPP5D (Inositol polyphosphate-5-phosphatase, 145kD); ISG20 (Interferon stimulated gene (20kD)); ITGA2 (Integrin, alpha 2 (CD49B, alpha 2 subunit of VLA-2 receptor)); ITGA3 (Integrin, alpha 3 (antigen CD49C, alpha 3 subunit of VLA-3 receptor)); ITGA4 (Integrin, alpha 4 (antigen CD49D, alpha 4 subunit of VLA-4 receptor)); ITGA5 (Integrin, alpha 5 (fibronectin receptor, alpha polypeptide)); ITGA6 (Integrin, alpha 6); ITGA7 (Integrin, alpha 7); ITGA9 (Integrin, alpha 9); ITGAE (Integrin, alpha E (antigen CD103, human mucosal lymphocyte antigen 1; alpha polypeptide)); ITGAL (Integrin, alpha L (antigen CD11A (p180), lymphocyte function-associated antigen 1; alpha polypeptide)); ITGAM (Integrin, alpha M (complement component receptor 3, alpha; also known as CD11b (p170), macrophage antigen alpha polypeptide)); ITGAV (Integrin, alpha V (vitronectin receptor, alpha polypeptide, antigen CD51)); ITGAX (Integrin, alpha X (antigen CD11C (p150), alpha polypeptide)); ITGB 1 (Integrin, beta 1 (fibronectin receptor, beta polypeptide, antigen CD29 includes MDF2, MSK12)); ITGB2 (Integrin, beta 2 (antigen CD18 (p95), lymphocyte function-associated antigen 1; macrophage antigen 1 (mac-1) beta subunit)); ITGB3 (Integrin, beta 3 (platelet glycoprotein IIIa, antigen CD61)); ITGB4 (Integrin, beta 4); ITGB5 (Integrin, beta 5); ITGB7 (Integrin, beta 7); ITGB8 (Integrin, beta 8); ITPKB (Inositol 1,4,5-trisphosphate 3-kinase B); JAK1 (Janus kinase 1 (a protein tyrosine kinase)); JAK3 (Janus kinase 3 (a protein tyrosine kinase, leukocyte)); KCNN3 (Potassium intermediate/small conductance calcium-activated channel, subfamily N, member 3); KDR (Kinase insert domain receptor (a type III receptor tyrosine kinase)); KPNB1 (Karyopherin (importin) beta 1); LCAT (Lecithin-cholesterol acyltransferase); LIMK1 (LIM domain kinase 1); LIMK2 (LIM domain kinase 2); LRP 1 (Low density lipoprotein-related protein 1 (alpha-2-macroglobulin receptor)); LTK (Leukocyte tyrosine kinase); LY6E (Lymphocyte antigen 6 complex, locus E); MADH1 (MAD (mothers against decapentaplegic, Drosophila) homolog 1); MADH2 (MAD (mothers against decapentaplegic, Drosophila) homolog 2); MADH3 (MAD (mothers against decapentaplegic, Drosophila) homolog 3); MAP1B (Microtubule-associated protein 1B); MATK (Megakaryocyte-associated tyrosine kinase); MCL1 (Myeloid cell leukemia sequence 1 (BCL2-related)); MEKK3 (MAP/ERK kinase kinase 3); MEKK5 (MAP/ERK kinase kinase 5); MST1R (Macrophage stimulating 1 receptor (c-met-related tyrosine kinase)); MX1 (Myxovirus (influenza) resistance 1, homolog of murine (interferon-inducible protein p78)); MX2 (Myxovirus (influenza) resistance 2, homolog of murine); MYL2 (Myosin, light polypeptide 2, regulatory, cardiac, slow); MYL3 (Myosin, light polypeptide 3, alkali; ventricular, skeletal, slow); NCAM1 (Neural cell adhesion molecule 1); NCK1 (NCK adaptor protein 1); NEK3 (NIMA (never in mitosis gene a)-related kinase); NME1 (Non-metastatic cells 1, protein (NM23A) expressed in); NME2 (Non-metastatic cells 2, protein (NM23B) expressed in); NOS2A (Nitric oxide synthase 2A (inducible, hepatocytes)); NPM1 (Nucleophosmin (nucleolar phosphoprotein B23, numatrin)); NTRK3 (Neurotrophic tyrosine kinase, receptor, type 3); OAS1 (2',5'-oligoadenylate synthetase 1); OCLN (Occludin); PCDH1 (Protocadherin 1 (cadherin-like 1)); PCTK3 (PCTAIRE protein kinase 3); PDE4B (Phosphodiesterase 4B, cAMP-specific (dunce (Drosophila)-homolog phosphodiesterase E4)); PDGFRB (Platelet-derived growth factor receptor, beta polypeptide); PDK2 (Pyruvate dehydrogenase kinase, isoenzyme 2); PDK4 (Pyruvate dehydrogenase kinase, isoenzyme 4); PDPK1 (3-phosphoinositide dependent protein kinase-1); PECAM1 (Platelet/endothelial cell adhesion molecule (CD31 antigen)); PGY3 (P glycoprotein 3/multiple drug resistance 3); PHKA2 (Phosphorylase kinase, alpha 2 (liver), glycogen storage disease IX); PHKG2 (Phosphorylase kinase, gamma 2 (testis)); PIK3C3 (Phosphoinositide-3-kinase, class 3); PIK3CA (Phosphoinositide-3-kinase, catalytic, alpha polypeptide); PIK3CA (Phosphoinositide-3-kinase, catalytic, alpha polypeptide); PIK3CG (Phosphoinositide-3-kinase, catalytic, gamma polypeptide); PIM1 (Pim-1 oncogene); PKD1 (Polycystic kidney disease 1 (autosomal dominant)); PLA2G2A (Phospholipase A2, group IIA (platelets, synovial fluid)); PLCB4 (Phospholipase C, beta 4); PLCE (Phospholipase C, epsilon); PLCG2 (Phospholipase C, gamma 2 (phosphatidylinositol-specific)); PPP1CA (Protein phosphatase 1, catalytic subunit, alpha isoform); PPP1CB (Protein phosphatase 1, catalytic subunit, beta isoform); PPP1CC (Protein phosphatase 1, catalytic subunit, gamma isoform); PPP1R3 (Protein phosphatase 1, regulatory (inhibitor) subunit 3 (glycogen and sarcoplasmic reticulum binding subunit, skeletal muscle)); PPP2CA (Protein phosphatase 2 (formerly 2A), catalytic subunit, alpha isoform); PPP2R1B (Protein phosphatase 2 (formerly 2A), regulatory subunit A (PR 65), beta isoform); PPP2R2A (Protein phosphatase 2 (formerly 2A), regulatory subunit B (PR 52), alpha isoform); PPP2R3 (Protein phosphatase 2 (formerly 2A), regulatory subunit B" (PR 72), alpha isoform and (PR 130), beta isoform); PPP2R4 (Protein phosphatase 2A, regulatory subunit B' (PR 53)); PPP2R5A (Protein phosphatase 2, regulatory subunit B (B56), alpha isoform); PPP2R5B (Protein phosphatase 2, regulatory subunit B (B56), beta isoform); PPP2R5D (Protein phosphatase 2, regulatory subunit B (B56), delta isoform); PPP2R5E (Protein phosphatase 2, regulatory subunit B (B56), epsilon isoform); PPP3CA (Protein phosphatase 3 (formerly 2B), catalytic subunit, alpha isoform (calcineurin A alpha)); PPP3CB (Protein phosphatase 3 (formerly 2B), catalytic subunit, beta isoform (calcineurin A beta)); PPP4C (Protein phosphatase 4 (formerly X), catalytic subunit); PRKAA2 (Protein kinase, AMP-activated, alpha 2 catalytic subunit); PRKACA (Protein kinase, cAMP-dependent, catalytic, alpha); PRKACB (Protein kinase, cAMP-dependent, catalytic, beta); PRKACG (Protein kinase, cAMP-dependent, catalytic, gamma); PRKAG1 (Protein kinase, AMP-activated, gamma 1 non-catalytic subunit); PRKAR1A (Protein kinase, cAMP-dependent, regulatory, type I, alpha (tissue specific extinguisher 1)); PRKAR1B (Protein kinase, cAMP-dependent, regulatory, type I, beta); PRKAR2B (Protein kinase, cAMP-dependent, regulatory, type II, beta); PRKCA (Protein kinase C, alpha); PRKCB 1 (Protein kinase C, beta 1); PRKCG (Protein kinase C, gamma); PRKCI (Protein kinase C, iota); PRKCL1 (Protein kinase C-like 1); PRKCL2 (Protein kinase C-like 2); PRKCM (Protein kinase C, mu); PRKCQ (Protein kinase C, theta); PRKCZ (Protein kinase C, zeta); PRKG1 (Protein kinase, cGMP-dependent, type I); PRKG2 (Protein kinase, cGMP-dependent, type II); PRKM1 (Protein kinase, mitogen-activated 1 (MAP kinase 1; p40, p41)); PRKM10 (Protein kinase mitogen-activated 10 (MAP kinase)); PRKM11 (Protein kinase mitogen-activated 11); PRKM13 (Protein kinase mitogen- activated 13); PRKM3 (Protein kinase, mitogen-activated 3 (MAP kinase 3; p44)); PRKM6 (Protein kinase, mitogen-activated 6 (extracellular signal-regulated kinase, p97)); PRKM7 (Protein kinase mitogen-activated 7 (MAP kinase)); PRKM8 (Protein kinase mitogen-activated 8 (MAP kinase)); PRKM9 (Protein kinase mitogen-activated 9 (MAP kinase)); PRKMK2 (Protein kinase, mitogen-activated, kinase 2, p45 (MAP kinase kinase 2)); PRKMK3 (Protein kinase, mitogen-activated, kinase 3 (MAP kinase kinase 3)); PRKMK5 (Protein kinase, mitogen-activated, kinase 5 (MAP kinase kinase 5)); PRKMK7 (Protein kinase, mitogen-activated, kinase 7 (MAP kinase kinase 7)); PRKR (Protein kinase, interferon-inducible double stranded RNA dependent); PRKR (Protein kinase, interferon-inducible double stranded RNA dependent); PSME1 (Proteasome (prosome, macropain) activator subunit 1 (PA28 alpha)); PTEN (Phosphatase and tensin homolog (mutated in multiple advanced cancers 1)); PTK7 (PTK7 protein tyrosine kinase 7); PTK9 (Protein tyrosine kinase 9); PTPN1 (Protein tyrosine phosphatase, non-receptor type 1); PTPN12 (Protein tyrosine phosphatase, non-receptor type 12); PTPN13 (Protein tyrosine phosphatase, non-receptor type 13 (APO-1/CD95 (Fas)-associated phosphatase)); PTPN2 (Protein tyrosine phosphatase, non-receptor type 2); PTPN3 (Protein tyrosine phosphatase, non-receptor type 3); PTPN4 (Protein tyrosine phosphatase, non-receptor type 4 (megakaryocyte)); PTPN6 (Protein tyrosine phosphatase, non-receptor type 6); PTPN7 (Protein tyrosine phosphatase, non-receptor type 7); PTPN9 (Protein tyrosine phosphatase, non-receptor type 9); PTPRA (Protein tyrosine phosphatase, receptor type, alpha polypeptide); PTPRB (Protein tyrosine phosphatase, receptor type, beta polypeptide); PTPRC (Protein tyrosine phosphatase, receptor type, c polypeptide); PTPRD (Protein tyrosine phosphatase, receptor type, D); PTPRF (Protein tyrosine phosphatase, receptor type, F); PTPRG (Protein tyrosine phosphatase, receptor type, gamma polypeptide); PTPRH (Protein tyrosine phosphatase, receptor type, H); PTPRK (Protein tyrosine phosphatase, receptor type, K); PTPRM (Protein tyrosine phosphatase, receptor type, M); PTPRN (Protein tyrosine phosphatase, receptor type, N); PTPRN2 (Protein tyrosine phosphatase, receptor type, N polypeptide 2); PTX3 (Pentaxin-related gene, rapidly induced by IL-1 beta); RAB8IP (Rab8 interacting protein (GC kinase)); RAB8IP (Rab8 interacting protein (GC kinase)); RAD9 (RAD9 (S. pombe) homolog); RASA1 (RAS p21 protein activator (GTPase activating protein) 1); RET (Ret proto-oncogene (multiple endocrine neoplasia MEN2A, MEN2B and medullary thyroid carcinoma 1, Hirschsprung disease)); RGS1 (Regulator of G-protein signalling 1); RGS16 (Regulator of G-protein signalling 16); RGS7 (Regulator of G-protein signalling 7); RHOK (Rhodopsin kinase); ROCK1 (Rho-associated, coiled-coil containing protein kinase 1); RPS6KA2 (Ribosomal protein S6 kinase, 90kD, polypeptide 2); RPS6KB1 (Ribosomal protein S6 kinase, 70kD, polypeptide 1); RSN (Restin (Reed-Steinberg cell-expressed intermediate filament-associated protein)); RYK (RYK receptor-like tyrosine kinase); SAPK3 (Stress-activated protein kinase 3); SELE (Selectin E (endothelial adhesion molecule 1)); SELL (Selectin L (lymphocyte adhesion molecule 1)); SELP (Selectin P (granule membrane protein 140kD, antigen CD62)); SERK1 (SAPK/Erk kinase 1); SFN (Stratifin); SH3D1B (SH3 domain protein 1B); SLC2A3 (Solute carrier family 2 (facilitated glucose transporter), member 3); SLC2A5 (Solute carrier family 2 (facilitated glucose transporter), member 5); SPTA1 (Spectrin, alpha, erythrocytic 1 (elliptocytosis 2)); SPTB (Spectrin, beta, erythrocytic (includes sperocytosis, clinical type I)); SRC (V-src avian sarcoma (Schmidt-Ruppin A-2) viral oncogene homolog); SRF (Serum response factor (c-fos serum response element-binding transcription factor)); SRPK2 (SFRS protein kinase 2); STK11 (Serine/threonine kinase 11 (Peutz-Jeghers syndrome)); STK2 (Serine/threonine kinase 2); STK3 (Serine/threonine kinase 3 (Ste20, yeast homolog)); STK4 (Serine/threonine kinase 4); SYK (Spleen tyrosine kinase); TAP1 (Transporter 1, ABC (ATP binding cassette)); TAPBP (TAP binding protein (tapasin)); TBCC (Tubulin-specific chaperone c); TESK1 (Testis-specific kinase 1); TGFBI (Transforming growth factor, beta-induced, 68kD); THBS1 (Thrombospondin 1); TIAM1 (T-cell lymphoma invasion and metastasis 1); TIEG (TGFB inducible early growth response); TK2 (Thymidine kinase 2, mitochondrial); TNFAIP1 (Tumor necrosis factor, alpha-induced protein 1 (endothelial)); TNFAIP1 (Tumor necrosis factor, alpha-induced protein 1 (endothelial)); TNFSF5 (Tumor necrosis factor (ligand) superfamily, member 5); TTK (TTK protein kinase); TTN (Titin); TUBA1 (Tubulin, alpha 1 (testis specific)); TUBG (Tubulin, gamma polypeptide); TXK (TXK tyrosine kinase); TYK2 (Tyrosine kinase 2); TYRO3 (TYRO3 protein tyrosine kinase); UBE1L (Ubiquitin-activating enzyme E1, like); UBE2A (Ubiquitin-conjugating enzyme E2A (RAD6 homolog)); UBE2B (Ubiquitin-conjugating enzyme E2B (RAD6 homolog)); VASP (Vasodilator-stimulated phosphoprotein); VAV2 (Vav 2 oncogene); VCAM1 (Vascular cell adhesion molecule 1); VCL (Vinculin); VIM (Vimentin); VRK2 (Vaccinia related kinase 2); WAS (Wiskott-Aldrich syndrome (ecezema-thrombocytopenia)); ZAP70 (Zeta-chain (TCR) associated protein kinase (70 kD)); and ZPK (Zipper (leucine) protein kinase).

### Development

ACCPN (Agenesis of corpus callosum and peripheral neuropathy (Andermann); ACVR1 (Activin A receptor, type I); ACVR1B (Activin A receptor, type IB); ACVR2 (Activin A receptor, type II); ACVR2B (Activin A receptor, type IIB); ACVRL1 (Activin A receptor type II-like 1); ADFN (Albinism-deafness syndrome); AES (Amino-terminal enhancer of split); AFD 1 (Acrofacial dysostosis 1, Nager type); AGC 1 (Aggrecan 1 (chondroitin sulfate proteoglycan 1, large aggregating proteoglycan, antigen identified by monoclonal antibody A0122)); AHO2 (Albright hereditary osteodystrophy-2); AIH3 (Amelogenesis imperfecta 3, hypomaturation or hypoplastic type); ALX3 (Aristaless-like homeobox 3); AMCD1 (Arthrogryposis multiplex congenital, distal, type 1); AMCD2B (Arthrogryposis multiplex congenita, distal, type 2B); AMCN (Arthrogryposis multiplex congenita, neurogenic); AMCX1 (Arthrogryposis multiplex congenita, X-linked (spinal muscular atrophy,); AMDM (Acromesomelic dysplasia, Maroteaux type); AMH (Anti-Mullerian hormone); AMHR2 (Anti-Mullerian hormone receptor, type II); AMMECR1 (Alport syndrome, mental retardation, midface hypoplasia and elliptocytosis chromosomal region, gene 1); ANOP1 (Anophthalmos 1 (with mental retardation, without limb anomalies or dental or urogenital abnormalities)); APC (Adenomatosis polyposis coli); ARIX (Aristaless (Drosophila) homeobox); ARVCF (Armadillo repeat gene deletes in velocardiofacial syndrome); ASCL1 (Achaete-scute complex (Drosophila) homolog-like 1); ASCL2 (Achaete-scute complex (Drosophila) homolog-like 2); ASH2L (Ash2 (absent, small, or homeotic, Drosophila, homolog)-like); ASMD (Anterior segment mesenchymal dysgenesis); ATD (Asphixiating thoracic dystrophy (chondroectodermal dysplasia-like syndrome)); ATF4 (Activating transcription factor 4 (tax-responsive enhancer element B67)); AXIN1 (Axin); AXIN2 (Axin 2 (conductin, axil)); BAD (BCL2-antagonist of cell death); BAPX1 (Bagpipe homeobox (Drosophila) homolog 1); BARD1 (BRCA1 associated RING domain 1); BARX2 (BarH-like homeobox 2); BAX (BCL2-associated X protein); BDB1 (Brachydactyly type B1); BDC 0; BDE (Brachydactyly type E); BDMR (Brachydactyly-mental retardation syndrome); BDNF (Brain-derived neurotrophic factor); BMP1 (Bone morphogenetic protein 1); BMP2 (Bone morphogenetic protein 2); BMP3 (Bone morphogenetic protein 3 (osteogenic)); BMP4 (Bone morphogenetic protein 4); BMP5 (Bone morphogenetic protein 5); BMP6 (Bone morphogenetic protein 6); BMP7 (Bone morphogenetic protein 7 (osteogenic protein 1)); BMPR1A (Bone morphogenetic protein receptor, type IA); BMPR1B (Bone morphogenetic protein receptor, type IB); BMPR2 (Bone morphogenetic protein receptor, type II (serine/threonine kinase)); CBFA1 (Core-binding factor, runt domain, alpha subunit 1); CBFA2 (Core-binding factor, runt domain, alpha subunit 2 (acute myeloid leukemia 1; aml1 oncogene)); CBFA3 (Core-binding factor, runt domain, alpha subunit 3); CER1 (Cerberus 1 (Xenopus laevis) homolog (cysteine knot superfamily)); CHH (Cartilage-hair hypoplasia); CHRD (Chordin); CHX10 (C elegans ceh-10 homeo domain-containing homolog); CREB2 (CAMP responsive element binding protein 2); CSH2 (Chorionic somatomammotropin hormone 2); DLK1 (Delta (Drosophila)-like 1); EBAF (Endometrial bleeding associated factor (left-right determination, factor A; transforming growth factor beta superfamily)); EDN1 (Endothelin 1); EDN2 (Endothelin 2); EDN3 (Endothelin 3); EDNRA (Endothelin receptor type A); EDNRB (Endothelin receptor type B); EDR2 (Early development regulator 2 (homolog of polyhomeotic 2)); EED (Embryonic ectoderm development protein); EFNA5 (Ephrin-A5); EN1 (Engrailed homolog 1); EN2 (Engrailed homolog 2); ENG (Endoglin (Osler-Rendu-Weber syndrome 1)); EOMES (Eomesodermin (Xenopus laevis) homolog); EPHA2 (EphA2); EPHB2 (EphB2); FAST-1 (Human homolog of Xenopus forkhead activin signal transducer-1); FGD1 (Faciogenital dysplasia (Aarskog-Scott syndrome)); FGF1 (Fibroblast growth factor 1 (acidic)); FGF10 (Fibroblast growth factor 10); FGF13 (Fibroblast growth factor 13); FGF14 (Fibroblast growth factor 14); FGF16 (Fibroblast growth factor 16); FGF2 (Fibroblast growth factor 2 (basic)); FGF3 (Fibroblast growth factor 3 (murine mammary tumor virus integration site (v-int-2) oncogene homolog)); FGF4 (Fibroblast growth factor 4 (heparin secretory transforming protein 1, Kaposi sarcoma oncogene)); FGF7 (Fibroblast growth factor 7 (keratinocyte growth factor)); FGF8 (Fibroblast growth factor 8 (androgen-induced)); FGFR1 (Fibroblast growth factor receptor 1 (fms-related tyrosine kinase 2, Pfeiffer syndrome)); FGFR2 (Fibroblast growth factor receptor 2 (bacteria-expressed kinase, keratinocyte growth factor receptor, craniofacial dysostosis 1, Crouzon syndrome, Pfeiffer syndrome, Jackson-Weiss syndrome)); FGFR3 (Fibroblast growth factor receptor 3 (achondroplasia, thanatophoric dwarfism)); FGFR4 (Fibroblast growth factor receptor 4); FKHL16 (Forkhead (Drosophila)-like 16); FKHL7 (Forkhead (Drosophila)-like 7); FRZB (Frizzled-related protein); FZD1 (Frizzled (Drosophila) homolog 1); FZD2 (Frizzled (Drosophila) homolog 2); FZD3 (Frizzled (Drosophila) homolog 3); FZD4 (Frizzled (Drosophila) homolog 4); FZD5 (Frizzled (Drosophila) homolog 5); FZD6 (Frizzled (Drosophila) homolog 6); FZD7 (Frizzled (Drosophila) homolog 7); FZD8 (Frizzled (Drosophila) homolog 8); FZD9 (Frizzled (Drosophila) homolog 9); GDF5 (Growth differentiation factor 5 (cartilage-derived morphogenetic protein-1)); GLI (Glioma-associated oncogene homolog (zinc finger protein)); GLI2 (GLI-Kruppel family member GLI2); GLI3 (GLI-Kruppel family member GLI3 (Greig cephalopolysyndactyly syndrome)); GLI4 (GLI-Kruppel family member GLI4); GSC (Goosecoid); GSCL (Goosecoid-like); HESX1 (Homeo box gene expressed in ES cells; Rathke pouch homeo box); HLXB9 (Homeo box HB9); HNF3A (Hepatocyte nuclear factor 3, alpha); HNF3B (Hepatocyte nuclear factor 3, beta); HNF3G (Hepatocyte nuclear factor 3, gamma); HNF4A (Hepatocyte nuclear factor 4, alpha); HNF4B (Hepatocyte nuclear factor 4, beta); HNF4G (Hepatocyte nuclear factor 4, gamma); HNF6A (Hepatocyte nuclear factor 6, alpha); HOX11 (Homeo box 11 (T-cell lymphoma 3-associated breakpoint)); HOXA1 (Homeo box A1); HOXA10 (Homeo box A10); HOXA11 (Homeo box A11); HOXA13 (Homeo box A13); HOXA2 (Homeo box A2); HOXA3 (Homeo box A3); HOXA4 (Homeo box A4); HOXA5 (Homeo box A5); HOXA6 (Homeo box A6); HOXA7 (Homeo box A7); HOXA9 (Homeo box A9); HOXA@ (Homeo box A cluster); HOXB1 (Homeo box B1); HOXB2 (Homeo box B2); HOXB3 (Homeo box B3); HOXB4 (Homeo box B4); HOXB5 (Homeo box B5); HOXB6 (Homeo box B6); HOXB7 (Homeo box B7); HOXB8 (Homeo box B8); HOXB9 (Homeo box B9); HOXC 10 (Homeo box C10); HOXC 11 (Homeo box C11); HOXC 12 (Homeo box C12); HOXC 13 (Homeo box C 13); HOXC4 (Homeo box C4); HOXC5 (Homeo box C5); HOXC6 (Homeo box C6); HOXC8 (Homeo box C8); HOXC9 (Homeo box C9); HOXD1 (Homeo box D1); HOXD10 (Homeo box D10); HOXD11 (Homeo box D11); HOXD12 (Homeo box D12); HOXD13 (Homeo box D13); HOXD3 (Homeo box D3); HOXD4 (Homeo box D4); HOXD8 (Homeo box D8); HOXD9 (Homeo box D9); IHH (Indian hedgehog (Drosophila) homolog); INHBA (Inhibin, beta A (activin A, activin AB alpha polypeptide)); INHBB (Inhibin, beta B (activin AB beta polypeptide)); ISL1 (ISL1 transcription factor, LIM/homeodomain, (islet-1)); JAG1 (Jagged1 (Alagille syndrome)); JAG2 (Jagged 2); LEF1 (Lymphoid enhancer-binding factor 1); LHX1 (LIM homeobox protein 1); LHX2 (LIM HOX gene 2); LHX3 (LIM/homeodomain protein LHX3); LMX1A (LIM homeobox transcription factor 1, alpha); LMX1B (LIM homeobox transcription factor 1, beta); MADH1 (MAD (mothers against decapentaplegic, Drosophila) homolog 1); MADH2 (MAD (mothers against decapentaplegic, Drosophila) homolog 2); MADH3 (MAD (mothers against decapentaplegic, Drosophila) homolog 3); MADH4 (MAD (mothers against decapentaplegic, Drosophila) homolog 4); MADH5 (MAD (mothers against decapentaplegic, Drosophila) homolog 5); MADH6 (MAD (mothers against decapentaplegic, Drosophila) homolog 6); MADH7 (MAD (mothers against decapentaplegic, Drosophila) homolog 7); MADH9 (MAD (mothers against decapentaplegic, Drosophila) homolog 9); MEIS1 (Meis1 (mouse) homolog); MEIS2 (Meis (mouse) homolog 2); MEIS3 (Meis (mouse) homolog 3); MEKK5 (MAP/ERK kinase kinase 5); MLLT1 (Myeloid/lymphoid or mixed-lineage leukemia (trithorax (Drosophila) homolog); translocated to, 1); MSX1 (Msh (Drosophila) homeo box homolog 1 (formerly homeo box 7)); MSX2 (Msh (Drosophila) homeo box homolog 2); NOG (Noggin); ORW2 (Osler-Rendu-Weber syndrome 2); PAX1 (Paired box gene 1); PAX1 (Paired box gene 1); PAX2 (Paired box gene 2); PAX3 (Paired box gene 3 (Waardenburg syndrome 1)); PAX4 (Paired box gene 4); PAX5 (Paired box gene 5 (B-cell lineage specific activator protein)); PAX6 (Paired box gene 6 (aniridia, keratitis)); PAX7 (Paired box gene 7); PAX8 (Paired box gene 8); PAX9 (Paired box gene 9); PBX1 (Pre-B-cell leukemia transcription factor 1); PBX2 (Pre-B-cell leukemia transcription factor 2); PBX3 (Pre-B-cell leukemia transcription factor 3); PITX2 (Paired-like homeodomain transcription factor 2); PKHD1 (Polycystic kidney and hepatic disease 1 (autosomal recessive)); PROP1 (Prophet of Pit1, paired-like homeodomain transcription factor); PTCH (Patched (Drosophila) homolog); PTCH2 (Patched (Drosophila) homolog 2); RARA (Retinoic acid receptor, alpha); RARB (Retinoic acid receptor, beta); RARG (Retinoic acid receptor, gamma); RET (Ret proto-oncogene (multiple endocrine neoplasia MEN2A, MEN2B and medullary thyroid carcinoma 1, Hirschsprung disease)); RIEG2 (Rieger syndrome 2); RXRA (Retinoid X receptor, alpha); RXRB (Retinoid X receptor, beta); RXRG (Retinoid X receptor, gamma); SFRP1 (Secreted frizzled-related protein 1); SFRP2 (Secreted frizzled-related protein 2); SFRP4 (Secreted frizzled-related protein 4); SFRP5 (Secreted frizzled-related protein 5); SHH (Sonic hedgehog (Drosophila) homolog); SMARCA2 (SWI/SNF related, matrix associated, actin dependent regulator of chromatin, subfamily a, member 2); SMARCA4 (SWI/SNF related, matrix associated, actin dependent regulator of chromatin, subfamily a, member 4); SMARCB1 (SWI/SNF related, matrix associated, actin dependent regulator of chromatin, subfamily b, member 1); SMARCC1 (SWI/SNF related, matrix associated, actin dependent regulator of chromatin, subfamily c, member 1); SMARCC2 (SWI/SNF related, matrix associated, actin dependent regulator of chromatin, subfamily c, member 2); SMARCD1 (SWI/SNF related, matrix associated, actin dependent regulator of chromatin, subfamily d, member 1); SMARCD2 (SWI/SNF related, matrix associated, actin dependent regulator of chromatin, subfamily d, member 2); SMARCD3 (SWI/SNF related, matrix associated, actin dependent regulator of chromatin, subfamily d, member 3); SMARCE1 (SWI/SNF related, matrix associated, actin dependent regulator of chromatin, subfamily e, member 1); SMOH (Smoothened (Drosophila) homolog); SOX1 (SRY (sex determining region Y)-box 1); SOX10 (SRY (sex-determining region Y)-box 10); SOX2 (SRY (sex determining region Y)-box 2); SOX9 (SRY (sex-determining region Y)-box 9 (campomelic dysplasia, autosomal sex-reversal)); SRF (Serum response factor (c-fos serum response element-binding transcription factor)); STAT3 (Signal transducer and activator of transcription 3 (acute-phase response factor)); T (T brachyury (mouse) homolog); TBX1 (T-box 1); TBX10 (T-box 10); TBX10 (T-box 10); TBX15 (T-box 15); TBX18 (T-box 18); TBX19 (T-box 19); TBX2 (T-box 2); TBX3 (T-box 3 (ulnar mammary syndrome)); TBX4 (T-box 4); TBX5 (T-box 5); TBX6 (T-box 6); TBX7 (T-box 7); TFAP2A (Transcription factor AP-2 alpha (activating enhancer-binding protein 2 alpha)); TFAP2B (Transcription factor AP-2 beta (activating enhancer-binding protein 2 beta)); TFCOUP2 (Transcription factor COUP 2 (chicken ovalbumin upstream promoter 2, apolipoprotein regulatory protein)); TGFBR1 (Transforming growth factor, beta receptor I (activin A receptor type II-like kinase, 53kD)); TWIST (Twist (Drosophila) homolog); WNT1 (Wingless-type MMTV integration site family, member 1); WNT10B (Wingless-type MMTV integration site family, member 10B); WNT11 (Wingless-type MMTV integration site family, member 11); WNT14 (Wingless-type MMTV integration site family, member 14); WNT15 (Wingless-type MMTV integration site family, member 15); WNT2 (Wingless-type MMTV integration site family member 2); WNT2B (Wingless-type MMTV integration site family, member 2B); WNT3 (Wingless-type MMTV integration site family, member 3); WNT5A (Wingless-type MMTV integration site family, member 5A); WNT6 (Wingless-type MMTV integration site family, member 6); WNT7A (Wingless-type MMTV integration site family, member 7A); WNT7B (Wingless-type MMTV integration site family, member 7B); WNT8A (Wingless-type MMTV integration site family, member 8A); WNT8B (Wingless-type MMTV integration site family, member 8B); ZIC3 (Zic family member 3 (odd-paired Drosophila homolog, heterotaxy 1)); and ZIC3 (Zic family member 3 (odd-paired Drosophila homolog, heterotaxy 1)).

### DNA adducts

DFFA (DNA fragmentation factor, 45 kD, alpha subunit); DNMT1 (DNA (cytosine-5-)-methyltransferase 1); DNMT2 (DNA (cytosine-5-)-methyltransferase 2); IGHMBP2 (Immunoglobulin mu binding protein 2); LIG1 (Ligase I, DNA, ATP-dependent); LIG3 (Ligase III, DNA, ATP-dependent); LIG4 (Ligase IV, DNA, ATP-dependent); MGMT (O-6-methylguanine-DNA methyltransferase); NTHL1 (Nth (E.coli endonuclease III)-like 1); PRIM1 (Primase, polypeptide 1 (49kD)); RAG1 (Recombination activating gene 1); RFC3 (Replication factor C (activator 1) 3 (38kD)); RFC4 (Replication factor C (activator 1) 4 (37kD)); TERT (Telomerase reverse transcriptase); TOP1 (Topoisomerase (DNA) I); TOP2A (Topoisomerase (DNA) II alpha (170kD)); TOP2B (Topoisomerase (DNA) II beta (180kD)); TOP3 (Topoisomerase (DNA) III); and TOP3B (Topoisomerase (DNA) III beta).

### DNA damage

ADPRT (ADP-ribosyltransferase (NAD+; poly (ADP-ribose) polymerase)); APEX (APEX nuclease (multifunctional DNA repair enzyme)); ATM (Ataxia telangiectasia mutated (includes complementation groups A, C and D)); BLM (Bloom syndrome); BRCA1 (Breast cancer 1, early onset); BRCA2 (Breast cancer 2, early onset); CKN1 (Cockayne syndrome 1 (classical)); DDB1 (Damage-specific DNA binding protein 1 (127kD)); DDB2 (Damage-specific DNA binding protein 2 (48kD)); DDIT1 (DNA-damage-inducible transcript 1); DDIT1L (DNA-damage-inducible transcript 1-like); DDIT3 (DNA-damage-inducible transcript 3); DFFA (DNA fragmentation factor, 45 kD, alpha subunit); DNMT1 (DNA (cytosine-5-)-methyltransferase 1); DNMT2 (DNA (cytosine-5-)-methyltransferase 2); ERCC1 (Excision repair cross-complementing rodent repair deficiency, complementation group 1 (includes overlapping antisense sequence)); ERCC2 (Excision repair cross-complementing rodent repair deficiency, complementation group 2 (xeroderma pigmentosum D)); ERCC3 (Excision repair cross-complementing rodent repair deficiency, complementation group 3 (xeroderma pigmentosum group B complementing)); ERCC5 (Excision repair cross-complementing rodent repair deficiency, complementation group 5 (xeroderma pigmentosum, complementation group G (Cockayne syndrome))); ERCC6 (Excision repair cross-complementing rodent repair deficiency, complementation group 6); FANCA (Fanconi anemia, complementation group A); FANCB (Fanconi anemia, complementation group B); FANCC (Fanconi anemia, complementation group C); FANCG (Fanconi anemia, complementation group G); G22P1 (Thyroid autoantigen 70kD (Ku antigen)); GTBP (G/T mismatch-binding protein); IGHMBP2 (Immunoglobulin mu binding protein 2); INPPL1 (Inositol polyphosphate phosphatase-like 1); LIG1 (Ligase I, DNA, ATP-dependent); LIG3 (Ligase III, DNA, ATP-dependent); LIG3 (Ligase III, DNA, ATP-dependent); LIG4 (Ligase IV, DNA, ATP-dependent); MGMT (O-6-methylguanine-DNA methyltransferase); MLH1 (MutL (E. coli) homolog 1 (colon cancer, nonpolyposis type 2)); MPG (N-methylpurine-DNA glycosylase); MSH2 (MutS (E. coli) homolog 2 (colon cancer, nonpolyposis type 1)); MSH3 (MutS (E. coli) homolog 3); MSH4 (MutS (E. coli) homolog 4); MSH5 (MutS (E. coli) homolog 5); MTH1 (MutT (E. coli) human homolog (8-oxo-7,8-dihydroguanosine triphosphatase)); NTHL1 (Nth (E.coli endonuclease III)-like 1); PMS1 (Postmeiotic segregation increased (S. cerevisiae) 1); PMS2 (Postmeiotic segregation increased (S. cerevisiae) 2); PMS2L1 (Postmeiotic segregation increased 2-like 1); PMS2L11 (Postmeiotic segregation increased 2-like 11); PMS2L12 (Postmeiotic segregation increased 2-like 12); PMS2L2 (Postmeiotic segregation increased 2-like 2); PMS2L3 (Postmeiotic segregation increased 2-like 3); PMS2L4 (Postmeiotic segregation increased 2-like 4); PMS2L5 (Postmeiotic segregation increased 2-like 5); PMS2L6 (Postmeiotic segregation increased 2-like 6); PMS2L8 (Postmeiotic segregation increased 2-like 8); PMS2L9 (Postmeiotic segregation increased 2-like 9); POLB (Polymerase (DNA directed), beta); PRIM1 (Primase, polypeptide 1 (49kD)); PRKDC (Protein kinase, DNA-activated, catalytic polypeptide); RAD1 (RAD1 (S. pombe) homolog); RAD17 (RAD17 (S. pombe) homolog); RAD21 (RAD21 (S. pombe) homolog); RAD23A (RAD23 (S. cerevisiae) homolog A); RAD23B (RAD23 (S. cerevisiae) homolog B); RAD51 (RAD51 (S. cerevisiae) homolog (E coli RecA homolog)); RAD51C (RAD51 (S. cerevisiae) homolog C); RAD51L1 (RAD51 (S. cerevisiae)-like 1); RAD51L3 (RAD51 (S. cerevisiae)-like 3); RAD52 (RAD52 (S. cerevisiae) homolog); RAD54L (RAD54 (S.cerevisiae)-like); RAG1 (Recombination activating gene 1); RFC3 (Replication factor C (activator 1) 3 (38kD)); RFC4 (Replication factor C (activator 1) 4 (37kD)); TDG (Thymine-DNA glycosylase); TERT (Telomerase reverse transcriptase); TOP1 (Topoisomerase (DNA) I); TOP2A (Topoisomerase (DNA) II alpha (170kD)); TOP2B (Topoisomerase (DNA) II beta (180kD)); TOP3 (Topoisomerase (DNA) III); TOP3B (Topoisomerase (DNA) III beta); TP53 (Tumor protein p53 (Li-Fraumeni syndrome)); TRLP1 (TRNA leucine (AAG) pseudogene 1); UNG (Uracil-DNA glycosylase); WRN (Werner syndrome); XPA (Xeroderma pigmentosum, complementation group A); XPC (Xeroderma pigmentosum, complementation group C); XRCC1 (X-ray repair complementing defective repair in Chinese hamster cells 1); XRCC2 (X-ray repair complementing defective repair in Chinese hamster cells 2); XRCC3 (X-ray repair complementing defective repair in Chinese hamster cells 3); XRCC4 (X-ray repair complementing defective repair in Chinese hamster cells 4); XRCC5 (X-ray repair complementing defective repair in Chinese hamster cells 5 (double-strand-break rejoining; Ku autoantigen, 80kD)); and XRCC8 (X-ray repair complementing defective repair in Chinese hamster cells 8).

### DNA replication

ADAR (Adenosine deaminase, RNA-specific); ADPRT (ADP-ribosyltransferase (NAD+; poly (ADP-ribose) polymerase)); ATM (Ataxia telangiectasia mutated (includes complementation groups A, C and D)); ATR (Ataxia telangiectasia and Rad3 related); ATRX (Alpha thalassemia/mental retardation syndrome X-linked); BLM (Bloom syndrome); CENPB (Centromere protein B (80kD)); CENPC1 (Centromere protein C 1); CHD4 (Chromodomain helicase DNA binding protein 4); CHDL (Chromodomain-helicase-DNA-binding protein); CKN1 (Cockayne syndrome 1 (classical)); DNA2L (DNA2 (DNA replication helicase, yeast, homolog)-like); DNASE1 (Deoxyribonuclease I); DNASE1L1 (Deoxyribonuclease I-like 1); DNASE1L2 (Deoxyribonuclease I-like 2); ERCC1 (Excision repair cross-complementing rodent repair deficiency, complementation group 1 (includes overlapping antisense sequence)); ERCC2 (Excision repair cross-complementing rodent repair deficiency, complementation group 2 (xeroderma pigmentosum D)); ERCC3 (Excision repair cross-complementing rodent repair deficiency, complementation group 3 (xeroderma pigmentosum group B complementing)); ERCC4 (Excision repair cross-complementing rodent repair deficiency, complementation group 4); ERCC5 (Excision repair cross-complementing rodent repair deficiency, complementation group 5 (xeroderma pigmentosum, complementation group G (Cockayne syndrome))); ERCC6 (Excision repair cross-complementing rodent repair deficiency, complementation group 6); EXO1 (Exonuclease 1); FEN1 (Flap structure-specific endonuclease 1); G22P1 (Thyroid autoantigen 70kD (Ku antigen)); HMGIY (High-mobility group (nonhistone chromosomal) protein isoforms I and Y); HUS 1 (HUS1 (S. pombe) checkpoint homolog); LIG2 (Ligase II, DNA, ATP-dependent); MLH1 (MutL (E. coli) homolog 1 (colon cancer, nonpolyposis type 2)); MSH5 (MutS (E. coli) homolog 5); POLA (Polymerase (DNA directed), alpha); POLB (Polymerase (DNA directed), beta); POLD1 (Polymerase (DNA directed), delta 1, catalytic subunit (125kD)); RAD1 (RAD1 (S. pombe) homolog); RAD50 (RAD50 (S. cerevisiae) homolog); RAD51 (RAD51 (S. cerevisiae) homolog (E coli RecA homolog)); RAD51C (RAD51 (S. cerevisiae) homolog C); RAD52 (RAD52 (S. cerevisiae) homolog); RPA1 (Replication protein A1 (70kD)); RPA2 (Replication protein A2 (32kD)); RPA3 (Replication protein A3 (14kD)); RPA40 (RNA polymerase I subunit); SNRPA (Small nuclear ribonucleoprotein polypeptide A); SNRPA1 (Small nuclear ribonucleoprotein polypeptide A'); TOP1 (Topoisomerase (DNA) I); TOP2A (Topoisomerase (DNA) II alpha (170kD)); TOP3 (Topoisomerase (DNA) III); TOP3B (Topoisomerase (DNA) III beta); TR4 (TR4 nuclear hormone receptor); WRN (Werner syndrome); XPA (Xeroderma pigmentosum, complementation group A); XRCC1 (X-ray repair complementing defective repair in Chinese hamster cells 1); XRCC2 (X-ray repair complementing defective repair in Chinese hamster cells 2); XRCC3 (X-ray repair complementing defective repair in Chinese hamster cells 3); XRCC4 (X-ray repair complementing defective repair in Chinese hamster cells 4); and XRCC5 (X-ray repair complementing defective repair in Chinese hamster cells 5 (double-strand-break rejoining; Ku autoantigen, 80kD)).

### Gene regulation

ADA (Adenosine deaminase); ADPRT (ADP-ribosyltransferase (NAD+; poly (ADP-ribose) polymerase)); ADSS (Adenylosuccinate synthase); AHR (Aryl hydrocarbon receptor); ATBF1 (AT-binding transcription factor 1); ATF3 (Activating transcription factor 3); BARD1 (BRCA1 associated RING domain 1); CBF2 (CCAAT-box-binding transcription factor); CBFA2 (Core-binding factor, runt domain, alpha subunit 2 (acute myeloid leukemia 1; aml1 oncogene)); CBFA3 (Core-binding factor, runt domain, alpha subunit 3); CBFB (Core-binding factor, beta subunit); CEBPA (CCAAT/enhancer binding protein (C/EBP), alpha); CEBPB (CCAAT/enhancer binding protein (C/EBP), beta); CEBPD (CCAAT/enhancer binding protein (C/EBP), delta); CEBPE (CCAAT/enhancer binding protein (C/EBP), epsilon); CEBPG (CCAAT/enhancer binding protein (C/EBP), gamma); CENPE (Centromere protein E (312kD)); CHD1 (Chromodomain helicase DNA binding protein 1); CHD2 (Chromodomain helicase DNA binding protein 2); CHD3 (Chromodomain helicase DNA binding protein 3); CHDL (Chromodomain-helicase-DNA-binding protein); CREB2 (CAMP responsive element binding protein 2); CREBBP (CREB binding protein (Rubinstein-Taybi syndrome)); CSE1L (Chromosome segregation 1 (yeast homolog)-like); CSTF3 (Cleavage stimulation factor, 3' pre-RNA, subunit 3, 77kD); CTPS (CTP synthase); DCK (Deoxycytidine kinase); DCTD (DCMP deaminase); DGUOK (Deoxyguanosine kinase); DNASE1 (Deoxyribonuclease I); DNASE1L3 (Deoxyribonuclease I-like 3); DUT (DUTP pyrophosphatase); DXS423E (Segregation of mitotic chromosomes 1 (SMC1, yeast human homolog of;); E2F2 (E2F transcription factor 2); E2F5 (E2F transcription factor 5, p130-binding); EEF1A1 (Eukaryotic translation elongation factor 1 alpha 1); EEF2 (Eukaryotic translation elongation factor 2); EGR1 (Early growth response 1); EIF2B (Eukaryotic translation initiation factor 2B, subunit 1 (alpha, 26kD)); EIF3S6 (Eukaryotic translation initiation factor 3, subunit 6 (48kD)); EIF4E (Eukaryotic translation initiation factor 4E); EIF4G2 (Eukaryotic translation initiation factor 4 gamma, 2); ELF1 (E74-like factor 1 (ets domain transcription factor)); ELF3 (E74-like factor 3 (ets domain transcription factor)); ELK4 (ELK4, ETS-domain protein (SRF accessory protein 1) NOTE: Symbol and name provisional); EP300 (E1A binding protein p300); ESR1 (Estrogen receptor 1); ETV3 (Ets variant gene 3); ETV4 (Ets variant gene 4 (E1A enhancer-binding protein, E1AF)); ETV5 (Ets variant gene 5 (ets-related molecule)); FKHR (Forkhead (Drosophila) homolog 1 (rhabdomyosarcoma)); FLI1 (Friend leukemia virus integration 1); GART (Phosphoribosylglycinamide formyltransferase, phosphoribosylglycinamide synthetase, phosphoribosylaminoimidazole synthetase); GATA1 (GATA-binding protein 1 (globin transcription factor 1)); GATA2 (GATA-binding protein 2); GATA3 (GATA-binding protein 3); GATA4 (GATA-binding protein 4); GLI (Glioma-associated oncogene homolog (zinc finger protein)); GRSF1 (G-rich RNA sequence binding factor 1); H4FI (H4 histone family, member I); HIF1A (Hypoxia-inducible factor 1, alpha subunit (basic helix-loop-helix transcription factor)); HIVEP1 (Human immunodeficiency virus type I enhancer-binding protein 1); HLF (Hepatic leukemia factor); HMG17 (High-mobility group (nonhistone chromosomal) protein 17); HMG2 (High-mobility group (nonhistone chromosomal) protein 2); HNRPK (Heterogeneous nuclear ribonucleoprotein K); ICSBP1 (Interferon consensus sequence binding protein 1); ID1 (Inhibitor of DNA binding 1, dominant negative helix-loop-helix protein); ID2 (Inhibitor of DNA binding 2, dominant negative helix-loop-helix protein); ID3 (Inhibitor of DNA binding 3, dominant negative helix-loop-helix protein); ID4 (Inhibitor of DNA binding 4, dominant negative helix-loop-helix protein); IRF1 (Interferon regulatory factor 1); IRF2 (Interferon regulatory factor 2); IRF4 (Interferon regulatory factor 4); IRF5 (Interferon regulatory factor 5); IRF7 (Interferon regulatory factor 7); JUN (V-jun avian sarcoma virus 17 oncogene homolog); JUND (Jun D proto-oncogene); LAF4 (Lymphoid nuclear protein related to AF4); LYL1 (Lymphoblastic leukemia derived sequence 1); MAFG (V-maf musculoaponeurotic fibrosarcoma (avian) oncogene family, protein G); MAX (MAX protein); MDM2 (Mouse double minute 2, human homolog of; p53-binding protein); MHC2TA (MHC class II transactivator); MKI67 (Antigen identified by monoclonal antibody Ki-67); MNDA (Myeloid cell nuclear differentiation antigen); MSX1 (Msh (Drosophila) homeo box homolog 1 (formerly homeo box 7)); MTHFD (5,10-methylenetetrahydrofolate dehydrogenase, 5,10-methylenetetrahydrofolate cyclohydrolase, 10-formyltetrahydrofolate synthetase); MYC (V-myc avian myelocytomatosis viral oncogene homolog); NCBP (Nuclear cap binding protein, 80kD); NCBP (Nuclear cap binding protein, 80kD); NDP52 (Nuclear domain 10 protein); NFE2 (Nuclear factor (erythroid-derived 2), 45kD); NFKB 1 (Nuclear factor of kappa light polypeptide gene enhancer in B-cells 1 (p105)); NFKB2 (Nuclear factor of kappa light polypeptide gene enhancer in B-cells 2 (p49/p100)); NFYA (Nuclear transcription factor Y, alpha); NP (Nucleoside phosphorylase); NUMA1 (Nuclear mitotic apparatus protein 1); ODC1 (Ornithine decarboxylase 1); PAX3 (Paired box gene 3 (Waardenburg syndrome 1)); PBX1 (Pre-B-cell leukemia transcription factor 1); PBX3 (Pre-B-cell leukemia transcription factor 3); PCNA (Proliferating cell nuclear antigen); PEX6 (Peroxisomal biogenesis factor 6); PML (Promyelocytic leukemia); POU2AF1 (POU domain, class 2, associating factor 1); POU2F1 (POU domain, class 2, transcription factor 1); POU2F2 (POU domain, class 2, transcription factor 2); PPAT (Phosphoribosyl pyrophosphate amidotransferase); PRPS1 (Phosphoribosyl pyrophosphate synthetase 1); PTB (Polypyrimidine tract binding protein (heterogeneous nuclear ribonucleoprotein I)); RANBP2 (RAN binding protein 2); RARB (Retinoic acid receptor, beta); RARG (Retinoic acid receptor, gamma); RECQL (RecQ protein-like (DNA helicase Q1-like)); RENBP (Renin-binding protein); RPL27 (Ribosomal protein L27); RPL32 (Ribosomal protein L32); RPL5 (Ribosomal protein L5); RPS16 (Ribosomal protein S16); RPS21 (Ribosomal protein S21); RPS5 (Ribosomal protein S5); RPS9 (Ribosomal protein S9); RXRA (Retinoid X receptor, alpha); SATB 1 (Special AT-rich sequence binding protein 1 (binds to nuclear matrix/scaffold-associating DNA's)); SFRS7 (Splicing factor, arginine/serine-rich 7 (35kD)); SKIL (SKI-like); SMARCA2 (SWI/SNF related, matrix associated, actin dependent regulator of chromatin, subfamily a, member 2); SOX4 (SRY (sex determining region Y)-box 4); SP100 (Nuclear antigen Sp 100); SPIB (Spi-B transcription factor (Spi-1/PU.1 related)); SRF (Serum response factor (c-fos serum response element-binding transcription factor)); STAT3 (Signal transducer and activator of transcription 3 (acute-phase response factor)); STAT4 (Signal transducer and activator of transcription 4); STAT5A (Signal transducer and activator of transcription 5A); TAF2C2 (TATA box binding protein (TBP)-associated factor, RNA polymerase II, C2, 105kD); TAL2 (T-cell acute lymphocytic leukemia 2); TCEB1L (Transcription elongation factor B (SIII), polypeptide 1-like); TCF12 (Transcription factor 12 (HTF4, helix-loop-helix transcription factors 4)); TCF3 (Transcription factor 3 (E2A immunoglobulin enhancer binding factors E12/E47)); TCF7 (Transcription factor 7 (T-cell specific, HMG-box)); TFAP2B (Transcription factor AP-2 beta (activating enhancer-binding protein 2 beta)); TFAP4 (Transcription factor AP-4 (activating enhancer-binding protein 4)); TFDP2 (Transcription factor Dp-2 (E2F dimerization partner 2)); THRA (Thyroid hormone receptor, alpha (avian erythroblastic leukemia viral (v-erb-a) oncogene homolog)); TIAL1 (TIA1 cytotoxic granule-associated RNA-binding protein-like 1); TK1 (Thymidine kinase 1, soluble); TOP1 (Topoisomerase (DNA) I); TOP2B (Topoisomerase (DNA) II beta (180kD)); TP53 (Tumor protein p53 (Li-Fraumeni syndrome)); UBL1 (Ubiquitin-like 1 (sentrin)); WT1 (Wilms tumor 1); XPO1 (Exportin 1 (CRM1, yeast, homolog)); ZFP36 (Zinc finger protein homologous to Zfp-36 in mouse); ZNF173 (Zinc finger protein 173); ZNF200 (Zinc finger protein 200); ZNF42 (Zinc finger protein 42 (myeloid-specific retinoic acid- responsive));

### Immunology

A1BG (Alpha-1-B glycoprotein); A2M (Alpha-2-macroglobulin); AAA (Achalasia-addisonianism-alacrimia syndrome (Allgrove syndrome); AABT (Beta-amino acids, renal transport of); AACT (Alpha-1-antichymotrypsin); AARS (Alanyl-tRNA synthetase); ABAT (4-aminobutyrate aminotransferase); ABC7 (ATP-binding cassette 7); ABO (ABO blood group (transferase A, alpha); 1-3-N-acetylgalactosaminyltransferase; transferase B, alpha 1-3-galactosyltransferase);ACADL (Acyl-Coenzyme A dehydrogenase, long chain); ACADM (Acyl-Coenzyme A dehydrogenase, C-4 to C-12 straight chain); ACADS (Acyl-Coenzyme A dehydrogenase, C-2 to C-3 short chain); ACAT2 (Acetyl-Coenzyme A acetyltransferase 2 (acetoacetyl Coenzyme A thiolase)); ACHE (Acetylcholinesterase (YT blood group)); ACLS (Acrocallosal syndrome); ACO1 (Aconitase 1, soluble); ACO2 (Aconitase 2, mitochondrial); ACP1 (Acid phosphatase 1, soluble); ACP5 (Acid phosphatase 5, tartrate resistant); ACTC (Actin, alpha, cardiac muscle); ACTG2 (Actin, gamma 2, smooth muscle, enteric); ACTN2 (Actinin, alpha 2); ACUG (Arthrocutaneouveal granulomatosis (Blau syndrome)); ACY1 (Aminoacylase 1); ADA (Adenosine deaminase); ADAM10 (A disintegrin and metalloprotease domain 10); ADAM8 (A disintegrin and metalloprotease domain 8); ADCYAP1 (Adenylate cyclase activating polypeptide 1 (pituitary)); ADD1 (Adducin 1 (alpha)); ADH1 (Alcohol dehydrogenase 1 (class I), alpha polypeptide); ADH2 (Alcohol dehydrogenase 2 (class I), beta polypeptide); ADH3 (Alcohol dehydrogenase 3 (class I), gamma polypeptide); ADH4 (Alcohol dehydrogenase 4 (class II), pi polypeptide); ADH5 (Alcohol dehydrogenase 5 (class III), chi polypeptide); ADH5 (Alcohol dehydrogenase 5 (class III), chi polypeptide); ADK (Adenosine kinase); ADORA1 (Adenosine A1 receptor); ADORA2A (Adenosine A2a receptor); ADORA2A (Adenosine A2a receptor); ADPRT (ADP-ribosyltransferase (NAD+; poly (ADP-ribose) polymerase)); ADRA2A (Adrenergic, alpha-2A-, receptor); ADRA2B (Adrenergic, alpha-2B-, receptor); ADRA2C (Adrenergic, alpha-2C-, receptor); ADRB1 (Adrenergic, beta-1-, receptor); ADRB2 (Adrenergic, beta-2-, receptor, surface); ADRB3 (Adrenergic, beta-3-, receptor); ADSS (Adenylosuccinate synthase); AFP (Alpha-fetoprotein); AGA (Aspartylglucosaminidase); AGMX2 (Agammaglobulinemia, X-linked 2 (with growth hormone deficiency)); AGT (Angiotensinogen); AGTR1 (Angiotensin receptor 1); AGTR2 (Angiotensin receptor 2); AGXT (Alanine-glyoxylate aminotransferase (oxalosis I; hyperoxaluria I; glycolicaciduria; serine-pyruvate aminotransferase)); AHCY (S-adenosylhomocysteine hydrolase); AHR (Aryl hydrocarbon receptor); AHSG (Alpha-2-HS-glycoprotein); AIH2 (Amelogenesis imperfecta 2, hypocalcification (autosomal dominant)); AIRE (Autoimmune regulator (automimmune) polyendocrinopathy candidiasis ectodermal dystrophy)); AK1 (Adenylate kinase 1); AKT1 (V-akt murine thymoma viral oncogene homolog 1); ALAD (Aminolevulinate, delta-, dehydratase); ALAS2 (Aminolevulinate, delta-, synthase 2) (sideroblastic/hypochromic anemia)); ALB (Albumin); ALD (Adrenoleukodystrophy/adrenomyeloneuropathy); ALDH1 (Aldehyde dehydrogenase 1, soluble); ALDH2 (Aldehyde dehydrogenase 2, mitochondrial); ALDH6 (Aldehyde dehydrogenase 6); ALDH9 (Aldehyde dehydrogenase 9 (gamma-aminobutyraldehyde dehydrogenase, E3 isozyme)); ALDOA (Aldolase A, fructose-bisphosphate); ALDOB (Aldolase B, fructose-bisphosphate); ALOX12 (Arachidonate 12-lipoxygenase); ALOX5 (Arachidonate 5-lipoxygenase); ALPL (Alkaline phosphatase, liver/bone/kidney); ALPP (Alkaline phosphatase, placental (Regan isozyme)); ALPPL2 (Alkaline phosphatase, placental-like 2); AMBP (Alpha-1-microglobulin/bikunin precursor); AMCN (Arthrogryposis multiplex congenita, neurogenic); AMELX (Amelogenin (X chromosome, amelogenesis imperfecta 1)); AMH (Anti-Mullerian hormone); AMPH (Amphiphysin (Stiff-Mann syndrome with breast cancer 128kD autoantigen)); AMPHL (Amphiphysin-like); AMY1A (Amylase, alpha 1A; salivary); AMY2A (Amylase, alpha 2A; pancreatic); ANK1 (Ankyrin 1, erythrocytic); ANPEP (Alanyl (membrane) aminopeptidase (aminopeptidase N, aminopeptidase M, microsomal aminopeptidase, CD13, p150)); ANX11 (Annexin XI (56kD autoantigen)); ANX3 (Annexin III (lipocortin III, 1,2-cyclic-inositol-phosphate phosphodiesterase, placental anticoagulant protein III, calcimedin 35-alpha)); ANX4 (Annexin IV (placental anticoagulant protein II)); ANX5 (Annexin V (endonexin II)); ANX8 (Annexin VIII); APAF1 (Apoptotic protease activating factor); APBA2 (Amyloid beta (A4) precursor protein-binding, family A, member 2 (X11-like)); APBB1 (Amyloid beta (A4) precursor protein-binding, family B, member 1 (Fe65)); APC (Adenomatosis polyposis coli); APCS (Amyloid P component, serum); APEH (N-acylaminoacyl-peptide hydrolase); API1 (Apoptosis inhibitor 1); API2 (Apoptosis inhibitor 2); API3 (Apoptosis inhibitor 3); API4 (Apoptosis inhibitor 4 (survivin)); APOA1 (Apolipoprotein A-I); APOA2 (Apolipoprotein A-II); APOA4 (Apolipoprotein A-IV); APOB (Apolipoprotein B (including Ag(x) antigen)); APOC2 (Apolipoprotein C-II); APOC3 (Apolipoprotein C-III); APOD (Apolipoprotein D); APOE (Apolipoprotein E); APOH (Apolipoprotein H (beta-2-glycoprotein I)); APP (Amyloid beta (A4) precursor protein (protease nexin-II, Alzheimer disease)); APRT (Adenine phosphoribosyltransferase); AQP1 (Aquaporin 1 (channel-forming integral protein, 28kD)); AQP2 (Aquaporin 2 (collecting duct)); AR (Androgen receptor (dihydrotestosterone receptor; testicular feminization; spinal and bulbar muscular atrophy; Kennedy disease)); AREG (Amphiregulin (schwannoma-derived growth factor)); ARHE (Ras homolog gene family, member E); ARHGAP1 (Rho GTPase activating protein 1); ARHGAP5 (Rho GTPase activating protein 5); ARNT (Aryl hydrocarbon receptor nuclear translocator); ARNTL (Aryl hydrocarbon receptor nuclear translocator-like); ARSA (Arylsulfatase A); ART1 (ADP-ribosyltransferase 1); ARVD2 (Arrhythmogenic right ventricular dysplasia 2); ASL (Argininosuccinate lyase); ASNS (Asparagine synthetase); ASPH (Aspartate beta-hydroxylase); AT3 (Antithrombin III); ATCAY (Ataxia, cerebellar, Cayman type); ATM (Ataxia telangiectasia mutated (includes complementation) groups A, C and D)); ATOX1 (ATX1 (antioxidant protein 1, yeast) homolog 1); ATP1A1 (ATPase, Na+/K+ transporting, alpha 1 polypeptide); ATP2B2 (ATPase, Ca++ transporting, plasma membrane 2); ATP4B (ATPase, H+/K+ exchanging, beta polypeptide); ATP7B (ATPase, Cu++ transporting, beta polypeptide (Wilson disease)); ATR (Ataxia telangiectasia and Rad3 related); ATRN (Attractin (with dipeptidylpeptidase IV activity)); ATRX (Alpha thalassemia/mental retardation syndrome X-linked); ATSV (Axonal transport of synaptic vesicles); AUH (AU RNA-binding protein/enoyl-Coenzyme A hydratase); AVP (Arginine vasopressin (neurophysin II, antidiuretic hormone, diabetes insipidus, neurohypophyseal)); AVPR1A (Arginine vasopressin receptor 1A); AVPR2 (Arginine vasopressin receptor 2 (nephrogenic diabetes insipidus)); AXL (AXL receptor tyrosine kinase); AZGP1 (Alpha-2-glycoprotein 1, zinc); AZU1 (Azurocidin 1 (cationic antimicrobial protein 37)); B120 (Brain protein 120); B2M (Beta-2-microglobulin); BAG1 (BCL2-associated athanogene); BAK1 (BCL2-antagonist/killer 1); BAX (BCL2-associated X protein); BCHE (Butyrylcholinesterase); BCKDHA (Branched chain keto acid dehydrogenase E1, alpha polypeptide (maple syrup urine disease)); BCL1 (B-cell CLL/lymphoma 1); BCL2 (B-cell CLL/lymphoma 2); BCL2A1 (BCL2-related protein A1); BCL2L1; BCL2L2 (BCL2-like 2); BCL7 (B-cell CLL/lymphoma 7); BCPM (Benign chronic pemphigus (Hailey-Hailey disease)); BCPR (Breast cancer-related regulator of TP53); BCR (Breakpoint cluster region); BDKRB1 (Bradykinin receptor B1); BDKRB2 (Bradykinin receptor B2); BENE (BENE protein); BF (B-factor, properdin); BGLAP (Bone gamma-carboxyglutamate (gla) protein (osteocalcin)); BGP (Biliary glycoprotein); BHR1 (Bronchial hyperresponsiveness-1 (bronchial asthma)); BID (BH3 interacting domain death agonist); BLK (B lymphoid tyrosine kinase); BLM (Bloom syndrome); BLMH (Bleomycin hydrolase); BMI1 (Murine leukemia viral (bmi-1) oncogene homolog); BN51T (BN51 (BHK21) temperature sensitivity complementing); BPAG1 (Bullous pemphigoid antigen 1 (230/240kD)); BPI (Bactericidal/permeability-increasing protein); BRAF (V-raf murine sarcoma viral oncogene homolog B1); BRAK (CXC chemokine in breast and kidney); BRCA1 (Breast cancer 1, early onset); BRCA2 (Breast cancer 2, early onset); BSEP (Bile salt export pump (ABC member 16, MDR/TAP subfamily)); BST1 (Bone marrow stromal cell antigen 1); BTD (Biotinidase); BTK (Bruton agammaglobulinemia tyrosine kinase); BUB 1 (Budding uninhibited by benzimidazoles 1 (yeast homolog)); C1NH (Complement component 1 inhibitor (angioedema, hereditary)); C1QA (Complement component 1, q subcomponent, alpha polypeptide); C1QB (Complement component 1, q subcomponent, beta Polypeptide); C1QBP (Complement component 1, q subcomponent binding protein); C1QG (Complement component 1, q subcomponent, gamma polypeptide); C1R (Complement component 1, r subcomponent); C1S (Complement component 1, s subcomponent); C2 (Complement component 2); C3 (Complement component 3); C3AR1 (Complement component 3a receptor 1); C4A (Complement component 4A); C4B (Complement component 4B); C4BPA (Complement component 4-binding protein, alpha); C4BPAL2 (Complement component 4-binding protein, alpha-like 2); C4BPB (Complement component 4-binding protein, beta); C5 (Complement component 5); C5R1 (Complement component 5 receptor 1 (C5a ligand)); C6 (Complement component 6); C7 (Complement component 7); C8A (Complement component 8, alpha polypeptide); C8B (Complement component 8, beta polypeptide); C8G (Complement component 8, gamma polypeptide); C9 (Complement component 9); CA1 (Carbonic anhydrase I); CA2 (Carbonic anhydrase II); CACNA1S (Calcium channel, voltage-dependent, L type, alpha 1S subunit); CACNB2 (Calcium channel, voltage-dependent, beta 2 subunit); CALCA (Calcitonin/calcitonin-related polypeptide, alpha); CALCR (Calcitonin receptor); CAMP (Cathelicidin antimicrobial peptide); CAPG (Capping protein (actin filament), gelsolin-like); CAPN3 (Calpain, large polypeptide L3); CASP1 (Caspase 1, apoptosis-related cysteine protease (interleukin 1, beta, convertase)); CASP10 (Caspase 10, apoptosis-related cysteine protease); CASP2 (Caspase 2, apoptosis-related cysteine protease (neural precursor cell expressed, developmentally down-regulated 2)); CASP3 (Caspase 3, apoptosis-related cysteine protease); CASP4 (Caspase 4, apoptosis-related cysteine protease); CASP5 (Caspase 5, apoptosis-related cysteine protease); CASP6 (Caspase 6, apoptosis-related cysteine protease); CASP7 (Caspase 7, apoptosis-related cysteine protease); CASP8 (Caspase 8, apoptosis-related cysteine protease); CASP9 (Caspase 9, apoptosis-related cysteine protease); CASR (Calcium-sensing receptor (hypocalciuric hypercalcemia 1, severe neonatal hyperparathyroidism)); CAT (Catalase); CAV3 (Caveolin 3); CBBM (Blue-monochromatic colorblindness (blue cone monochromacy)); CBFA2 (Core-binding factor, runt domain, alpha subunit 2 (acute myeloid leukemia 1; aml1 oncogene)); CBFA2T1 (Core-binding factor, runt domain, alpha subunit 2; translocated to, 1; cyclin D-related); CBFA2T2 (Core-binding factor, runt domain, alpha subunit 2; translocated to, 2); CBFA2T3 (Core-binding factor, runt domain, alpha subunit 2; translocated to, 3); CBFB (Core-binding factor, beta subunit); CBLN1 (Cerebellin 1 precursor); CBR1 (Carbonyl reductase 1); CBS (Cystathionine-beta-synthase); CBX4 (Chromobox homolog 4 (Drosophila Pc class)); CCAL1 (Chondrocalcinosis 1 (calcium pyrophosphate-deposition disease, early onset osteoarthritis)); CCR1 (Chemokine (C-C motif) receptor 1); CCR1 (Chemokine (C-C motif) receptor 1); CCR2 (Chemokine (C-C motif) receptor 2); CCR3 (Chemokine (C-C motif) receptor 3); CCR3 (Chemokine (C-C motif) receptor 3); CCR4 (Chemokine (C-C motif) receptor 4); CCR5 (Chemokine (C-C motif) receptor 5); CCR5 (Chemokine (C-C motif) receptor 5); CCR6 (Chemokine (C-C motif) receptor 6); CCR7 (Chemokine (C-C motif) receptor 7); CCR7 (Chemokine (C-C motif) receptor 7); CCR8 (Chemokine (C-C motif) receptor 8); CD14 (CD14 antigen); CD151 (CD 151 antigen); CD 19 (CD 19 antigen); CD1A (CD1A antigen, a polypeptide); CD1B (CD1B antigen, b polypeptide); CD2 (CD2 antigen (p50), sheep red blood cell receptor); CD20 (CD20 antigen); CD22 (CD22 antigen); CD36 (CD36 antigen (collagen type I receptor, thrombospondin receptor)); CD36L1 (CD36 antigen (collagen type I receptor, thrombospondin receptor)-like 1); CD39 (CD39 antigen); CD3E (CD3E antigen, epsilon polypeptide (TiT3 complex)); CD3G (CD3G antigen, gamma polypeptide (TiT3 complex)); CD3Z (CD3Z antigen, zeta polypeptide (TiT3 complex)); CD4 (CD4 antigen (p55)); CD44 (CD44 antigen (homing function and Indian blood group system)); CD47 (CD47 antigen (Rh-related antigen, integrin-associated signal transducer)); CD5 (CD5 antigen (p56-62)); CD53 (CD53 antigen); CD58 (CD58 antigen, (lymphocyte function-associated antigen 3)); CD59 (CD59 antigen p18-20 (antigen identified by monoclonal antibodies 16.3A5, EJ16, EJ30, EL32 and G344)); CD5L (CD5 antigen-like (scavenger receptor cysteine rich family)); CD6 (CD6 antigen); CD63 (CD63 antigen (melanoma 1 antigen)); CD68 (CD68 antigen); CD69 (CD69 antigen (p60, early T-cell activation antigen)); CD7 (CD7 antigen (p41)); CD74 (CD74 antigen (invariant polypeptide of major histocompatibility complex, class II antigen-associated)); CD79A (CD79A antigen (immunoglobulin-associated alpha)); CD79B (CD79B antigen (immunoglobulin-associated beta)); CD80 (CD80 antigen (CD28 antigen ligand 1, B7-1 antigen)); CD81 (CD81 antigen (target of antiproliferative antibody 1)); CD86 (CD86 antigen (CD28 antigen ligand 2, B7-2 antigen)); CD8A (CD8 antigen, alpha polypeptide (p32)); CDA (Cytidine deaminase); CDAN1 (Congenital dyserythropoietic anemia, type I); CDAN2 (Congenital dyserythropoietic anemia, type II); CDC2L1 (Cell division cycle 2-like 1 (PITSLRE proteins)); CDH12 (Cadherin 12 (N-cadherin 2)); CDK2 (Cyclin-dependent kinase 2); CDK4 (Cyclin-dependent kinase 4); CDKN1A (Cyclin-dependent kinase inhibitor 1A (p21, Cip1)); CDKN1B (Cyclin-dependent kinase inhibitor 1B (p27, Kip1)); CDKN1C (Cyclin-dependent kinase inhibitor 1C (p57, Kip2)); CDKN2A (Cyclin-dependent kinase inhibitor 2A (melanoma, p16, inhibits CDK4)); CDKN2C (Cyclin-dependent kinase inhibitor 2C (p18, inhibits CDK4)); CDR1 (Cerebellar degeneration-related protein (34kD)); CDR2 (Cerebellar degeneration-related protein (62kD)); CDSN (Corneodesmosin); CDW52 (CDW52 antigen (CAMPATH-1 antigen)); CEBPB (CCAAT/enhancer binding protein (C/EBP), beta); CEBPE (CCAAT/enhancer binding protein (C/EBP), epsilon); CEL (Carboxyl ester lipase (bile salt-stimulated lipase)); CELL (Carboxyl ester lipase-like (bile salt-stimulated lipase-like)); CENPB (Centromere protein B (80kD)); CENPC 1 (Centromere protein C 1); CES1 (Carboxylesterase 1 (monocyte/macrophage serine esterase 1)); CETP (Cholesteryl ester transfer protein, plasma); CFTR (Cystic fibrosis transmembrane conductance regulator); CGA (Glycoprotein hormones, alpha polypeptide); CHC1 (Chromosome condensation 1); CHE2 (Cholinesterase (serum) 2); CHGA (Chromogranin A (parathyroid secretory protein 1)); CHH (Cartilage-hair hypoplasia); CHIT1 (Chitinase 1); CHM (Choroideremia (Rab escort protein 1)); CHML (Choroideremia-like (Rab escort protein 2)); CHRM4 (Cholinergic receptor, muscarinic 4); CHRNA1 (Cholinergic receptor, nicotinic, alpha polypeptide 1 (muscle)); CHRNA7 (Cholinergic receptor, nicotinic, alpha polypeptide 7); CHS1 (Chediak-Higashi syndrome 1); CISH (Cytokine inducible SH2-containing protein); CKB (Creatine kinase, brain); CKM (Creatine kinase, muscle); CKN1 (Cockayne syndrome 1 (classical)); CLA1 (Cerebellar ataxia 1 (autosomal recessive)); CLCN1 (Chloride channel 1, skeletal muscle); CLCN4 (Chloride channel 4); CLCNKB (Chloride channel Kb); CLDN3 (Claudin 3); CLN3 (Ceroid-lipofuscinosis, neuronal 3, juvenile (Batten, Spielmeyer-Vogt disease)); CLTA (Clathrin, light polypeptide (Lca)); CLU (Clusterin (complement lysis inhibitor, SP-40,40, sulfated glycoprotein 2, testosterone-repressed prostate message 2, apolipoprotein J)); CMA1 (Chymase 1, mast cell); CMAR (Cell matrix adhesion regulator); CMD1A (Cardiomyopathy, dilated 1A (autosomal dominant)); CMKBR9 (Chemokine (C-C motif) receptor 9); CMKBR9 (Chemokine (C-C motif) receptor 9); CMKLR1 (Chemokine-like receptor 1); CNC (Carney complex, multiple neoplasia and lentiginosis); CNGA1 (Cyclic nucleotide gated channel alpha 1); CNK (Cytokine-inducible kinase); CNP (2',3'-cyclic nucleotide 3' phosphodiesterase); CNTF (Ciliary neurotrophic factor); CNTFR (Ciliary neurotrophic factor receptor); COIL (Coilin p80); COL11A2 (Collagen, type XI, alpha 2); COL17A1 (Collagen, type XVII, alpha 1); COL1A1 (Collagen, type I, alpha 1); COL1A2 (Collagen, type I, alpha 2); COL2A1 (Collagen, type II, alpha 1 (primary osteoarthritis, spondyloepiphyseal dysplasia, congenital)); COL3A1 (Collagen, type III, alpha 1 (Ehlers-Danlos syndrome type IV, autosomal dominant)); COL4A1 (Collagen, type IV, alpha 1); COL4A3 (Collagen, type IV, alpha 3 (Goodpasture antigen)); COL5A1 (Collagen, type V, alpha 1); COL7A1 (Collagen, type VII, alpha 1 (epidermolysis bullosa, dystrophic, dominant and recessive)); COL9A2 (Collagen, type IX, alpha 2); COMT (Catechol-O-methyltransferase); COX4 (Cytochrome c oxidase subunit IV); CP (Ceruloplasmin (ferroxidase)); CP20 (Lymphocyte cytosolic protein, molecular weight 20kD); CPE (Carboxypeptidase E); CPM (Carboxypeptidase M); CPO (Coproporphyrinogen oxidase (coproporphyria, harderoporphyria)); CPS1 (Carbamoyl-phosphate synthetase 1, mitochondrial); CR1 (Complement component (3b/4b) receptor 1, including Knops blood group system); CR1L (Complement component (3b/4b) receptor 1-like); CR2 (Complement component (3d/Epstein Barr virus) receptor 2); CREB 1 (CAMP responsive element binding protein 1); CREBL1 (CAMP responsive element binding protein-like 1); CREM (CAMP responsive element modulator); CRP (C-reactive protein, pentraxin-related); CRS1C (Cryptidin-related sequence-1C); CRX (Cone-rod homeobox); CRYAB (Crystallin, alpha B); CSBP1 (Cytokine suppressive anti-inflammatory drug binding protein 1 (p38 MAP kinase)); CSE1L (Chromosome segregation 1 (yeast homolog)-like); CSF1 (Colony stimulating factor 1 (macrophage)); CSF1 (Colony stimulating factor 1 (macrophage)); CSF1R (Colony stimulating factor 1 receptor, formerly McDonough feline sarcoma viral (v-fms) oncogene homolog); CSF2 (Colony stimulating factor 2 (granulocyte-macrophage)); CSF2RA (Colony stimulating factor 2 receptor, alpha, low-affinity (granulocyte-macrophage)); CSF2RB (Colony stimulating factor 2 receptor, beta, low-affinity (granulocyte-macrophage)); CSF2RY (Granulocyte-macrophage colony-stimulating factor receptor, alpha); CSF3 (Colony stimulating factor 3 (granulocyte)); CSF3R (Colony stimulating factor 3 receptor (granulocyte)); CSF3R (Colony stimulating factor 3 receptor (granulocyte)); CSH1 (Chorionic somatomammotropin hormone 1 (placental lactogen)); CSMF (Chondrosarcoma, extraskeletal myxoid, fused to EWS in); CSN1 (Casein, alpha); CSN2 (Casein, beta); CSNU3 (Cystinuria type 3); CSPG2 (Chondroitin sulfate proteoglycan 2 (versican)); CSPG3 (Chondroitin sulfate proteoglycan 3 (neurocan)); CSRP1 (Cysteine and glycine-rich protein 1); CSRP2 (Cysteine and glycine-rich protein 2 (LIM domain only, smooth muscle)); CST3 (Cystatin C (amyloid angiopathy and cerebral hemorrhage)); CSTB (Cystatin B (stefin B)); CTF1 (Cardiotrophin 1); CTGF (Connective tissue growth factor); CTLA4 (Cytotoxic T-lymphocyte-associated protein 4); CTNS (Cystinosis, nephropathic); CTSB (Cathepsin B); CTSC (Cathepsin C); CTSF (Cathepsin F); CTSG (Cathepsin G); CTSK (Cathepsin K (pycnodysostosis)); CTSL (Cathepsin L); CTSS (Cathepsin S); CUBN (Cubilin (intrinsic factor-cobalamin receptor)); CX3CR1 (Chemokine (C-X3-C) receptor 1); CXCR4 (Chemokine (C-X-C motif), receptor 4 (fusin)); CYBA (Cytochrome b-245, alpha polypeptide); CYBB (Cytochrome b-245, beta polypeptide (chronic granulomatous disease)); CYP11A (Cytochrome P450, subfamily XIA (cholesterol side chain cleavage)); CYP11B2 (Cytochrome P450, subfamily XIB (steroid 11-beta-hydroxylase), polypeptide 2); CYP17 (Cytochrome P450, subfamily XVII (steroid 17-alpha-hydroxylase), adrenal hyperplasia); CYP 19 (Cytochrome P450, subfamily XIX (aromatization of androgens)); CYP1A2 (Cytochrome P450, subfamily I (aromatic compound-inducible), polypeptide 2); CYP1B1 (Cytochrome P450, subfamily I (dioxin-inducible), polypeptide 1 (glaucoma 3, primary infantile)); CYP21 (Cytochrome P450, subfamily XXI (steroid 21-hydroxylase, congenital adrenal hyperplasia)); CYP27A1 (Cytochrome P450, subfamily XXVIIA (steroid 27-hydroxylase, cerebrotendinous xanthomatosis), polypeptide 1); CYP2A (Cytochrome P450, subfamily IIA (phenobarbital-inducible)); CYP2A6 (Cytochrome P450, subfamily IIA (phenobarbital-inducible), polypeptide 6); CYP2C9 (Cytochrome P450, subfamily IIC (mephenytoin 4-hydroxylase), polypeptide 9); CYP2D@ (Cytochrome P450, subfamily IID (debrisoquine, sparteine, etc., -metabolizing) cluster); CYP2E (Cytochrome P450, subfamily IIE (ethanol-inducible)); CYP7A1 (Cytochrome P450, subfamily VIIA (cholesterol 7 alpha-monooxygenase), polypeptide 1); D2S69E (T-lymphocyte activation gene 519); D6S207E (Minor histocompatibility antigen HA-2); D6S2244E (Ke4 gene, mouse, human homolog of); D6S231E (DEK gene); D6S51E (HLA-B associated transcript-2); D6S52E (HLA-B associated transcript-3); D6S54E (HLA-B associated transcript-4); D6S81E (HLA-B associated transcript-1); D6S82E (HLA-B associated transcript-5); DAD1 (Defender against cell death 1); DAF (Decay accelerating factor for complement (CD55, Cromer blood group system)); DAG1 (Dystroglycan 1 (dystrophin-associated glycoprotein 1)); DAO (D-amino-acid oxidase); DAP (Death-associated protein); DAPK1 (Death-associated protein kinase 1); DAPK3 (Death-associated protein kinase 3); DBH (Dopamine beta-hydroxylase (dopamine beta-monooxygenase)); DCC (Deleted in colorectal carcinoma); DCP1 (Dipeptidyl carboxypeptidase 1 (angiotensin I converting enzyme)); DCX (Doublecortex; lissencephaly, X-linked (doublecortin)); DDX10 (DEAD/H (Asp-Glu-Ala-Asp/His) box polypeptide 10 (RNA helicase)); DDX11 (DEAD/H (Asp-Glu-Ala-Asp/His) box polypeptide 11 (S.cerevisiae CHL1-like helicase) ); DEFA1 (Defensin, alpha 1, myeloid-related sequence); DEFA4 (Defensin, alpha 4, corticostatin); DEFA5 (Defensin, alpha 5, Paneth cell-specific); DEFA6 (Defensin, alpha 6, Paneth cell-specific); DEFB1 (Defensin, beta 1); DF (D component of complement (adipsin)); DFFA (DNA fragmentation factor, 45 kD, alpha subunit); DFFB (DNA fragmentation factor, 40 kD, beta subunit); DGCR (DiGeorge syndrome chromosome region); DGI1 (Dentinogenesis imperfecta 1); DHFR (Dihydrofolate reductase); DI (Diego blood group); DIA1 (Diaphorase (NADH) (cytochrome b-5 reductase)); DM (Dystrophia myotonica (includes dystrophia myotonia protein kinase)); DMD (Dystrophin (muscular dystrophy, Duchenne and Becker types), includes DXS142, DXS164, DXS206, DXS230, DXS239, DXS268, DXS269, DXS270, DXS272); DMP1 (Dentin matrix acidic phosphoprotein); DNASE1 (Deoxyribonuclease I); DNASE1L1 (Deoxyribonuclease I-like 1); DNASE1L2 (Deoxyribonuclease I-like 2); DNASE1L3 (Deoxyribonuclease I-like 3); DNASE2 (Deoxyribonuclease II, lysosomal); DNTT (Deoxynucleotidyltransferase, terminal); DOCK1 (Dedicator of cyto-kinesis 1); DPP4 (Dipeptidylpeptidase IV (CD26, adenosine deaminase complexing protein 2)); DPYD (Dihydropyrimidine dehydrogenase); DRA (Down-regulated in adenoma); DRD2 (Dopamine receptor D2); DRD3 (Dopamine receptor D3); DRD4 (Dopamine receptor D4); DSG1 (Desmoglein 1); DSG3 (Desmoglein 3 (pemphigus vulgaris antigen)); DSP (Desmoplakin (DPI, DPII)); DTD (Diastrophic dysplasia); DTNA (Dystrobrevin, alpha); DTR (Diphtheria toxin receptor (heparin-binding epidermal growth factor-like growth factor)); DXS423E (Segregation of mitotic chromosomes 1 (SMC1, yeast human homolog of;); DXS435E (A-11 gene); DYS (Dysautonomia (Riley-Day syndrome, hereditary sensory autonomic neuropathy type III)); DYX2 (Dyslexia 2); E2F1 (E2F transcription factor 1); EBF (Early B-cell factor); ECB2); ECGF1 (Endothelial cell growth factor 1 (platelet-derived)); ED1 (Ectodermal dysplasia 1, anhidrotic); EDG1 (Endothelial differentiation, sphingolipid G-protein-coupled receptor, 1); EDN1 (Endothelin 1); EEC2 (Ectrodactyly, ectodermal dysplasia and cleft lip/palate syndrome 2); EGR1 (Early growth response 1); EGR2 (Early growth response 2 (Krox-20 (Drosophila) homolog)); EIF3S6 (Eukaryotic translation initiation factor 3, subunit 6 (48kD)); EIF4G1 (Eukaryotic translation initiation factor 4 gamma, 1); EIF4G2 (Eukaryotic translation initiation factor 4 gamma, 2); EIF5A (Eukaryotic translation initiation factor 5A); EJM1 (Epilepsy, juvenile myoclonic 1); ELA2 (Elastase 2, neutrophil); ELANH2 (Protease inhibitor 2 (anti-elastase), monocyte/neutrophil); ELAVL2 (ELAV (embryonic lethal, abnormal vision, Drosophila)-like 2); ELAVL4 (ELAV (embryonic lethal, abnormal vision, Drosophila)-like 4 (Hu antigen D)); ELN (Elastin (supravalvular aortic stenosis, Williams-Beuren syndrome)); ENG (Endoglin (Osler-Rendu-Weber syndrome 1)); ENPEP (Glutamyl aminopeptidase (aminopeptidase A)); EPB41 (Erythrocyte membrane protein band 4.1 (elliptocytosis 1, RH-linked)); EPB42 (Erythrocyte membrane protein band 4.2); EPHA3 (EphA3); EPHX1 (Epoxide hydrolase 1, microsomal (xenobiotic)); EPHX2 (Epoxide hydrolase 2, cytoplasmic); EPO (Erythropoietin); EPOR (Erythropoietin receptor); EPOR (Erythropoietin receptor); EPOR (Erythropoietin receptor); EPS 15 (Epidermal growth factor receptor pathway substrate 15); EPT (Epilepsy, partial); ERBB2 (V-erb-b2 avian erythroblastic leukemia viral oncogene homolog 2 (neuro/glioblastoma derived oncogene homolog)); ERCC1 (Excision repair cross-complementing rodent repair deficiency, complementation group 1 (includes overlapping antisense sequence)); ERCC2 (Excision repair cross-complementing rodent repair deficiency, complementation group 2 (xeroderma pigmentosum D)); ERCC3 (Excision repair cross-complementing rodent repair deficiency, complementation group 3 (xeroderma pigmentosum group B complementing)); ERCC4 (Excision repair cross-complementing rodent repair deficiency, complementation group 4); ERCC5 (Excision repair cross-complementing rodent repair deficiency, complementation group 5 (xeroderma pigmentosum, complementation group G (Cockayne syndrome))); ES 1 (ES1 (zebrafish) protein, human homolog of); ESB3 (Esterase B3); ESD (Esterase D/formylglutathione hydrolase); ESR1 (Estrogen receptor 1); ESR2 (Estrogen receptor 2 (ER beta)); ETV4 (Ets variant gene 4 (E1A enhancer-binding protein, E1AF)); ETV6 (Ets variant gene 6 (TEL oncogene)); EYCL3 (Eye color 3 (brown)); F10 (Coagulation factor X); F11 (Coagulation factor XI (plasma thromboplastin antecedent)); F12 (Coagulation factor XII (Hageman factor)); F13A1 (Coagulation factor XIII, A1 polypeptide); F13B (Coagulation factor XIII, B polypeptide); F2 (Coagulation factor II (thrombin)); F2R (Coagulation factor II (thrombin) receptor); F2RL2 (Coagulation factor II (thrombin) receptor-like 2); F3 (Coagulation factor III (thromboplastin, tissue factor)); F5 (Coagulation factor V (proaccelerin, labile factor)); F7 (Coagulation factor VII (serum prothrombin conversion accelerator)); F7R (Coagulation factor VII regulator); F8A (Factor VIII associated gene); F8C (Coagulation factor VIIIc, procoagulant component (hemophilia A)); F9 (Coagulation factor IX (plasma thromboplastic component, Christmas disease, hemophilia B)); FABP1 (Fatty acid binding protein 1, liver); FABP2 (Fatty acid binding protein 2, intestinal); FABP6 (Fatty acid binding protein 6, ileal (gastrotropin)); FADD (Fas (TNFRSF6)-associated via death domain); FAH (Fumarylacetoacetate); FANCA (Fanconi anemia, complementation group A); FANCB (Fanconi anemia, complementation group B); FANCC (Fanconi anemia, complementation group C); FANCE (Fanconi anemia, complementation group E); FANCG (Fanconi anemia, complementation group G); FAT (FAT tumor suppressor (Drosophila) homolog); FBLN1 (Fibulin 1); FBN1 (Fibrillin 1 (Marfan syndrome)); FBP1 (Fructose-bisphosphatase 1); FCAR (Fc fragment of IgA, receptor for); FCER1A (Fc fragment of IgE, high affinity I, receptor for; alpha polypeptide); FCER1B (Fc fragment of IgE, high affinity I, receptor for; beta polypeptide); FCER2 (Fc fragment of IgE, low affinity II, receptor for (CD23A)); FCGR1A (Fc fragment of IgG, high affinity Ia, receptor for (CD64)); FCGR1B (Fc fragment of IgG, high affinity Ib, receptor for (CD64)); FCGR1B (Fc fragment of IgG, high affinity Ib, receptor for (CD64)); FCGR2A (Fc fragment of IgG, low affinity IIa, receptor for (CD32)); FCGR2B (Fc fragment of IgG, low affinity IIb, receptor for (CD32)); FCGR2C (Fc fragment of IgG, low affinity IIb, receptor for (CD32)); FCGR3A (Fc fragment of IgG, low affinity IIIa, receptor for (CD16)); FCGR3B (Fc fragment of IgG, low affinity IIIb, receptor for (CD16)); FCN1 (Ficolin (collagen/fibrinogen domain-containing) 1); FDH (Formaldehyde dehydrogenase); FEA (F9 embryonic antigen); FEB1 (Febrile convulsions 1febrile convulsions 1); FECH (Ferrochelatase (protoporphyria)); FES (Feline sarcoma (Snyder-Theilen) viral (v-fes)/Fujinami avian sarcoma (PRCII) viral (v-fps) oncogene homolog); FGA (Fibrinogen, A alpha polypeptide); FGB (Fibrinogen, B beta polypeptide); FGF1 (Fibroblast growth factor 1 (acidic)); FGF9 (Fibroblast growth factor 9 (glia-activating factor)); FGFR1 (Fibroblast growth factor receptor 1 (fms-related tyrosine kinase 2, Pfeiffer syndrome)); FGFR3 (Fibroblast growth factor receptor 3 (achondroplasia, thanatophoric dwarfism)); FGG (Fibrinogen, gamma polypeptide); FGR (Gardner-Rasheed feline sarcoma viral (v-fgr) oncogene homolog); FHR2 (Factor H-related gene 2); FKHR (Forkhead (Drosophila) homolog 1 (rhabdomyosarcoma)); FLG (Filaggrin); FLNB (Filamin B, beta (actin-binding protein-278)); FLT3 (Fms-related tyrosine kinase 3); FLT3LG (Fms-related tyrosine kinase 3 ligand); FMO1 (Flavin containing monooxygenase 1); FMO3 (Flavin containing monooxygenase 3); FN1 (Fibronectin 1); FOLR1 (Folate receptor 1 (adult)); FOS (V-fos FBJ murine osteosarcoma viral oncogene homolog); FOSB (FBJ murine osteosarcoma viral oncogene homolog B); FPDMM); FPR1 (Formyl peptide receptor 1); FPRL1 (Formyl peptide receptor-like 1); FR (Froese blood group); FRAP1 (FK506 binding protein 12-rapamycin associated protein 1); FRDA (Friedreich ataxia); FRG1 (FSHD region gene 1); FSHMD1A (Facioscapulohumeral muscular dystrophy 1A); FTL (Ferritin, light polypeptide); FTNB (Fertilin beta (a disintegrin and metalloproteinase domain 2)); FUCA1 (Fucosidase, alpha-L- 1, tissue); FUCA2 (Fucosidase, alpha-L- 2, plasma); FUT2 (Fucosyltransferase 2 (secretor status included)); FUT3 (Fucosyltransferase 3 (galactoside 3(4)-L-fucosyltransferase, Lewis blood group included)); FXR1 (Fragile X mental retardation, autosomal homolog); DAD 1 (Defender against cell death 1); DAF (Decay accelerating factor for complement (CD55, Cromer blood group system)); DAG1 (Dystroglycan 1 (dystrophin-associated glycoprotein 1)); DAO (D-amino-acid oxidase); DAP (Death-associated protein); DAPK1 (Death-associated protein kinase 1); DAPK3 (Death-associated protein kinase 3); DBH (Dopamine beta-hydroxylase (dopamine beta-monooxygenase)); DCC (Deleted in colorectal carcinoma); DCP1 (Dipeptidyl carboxypeptidase 1 (angiotensin I converting enzyme)); DCX (Doublecortex; lissencephaly, X-linked (doublecortin)); DDX10 (DEAD/H (Asp-Glu-Ala-Asp/His) box polypeptide 10 (RNA helicase)); DDX11 (DEAD/H (Asp-Glu-Ala-Asp/His) box polypeptide 11 (S.cerevisiae CHL1-like helicase)); DEFA1 (Defensin, alpha 1, myeloid-related sequence); DEFA4 (Defensin, alpha 4, corticostatin); DEFA5 (Defensin, alpha 5, Paneth cell-specific); DEFA6 (Defensin, alpha 6, Paneth cell-specific); DEFB1 (Defensin, beta 1); DF (D component of complement (adipsin)); DFFA (DNA fragmentation factor, 45 kD, alpha subunit); DFFB (DNA fragmentation factor, 40 kD, beta subunit); DGCR (DiGeorge syndrome chromosome region); DGI1 (Dentinogenesis imperfecta 1); DHFR (Dihydrofolate reductase); DI (Diego blood group); DIA1 (Diaphorase (NADH) (cytochrome b-5 reductase)); DM (Dystrophia myotonica (includes dystrophia myotonia protein kinase)); DMD (Dystrophin (muscular dystrophy, Duchenne and Becker types), includes DXS 142, DXS164, DXS206, DXS230, DXS239, DXS268, DXS269, DXS270, DXS272); DMP1 (Dentin matrix acidic phosphoprotein); DNASE1 (Deoxyribonuclease I); DNASE1L1 (Deoxyribonuclease I-like 1); DNASE1L2 (Deoxyribonuclease I-like 2); DNASE1L3 (Deoxyribonuclease I-like 3); DNASE2 (Deoxyribonuclease II, lysosomal); DNTT (Deoxynucleotidyltransferase, terminal); DOCK1 (Dedicator of cyto-kinesis 1); DPP4 (Dipeptidylpeptidase IV (CD26, adenosine deaminase complexing protein 2)); DPYD (Dihydropyrimidine dehydrogenase); DRA (Down-regulated in adenoma); DRD2 (Dopamine receptor D2); DRD3 (Dopamine receptor D3); DRD4 (Dopamine receptor D4); DSG1 (Desmoglein 1); DSG3 (Desmoglein 3 (pemphigus vulgaris antigen)); DSP (Desmoplakin (DPI, DPII)); DTD (Diastrophic dysplasia); DTNA (Dystrobrevin, alpha); DTR (Diphtheria toxin receptor (heparin-binding epidermal growth factor-like growth factor)); DXS423E (Segregation of mitotic chromosomes 1 (SMC1, yeast human homolog of;); DXS435E (A-11 gene); DYS (Dysautonomia (Riley-Day syndrome, hereditary sensory autonomic neuropathy type III)); DYX2 (Dyslexia 2); E2F1 (E2F transcription factor 1); EBF (Early B-cell factor ECB2); ECGF1 Endothelial cell growth factor 1 (platelet-derived)); ED1 (Ectodermal dysplasia 1, anhidrotic); EDG1 (Endothelial differentiation, sphingolipid); G-protein-coupled receptor, 1); EDN1 (Endothelin 1); EEC2 (Ectrodactyly, ectodermal dysplasia and cleft lip/palate syndrome 2); EGR1 (Early growth response 1); EGR2 (Early growth response 2 (Krox-20 (Drosophila) homolog)); EIF3S6 (Eukaryotic translation initiation factor 3, subunit 6 (48kD)); EIF4G1 (Eukaryotic translation initiation factor 4 gamma, 1); EIF4G2 (Eukaryotic translation initiation factor 4 gamma, 2); EIF5A (Eukaryotic translation initiation factor 5A); EJM1 (Epilepsy, juvenile myoclonic 1); ELA2 (Elastase 2, neutrophil); ELANH2 (Protease inhibitor 2 (anti-elastase), monocyte/neutrophil); ELAVL2 (ELAV (embryonic lethal, abnormal vision, Drosophila)-like 2); ELAVL4 (ELAV (embryonic lethal, abnormal vision, Drosophila)-like 4 (Hu antigen D)); ELN (Elastin (supravalvular aortic stenosis, Williams-Beuren syndrome)); ENG (Endoglin (Osler-Rendu-Weber syndrome 1)); ENPEP (Glutamyl aminopeptidase (aminopeptidase A)); EPB41 (Erythrocyte membrane protein band 4.1 (elliptocytosis 1, RH-linked)); EPB42 (Erythrocyte membrane protein band 4.2); EPHA3 (EphA3); EPHX1 (Epoxide hydrolase 1, microsomal (xenobiotic)); EPHX2 (Epoxide hydrolase 2, cytoplasmic); EPO (Erythropoietin); EPOR (Erythropoietin receptor); EPOR (Erythropoietin receptor); EPOR (Erythropoietin receptor); EPS 15 (Epidermal growth factor receptor pathway substrate 15); EPT (Epilepsy, partial); ERBB2 (V-erb-b2 avian erythroblastic leukemia viral oncogene homolog 2 (neuro/glioblastoma derived oncogene homolog)); ERCC1 (Excision repair cross-complementing rodent repair deficiency, complementation group 1 (includes overlapping antisense sequence)); ERCC2 (Excision repair cross-complementing rodent repair deficiency, complementation group 2 (xeroderma pigmentosum D)); ERCC3 (Excision repair cross-complementing rodent repair deficiency, complementation group 3 (xeroderma pigmentosum group B complementing)); ERCC4 (Excision repair cross-complementing rodent repair deficiency, complementation group 4); ERCC5 (Excision repair cross-complementing rodent repair deficiency, complementation group 5 (xeroderma pigmentosum, complementation group G (Cockayne syndrome))); ES 1 (ES1 (zebrafish) protein, human homolog of); ESB3 (Esterase B3); ESD (Esterase D/formylglutathione hydrolase); ESR1 (Estrogen receptor 1); ESR2 (Estrogen receptor 2 (ER beta)); ETV4 (Ets variant gene 4 (E1A enhancer-binding protein, E1AF)); ETV6 (Ets variant gene 6 (TEL oncogene)); EYCL3 (Eye color 3 (brown)); F10 (Coagulation factor X); F11 (Coagulation factor XI (plasma thromboplastin antecedent)); F12 (Coagulation factor XII (Hageman factor)); F13A1 (Coagulation factor XIII, A1 polypeptide); F13B (Coagulation factor XIII, B polypeptide); F2 (Coagulation factor II (thrombin)); F2R (Coagulation factor II (thrombin) receptor); F2RL2 (Coagulation factor II (thrombin) receptor-like 2); F3 (Coagulation factor III (thromboplastin, tissue factor)); F5 (Coagulation factor V (proaccelerin, labile factor)); F7 (Coagulation factor VII (serum prothrombin conversion accelerator)); F7R (Coagulation factor VII regulator); F8A (Factor VIII associated gene); F8C (Coagulation factor VIIIc, procoagulant component (hemophilia A)); F9 (Coagulation factor IX (plasma thromboplastic component, Christmas disease, hemophilia B)); FABP1 (Fatty acid binding protein 1, liver); FABP2 (Fatty acid binding protein 2, intestinal); FABP6 (Fatty acid binding protein 6, ileal (gastrotropin)); FADD (Fas (TNFRSF6)-associated via death domain); FAH (Fumarylacetoacetate); FANCA (Fanconi anemia, complementation group A); FANCB (Fanconi anemia, complementation group B); FANCC (Fanconi anemia, complementation group C); FANCE (Fanconi anemia, complementation group E); FANCG (Fanconi anemia, complementation group G); FAT (FAT tumor suppressor (Drosophila) homolog); FBLN1 (Fibulin 1); FBN1 (Fibrillin 1 (Marfan syndrome)); FBP1 (Fructose-bisphosphatase 1); FCAR (Fc fragment of IgA, receptor for); FCER1A (Fc fragment of IgE, high affinity I, receptor for; alpha polypeptide); FCER1B (Fc fragment of IgE, high affinity I, receptor for; beta polypeptide); FCER2 (Fc fragment of IgE, low affinity II, receptor for (CD23A)); FCGR1A (Fc fragment of IgG, high affinity Ia, receptor for (CD64)); FCGR1B (Fc fragment of IgG, high affinity Ib, receptor for (CD64)); FCGR1B (Fc fragment of IgG, high affinity Ib, receptor for (CD64)); FCGR2A (Fc fragment of IgG, low affinity IIa, receptor for (CD32)); FCGR2B (Fc fragment of IgG, low affinity IIb, receptor for (CD32)); FCGR2C (Fc fragment of IgG, low affinity IIb, receptor for (CD32)); FCGR3A (Fc fragment of IgG, low affinity IIIa, receptor for (CD16)); FCGR3B (Fc fragment of IgG, low affinity IIIb, receptor for (CD16)); FCN1 (Ficolin (collagen/fibrinogen domain-containing) 1); FDH (Formaldehyde dehydrogenase); FEA (F9 embryonic antigen); FEB 1 (Febrile convulsions 1febrile convulsions 1); FECH (Ferrochelatase (protoporphyria)); FES (Feline sarcoma (Snyder-Theilen) viral (v-fes)/Fujinami avian sarcoma (PRCII) viral (v-fps) oncogene homolog); FGA (Fibrinogen, A alpha polypeptide); FGB (Fibrinogen, B beta polypeptide); FGF1 (Fibroblast growth factor 1 (acidic)); FGF9 (Fibroblast growth factor 9 (glia-activating factor)); FGFR1 (Fibroblast growth factor receptor 1 (fins-related tyrosine kinase 2, Pfeiffer syndrome)); FGFR3 (Fibroblast growth factor receptor 3 (achondroplasia, thanatophoric dwarfism)); FGG (Fibrinogen, gamma polypeptide); FGR (Gardner-Rasheed feline sarcoma viral (v-fgr) oncogene homolog); FHR2 (Factor H-related gene 2); FKHR (Forkhead (Drosophila) homolog 1 (rhabdomyosarcoma)); FLG (Filaggrin); FLNB (Filamin B, beta (actin-binding protein-278)); FLT3 (Fms-related tyrosine kinase 3); FLT3LG (Fms-related tyrosine kinase 3 ligand); FMO1 (Flavin containing monooxygenase 1); FMO3 (Flavin containing monooxygenase 3); FN1 (Fibronectin 1); FOLR1 (Folate receptor 1 (adult)); FOS (V-fos FBJ murine osteosarcoma viral oncogene homolog); FOSB (FBJ murine osteosarcoma viral oncogene homolog B); FPDMM); FPR1 (Formyl peptide receptor 1); FPRL1 (Formyl peptide receptor-like 1); FR (Froese blood group); FRAP1 (FK506 binding protein 12-rapamycin associated protein); FRDA (Friedreich ataxia); FRG1); FSHD region gene 1); FSHMD1A (Facioscapulohumeral muscular dystrophy 1A); FTL (Ferritin, light polypeptide); FTNB (Fertilin beta (a disintegrin and metalloproteinase domain 2)); FUCA1 (Fucosidase, alpha-L-1, tissue); FUCA2 (Fucosidase, alpha-L- 2, plasma); FUT2 (Fucosyltransferase 2 (secretor status included)); FUT3 (Fucosyltransferase 3 (galactoside); 3(4)-L-fucosyltransferase, Lewis blood group included)); FXR1 (Fragile X mental retardation, autosomal homolog); FY (Duffy blood group); FYB (FYN-binding protein (FYB-120/130)); G22P1 (Thyroid autoantigen 70kD (Ku antigen)); G6PD (Glucose-6-phosphate dehydrogenase); GAA (Glucosidase, alpha; acid (Pompe disease, glycogen storage disease type II)); GABRA5 (Gamma-aminobutyric acid (GABA) A receptor, alpha 5); GABRA6 - (Gamma-aminobutyric acid (GABA) A receptor, alpha 6); GABRB 1 (Gamma-aminobutyric acid (GABA) A receptor, beta 1); GAD2 (Glutamate decarboxylase 2 (pancreatic islets and brain, 65kD)); GALC (Galactosylceramidase (Krabbe disease)); GALK1 (Galactokinase 1); GALT (Galactose-1-phosphate uridylyltransferase); GANC (Glucosidase, alpha; neutral C); GAS (Gastrin); GAS6 (Growth arrest-specific 6); GATA3 (GATA-binding protein 3); GBA (Glucosidase, beta; acid (includes glucosylceramidase)); GC (Group-specific component (vitamin D binding protein)); GCDH (Glutaryl-Coenzyme A dehydrogenase); GCGR (Glucagon receptor); GCK (Glucokinase (hexokinase 4, maturity onset diabetes of the young 2)); GCP (Green cone pigment (color blindness, deutan)); GDH (Glucose dehydrogenase); GEM (GTP-binding protein overexpressed in skeletal muscle); GFI1 (Growth factor independent 1); GGCX (Gamma-glutamyl carboxylase); GGT1 (Gamma-glutamyltransferase 1); GGTA1 (Glycoprotein, alpha-galactosyltransferase 1); GH1 (Growth hormone 1); GH2 (Growth hormone 2); GHR (Growth hormone receptor); GIF (Gastric intrinsic factor (vitamin B synthesis)); GLA (Galactosidase, alpha); GLC1B (Glaucoma 1, open angle, B (adult-onset)); GLCLC (Glutamate-cysteine ligase (gamma-glutamylcysteine synthetase), catalytic (72.8kD)); GLO1 (Glyoxalase I); GLP1R (Glucagon-like peptide 1 receptor); GLRB (Glycine receptor, beta); GLS (Glutaminase); GLUD1 (Glutamate dehydrogenase 1); GLYB (Glycine B complementing); GLYS1 (Glycosuria 1, renal); GNAQ (Guanine nucleotide binding protein (G protein), q polypeptide); GNAS 1 (Guanine nucleotide binding protein (G protein), alpha stimulating activity polypeptide 1); GNB3 (Guanine nucleotide binding protein (G protein), beta polypeptide 3); GNL1 (Guanine nucleotide binding protein-like 1); GOT2 (Glutamic-oxaloacetic transaminase 2, mitochondrial (aspartate aminotransferase 2)); GP1BA (Glycoprotein Ib (platelet), alpha polypeptide); GP1BB (Glycoprotein Ib (platelet), beta polypeptide); GP9 (Glycoprotein IX (platelet)); GPD2 (Glycerol-3-phosphate dehydrogenase 2 (mitochondrial)); GPR10 (G protein-coupled receptor 10); GPR13 (G protein-coupled receptor 13); GPR15 (G protein-coupled receptor 15); GPR2 (G protein-coupled receptor 2); GPR30 (G protein-coupled receptor 30); GPR4 (G protein-coupled receptor 4); GPR5 (G protein-coupled receptor 5); GPR9 (G protein-coupled receptor 9); GPT (Glutamic-pyruvate transaminase (alanine aminotransferase)); GPX1 (Glutathione peroxidase 1); GPX2 (Glutathione peroxidase 2 (gastrointestinal)); GRB10 (Growth factor receptor-bound protein 10); GRB14 (Growth factor receptor-bound protein 14); GRIK1 (Glutamate receptor, ionotropic, kainate 1); GRIK2 (Glutamate receptor, ionotropic, kainate 2); GRL (Glucocorticoid receptor); GRO1 (GRO1 oncogene (melanoma growth stimulating activity, alpha)); GRO2 (GRO2 oncogene); GRO3 (GRO3 oncogene); GRPR (Gastrin-releasing peptide receptor); GSN (Gelsolin (amyloidosis, Finnish type)); GSPT1 (G1 to S phase transition 1); GSR (Glutathione reductase); GSS (Glutathione synthetase); GSTA1 (Glutathione S-transferase A1); GSTA2 (Glutathione S-transferase A2); GSTA4 (Glutathione S-transferase A4); GSTM1 (Glutathione S-transferase M1); GSTM2 (Glutathione S-transferase M2 (muscle)); GSTM3 (Glutathione S-transferase M3 (brain)); GSTP1 (Glutathione S-transferase pi); GSTT1 (Glutathione S-transferase theta 1); GTF2H2 (General transcription factor IIH, polypeptide 2 (44kD subunit)); GTS (Gilles de la Tourette syndrome); GUCY1A3 (Guanylate cyclase 1, soluble, alpha 3); GUCY2D (Guanylate cyclase 2D, membrane (retina-specific)); GUSB (Glucuronidase, beta); GYPA (Glycophorin A (includes MN blood group)); GYPB (Glycophorin B (includes Ss blood group)); GYS1 (Glycogen synthase 1 (muscle)); GZMA (Granzyme A (granzyme 1, cytotoxic T-lymphocyte-associated serine esterase 3)); GZMB (Granzyme B (granzyme 2, cytotoxic T-lymphocyte-associated serine esterase 1)); GZMM (Granzyme M (lymphocyte met-ase 1)); H142T (Temperature sensitivity complementation, H142); HADHA (Hydroxyacyl-Coenzyme A dehydrogenase/3-ketoacyl-Coenzyme A thiolase/enoyl-Coenzyme A hydratase (trifunctional protein), alpha subunit); HADHSC (L-3-hydroxyacyl-Coenzyme A dehydrogenase, short chain); HAGH (Hydroxyacyl glutathione hydrolase; glyoxalase 2); HAL (Histidine ammonia-lyase); HBA1 (Hemoglobin, alpha 1); HBB (Hemoglobin, beta); HBG1 (Hemoglobin, gamma A); HBZ (Hemoglobin, zeta); HCF2 (Heparin cofactor II); HCFC1 (Host cell factor C1 (VP16-accessory protein)); HCR (Chemokine receptor); HD (Huntingtin (Huntington disease)); HDAC1 (Histone deacetylase 1); HEXA (Hexosaminidase A (alpha polypeptide)); HEXB (Hexosaminidase B (beta polypeptide)); HF1 (H factor 1 (complement)); HFE (Hemochromatosis); HFL1 (H factor (complement)-like 1); HGF (Hepatocyte growth factor (hepapoietin A; scatter factor)); HGL (Heregulin, alpha (45kD, ERBB2 p185-activator)); HIP1 (Huntingtin interacting protein 1); HIVEP1 (Human immunodeficiency virus type I enhancer-binding protein 1); HK1 (Hexokinase 1); HK2 (Hexokinase 2); HK3 (Hexokinase 3 (white cell)); HLA-A (Major histocompatibility complex, class I, A); HLA-B (Major histocompatibility complex, class I, B); HLA-C (Major histocompatibility complex, class I, C); HLA-DMA (Major histocompatibility complex, class II, DM alpha); HLA-DMB (Major histocompatibility complex, class II, DM beta); HLA-DNA (Major histocompatibility complex, class II, DN alpha); HLA-DOB (Major histocompatibility complex, class II, DO beta); HLA-DPA1 (Major histocompatibility complex, class II, DP alpha 1); HLA-DPB1 (Major histocompatibility complex, class II, DP beta 1); HLA-DQA1 (Major histocompatibility complex, class II, DQ alpha 1); HLA-DQB1 (Major histocompatibility complex, class II, DQ beta 1); HLA-DRA (Major histocompatibility complex, class II, DR alpha); HLA-DRB1 (Major histocompatibility complex, class II, DR beta 1); HLA-E (Major histocompatibility complex, class I, E); HLA-F (Major histocompatibility complex, class I, F); HLA-G (HLA-G histocompatibility antigen, class I, G); HLALS (Major histocompatibility complex, class I-like sequence (NOTE: symbol provisional)); HLCS (Holocarboxylase synthetase (biotin-[proprionyl-Coenzyme A-carboxylase (ATP-hydrolysing)] ligase)); HLX1 (H2.0 (Drosophila)-like homeo box 1); HM74 (Putative chemokine receptor; GTP-binding protein); HMAB (Monocyte antigen B); HMBS (Hydroxymethylbilane synthase); HMGCL (3-hydroxymethyl-3-methylglutaryl-Coenzyme A lyase ((hydroxymethylglutaricaciduria)); HMGIC (High-mobility group (nonhistone chromosomal) protein isoform I-C); HMSNL); HMX1 (Homeo box (H6 family) 1); HNRPD (Heterogeneous nuclear ribonucleoprotein D); HOXB5 (Homeo box B5); HOXD13 (Homeo box D13); HOXD8 (Homeo box D8); HP (Haptoglobin); HPE1 (Holoprosencephaly 1, alobar); HPN (Hepsin (transmembrane protease, serine 1)); HPR (Haptoglobin-related protein); HPRT1 (Hypoxanthine phosphoribosyltransferase 1 (Lesch-Nyhan syndrome)); HPS (Hermansky-Pudlak syndrome); HPX (Hemopexin); HR (Hairless (mouse) homolog); HRAS (V-Ha-ras Harvey rat sarcoma viral oncogene homolog); HRG (Histidine-rich glycoprotein); HRMT1L1 (HMT1 (hnRNP methyltransferase, S. cerevisiae)-like 1); HRMT1L2 (HMT1 (hnRNP methyltransferase, S. cerevisiae)-like 2); HRY (Hairy (Drosophila)-homolog); HSD17B3 (Hydroxysteroid (17-beta) dehydrogenase 3); HSD3B1 (Hydroxy-delta-5-steroid dehydrogenase, 3 beta- and steroid delta-isomerase 1); HSD3B2 (Hydroxy-delta-5-steroid dehydrogenase, 3 beta- and steroid delta-isomerase 2); HSPA1A (Heat shock 70kD protein 1); HSPA1L (Heat shock 70kD protein-like 1); HSPA2 (Heat shock 70kD protein 2); HSPA6 (Heat shock 70kD protein 6 (HSP70B')); HSPG2 (Heparan sulfate proteoglycan 2 (perlecan)); HTLVR (Human T-cell leukemia virus (I and II) receptor); HTN1 (Histatin 1); HTN3 (Histatin 3); HTR2A (5-hydroxytryptamine (serotonin) receptor 2A); HTR2C (5-hydroxytryptamine (serotonin) receptor 2C); HTR6 (5-hydroxytryptamine (serotonin) receptor 6); HTR7 (5-hydroxytryptamine (serotonin) receptor 7 (adenylate cyclase-coupled)); HVBS6 (Hepatitis B virus integration site 6); HY (Histocompatibility Y antigen); IARS (Isoleucine-tRNA synthetase); IBD1 (Inflammatory bowel disease 1); IBSP (Integrin-binding sialoprotein (bone sialoprotein, bone sialoprotein II)); ICAM1 (Intercellular adhesion molecule 1 (CD54), human rhinovirus receptor); ICAM2 (Intercellular adhesion molecule 2); ICAM3 (Intercellular adhesion molecule 3); ICAM4 (Intercellular adhesion molecule 4, Landsteiner-Wiener blood group); ICAM5 (Intercellular adhesion molecule 5, telencephalin); ICR5 (Ichthyosis congenita V, Sjogren-Larsson-like); ICS1 (Immotile cilia syndrome 1); ICT1 (Immature colon carcinoma transcript 1); IDDM10 (Insulin-dependent diabetes mellitus 10); IDDM11 (Insulin-dependent diabetes mellitus 11); IDDM15 (Insulin-dependent diabetes mellitus 15); IDDM17 (Insulin-dependent diabetes mellitus 17); IDDM4 (Insulin-dependent diabetes mellitus 4); IDDM6 (Insulin-dependent diabetes mellitus 6); IDDM7 (Insulin-dependent diabetes mellitus 7); IDDMX (Diabetes mellitus, insulin-dependent, X-linked, susceptibility to); IDH1 (Isocitrate dehydrogenase 1 (NADP+), soluble); IDS (Iduronate 2-sulfatase (Hunter syndrome)); IDUA (Iduronidase, alpha-L-); IF (I factor (complement)); IFNA1 (Interferon, alpha 1); IFNA10 (Interferon, alpha 10); IFNA13 (Interferon, alpha 13); IFNA2 (Interferon, alpha 2); IFNAR1 (Interferon (alpha, beta and omega) receptor 1); IFNAR2 (Interferon (alpha, beta and omega) receptor 2); IFNG (Interferon, gamma); IFNGR1 (Interferon gamma receptor 1); IFNGR2 (Interferon gamma receptor 2 (interferon gamma transducer 1)); IFNR (Interferon production regulator); IGAT (Immune response to synthetic polypeptide--IRGAT); IGER (IgE responsiveness (atopic)); IGES (Immunoglobulin E concentration, serum); IGF 1 (Insulin-like growth factor 1 (somatomedin C)); IGF1R (Insulin-like growth factor 1 receptor); IGF2 (Insulin-like growth factor 2 (somatomedin A)); IGF2R (Insulin-like growth factor 2 receptor); IGFBP 1 (Insulin-like growth factor binding protein 1); IGFBP 10 (Insulin-like growth factor binding protein 10); IGFBP2 (Insulin-like growth factor binding protein 2 (36kD)); IGFBP3 (Insulin-like growth factor binding protein 3); IGHA1 (Immunoglobulin alpha 1); IGHA2 (Immunoglobulin alpha 2 (A2M marker)); IGHE (Immunoglobulin epsilon); IGHG1 (Immunoglobulin gamma 1 (Gm marker)); IGHG2 (Immunoglobulin gamma 2 (Gm marker)); IGHV@ (Immunoglobulin heavy polypeptide, variable region (cluster)); IGJ (Immunoglobulin J polypeptide, linker protein for immunoglobulin alpha and mu polypeptides); IGKC (Immunoglobulin kappa constant region); IGKV (Immunoglobulin kappa variable region); IGLP 1 (Immune response to synthetic polypeptides-1); IGLP2 (Immune response to synthetic polypeptides-2); IL10 (Interleukin 10); IL10RA (Interleukin 10 receptor, alpha); IL10RA (Interleukin 10 receptor, alpha); IL10RB (Interleukin 10 receptor, beta); IL10RB (Interleukin 10 receptor, beta); IL11 (Interleukin 11); IL11RA (Interleukin 11 receptor, alpha); IL11RA (Interleukin 11 receptor, alpha); IL11RB (Interleukin 11 receptor, beta); IL12A (Interleukin 12A (natural killer cell stimulatory factor 1, cytotoxic lymphocyte maturation factor 1, p35)); IL12B (Interleukin 12B (natural killer cell stimulatory factor 2, cytotoxic lymphocyte maturation factor 2, p40)); IL12RB1 (Interleukin 12 receptor, beta 1); IL12RB2 (Interleukin 12 receptor, beta 2); IL12RB2 (Interleukin 12 receptor, beta 2); IL13 (Interleukin 13); IL13RA1 (Interleukin 13 receptor, alpha 1); IL13RA2 (Interleukin 13 receptor, alpha 2); IL13RA2 (Interleukin 13 receptor, alpha 2); IL15 (Interleukin 15); IL15RA (Interleukin 15 receptor, alpha); IL15RA (Interleukin 15 receptor, alpha); IL15RB (Interleukin 15 receptor, beta); IL16 (Interleukin 16 (lymphocyte chemoattractant factor)); IL17 (Interleukin 17 (cytotoxic T-lymphocyte-associated serine esterase 8)); IL18 (Interleukin 18 (interferon-gamma-inducing factor)); IL18BP (Interleukin 18 binding protein); IL18R1 (Interleukin 18 receptor 1); IL18RAP (Interleukin 18 receptor accessory protein); IL1A (Interleukin 1, alpha); IL1B (Interleukin 1, beta); IL1R1 (Interleukin 1 receptor, type I); IL1R1 (Interleukin 1 receptor, type I); IL1R2 (Interleukin 1 receptor, type II); IL1RAP (Interleukin 1 receptor accessory protein); IL1RL2 (Interleukin 1 receptor-like 2); IL1RN (Interleukin 1 receptor antagonist); IL2 (Interleukin 2); IL2RA (Interleukin 2 receptor, alpha); IL2RA (Interleukin 2 receptor, alpha); IL2RB (Interleukin 2 receptor, beta); IL2RB (Interleukin 2 receptor, beta); IL2RG (Interleukin 2 receptor, gamma (severe combined immunodeficiency) ); IL2RG (Interleukin 2 receptor, gamma (severe combined immunodeficiency)); IL3 (Interleukin 3 (colony-stimulating factor, multiple)); IL3RA (Interleukin 3 receptor, alpha (low affinity)); IL4 (Interleukin 4); IL4R (Interleukin 4 receptor); IL4R (Interleukin 4 receptor); IL5 (Interleukin 5 (colony-stimulating factor, eosinophil)); IL5RA (Interleukin 5 receptor, alpha); IL5RA (Interleukin 5 receptor, alpha); IL6 (Interleukin 6 (interferon, beta 2)); IL6R (Interleukin 6 receptor); IL6ST (Interleukin 6 signal transducer (gp130, oncostatin M receptor)); IL6ST (Interleukin 6 signal transducer (gp130, oncostatin M receptor)); IL7 (Interleukin 7); IL7R (Interleukin 7 receptor); IL8 (Interleukin 8); IL8RA (Interleukin 8 receptor, alpha); IL8RB (Interleukin 8 receptor, beta); IL8RB (Interleukin 8 receptor, beta); IL9 (Interleukin 9); IL9R (Interleukin 9 receptor); IL9R (Interleukin 9 receptor); ILF 1 (Interleukin enhancer binding factor 1); ILF2 (Interleukin enhancer binding factor 2, 45kD); ILF3 (Interleukin enhancer binding factor 3, 90kD); IMPA1 (Inositol(myo)-1(or 4)-monophosphatase 1); IMPT1 (Imprinted polyspecific membrane transporter 1); INLU (Lutheran inhibitor, dominant (monoclonal antibody A3D8) ); INP10 (Interferon (gamma)-induced cell line; protein 10 from INPP5D Inositol polyphosphate-5-phosphatase, 145kD); INS (Insulin); INSR (Insulin receptor); IPF1 (Insulin promoter factor 1, homeodomain transcription factor); IPOX (Intestinal pseudoobstruction, neuronal, primary idiopathic); IRAK1 (Interleukin-1 receptor-associated kinase 1); IRAK2 (Interleukin-1 receptor-associated kinase 2); IRF4 (Interferon regulatory factor 4); IRS1 (Insulin receptor substrate 1); ITGA1 (Integrin, alpha 1); ITGA2 (Integrin, alpha 2 (CD49B, alpha 2 subunit of VLA-2 receptor)); ITGA2B (Integrin, alpha 2b (platelet glycoprotein IIb of IIb/IIIa complex, antigen CD41B)); ITGA4 (Integrin, alpha 4 (antigen CD49D, alpha 4 subunit of VLA-4 receptor) ); ITGA5 (Integrin, alpha 5 (fibronectin receptor, alpha polypeptide)); ITGA6 (Integrin, alpha 6); ITGA7 (Integrin, alpha 7); ITGAD (Integrin, alpha D); ITGAL (Integrin, alpha L (antigen CD 11 A (p180), lymphocyte function-associated antigen 1; alpha polypeptide)); ITGAM (Integrin, alpha M (complement component receptor 3, alpha; also known as CD11b (p170), macrophage antigen alpha polypeptide)); ITGAV (Integrin, alpha V (vitronectin receptor, alpha polypeptide, antigen CD51)); ITGAX (Integrin, alpha X (antigen CD11C (p150), alpha polypeptide)); ITGB 1 (Integrin, beta 1 (fibronectin receptor, beta polypeptide, antigen CD29 includes MDF2, MSK12)); ITGB2 (Integrin, beta 2 (antigen CD18 (p95), lymphocyte function-associated antigen 1; macrophage antigen 1 (mac-1) beta subunit)); ITGB3 (Integrin, beta 3 (platelet glycoprotein IIIa, antigen CD61)); ITGB4 (Integrin, beta 4); ITGB4BP (Integrin beta 4 binding protein); ITGB5 (Integrin, beta 5); ITGB6 (Integrin, beta 6); ITGB7 (Integrin, beta 7); ITIH1 (Inter-alpha (globulin) inhibitor, H1 polypeptide); ITIH4 (Inter-alpha (globulin) inhibitor H4 (plasma Kallikrein-sensitive glycoprotein)); ITK (IL2-inducible T-cell kinase); IVL (Involucrin); JAK1 (Janus kinase 1 (a protein tyrosine kinase)); JAK2 (Janus kinase 2 (a protein tyrosine kinase)); JAK3 (Janus kinase 3 (a protein tyrosine kinase, leukocyte)); JPD (Juvenile periodontitis); JUP (Junction plakoglobin); KAL1 (Kallmann syndrome 1 sequence); KARS (Lysyl-tRNA synthetase); KCNA1 (Potassium voltage-gated channel, shaker-related subfamily, member 1 (episodic ataxia with myokymia)); KCNA2 (Potassium voltage-gated channel, shaker-related subfamily, member 2); KCNA3 (Potassium voltage-gated channel, shaker-related subfamily, member 3); KCNA5 (Potassium voltage-gated channel, shaker-related subfamily, member 5); KCNE1 (Potassium voltage-gated channel, Isk-related family, member 1); KCNJ12 (Potassium inwardly-rectifying channel, subfamily J, member 12); KCNJ3 (Potassium inwardly-rectifying channel, subfamily J, member 3); KCNQ1 (Potassium voltage-gated channel, KQT-like subfamily, member 1); KCNQ2 (Potassium voltage-gated channel, KQT-like subfamily, member 2); KDR (Kinase insert domain receptor (a type III receptor tyrosine kinase)); KEL (Kell blood group); KHK (Ketohexokinase (fructokinase)); KIR2DL4 (Killer cell immunoglobulin-like receptor, two domains, long cytoplasmic tail, 4); KIT (V-kit Hardy-Zuckerman 4 feline sarcoma viral oncogene homolog); KLKB 1 (Kallikrein B plasma, (Fletcher factor) 1); KLRC2 (Killer cell lectin-like receptor subfamily C, member 2); KLRC3 (Killer cell lectin-like receptor subfamily C, member 3); KLRC4 (Killer cell lectin-like receptor subfamily C, member 4); KLRD 1 (Killer cell lectin-like receptor subfamily D, member 1); KNG (Kininogen); KPNA1 (Karyopherin alpha 1 (importin alpha 5)); KRAS2 (V-Ki-ras2 Kirsten rat sarcoma 2 viral oncogene homolog); KRT4 (Keratin 4); KRT9 (Keratin 9 (epidermolytic palmoplantar keratoderma)); KRTHA4 (Keratin, hair, acidic, 4); KRTHB4 (Keratin, hair, basic, 4); KSR (Kinase suppressor of ras); L1CAM (L1 cell adhesion molecule (hydrocephalus, stenosis of queduct of Sylvius 1, MASA (mental retardation, aphasia, shuffling gait and adducted thumbs) syndrome, spastic paraplegia 1)); LAG3 (Lymphocyte-activation gene 3); LAG5 (Leukocyte antigen group 5); LAKL (Lymphokine-activated killer cell ligand); LALBA (Lactalbumin, alpha-); LAMA3 (Laminin, alpha 3 (nicein (150kD), kalinin (165kD), BM600 (150kD), epilegrin)); LAMC1 (Laminin, gamma 1 (formerly LAMB2)); LAMP2 (Lysosomal-associated membrane protein 2); LAMR1 (Laminin receptor 1 (67kD); Ribosomal protein SA); LBP (Lipopolysaccharide-binding protein); LCK (Lymphocyte-specific protein tyrosine kinase); LCN1 (Lipocalin 1 (protein migrating faster than albumin, tear prealbumin)); LCN2 (Lipocalin 2 (oncogene 24p3)); LCP1 (Lymphocyte cytosolic protein 1 (L-plastin)); LCP2 (Lymphocyte cytosolic protein 2 (SH2 domain-containing leukocyte protein of 76kD)); LDHA (Lactate dehydrogenase A); LDLR (Low density lipoprotein receptor (familia X02152); LDLR (Low density lipoprotein receptor (familial hypercholesterolemia)); LECT2 (Leukocyte cell-derived chemotaxin 2); LEP (Leptin (murine obesity homolog)); LEPR (Leptin receptor); LGALS 1 (Lectin, galactoside-binding, soluble, 1 (galectin 1)); LGALS3 (Lectin, galactoside-binding, soluble, 3 (galectin 3)); LGALS3BP (Lectin, galactoside-binding, soluble, 3 binding protein (galectin 6 binding protein)); LGALS9 (Lectin, galactoside-binding, soluble, 9 (galectin 9)); LHB (Luteinizing hormone beta polypeptide); LHCGR (Luteinizing hormone/choriogonadotropin receptor); LIF (Leukemia inhibitory factor (cholinergic differentiation factor)); LIFR (Leukemia inhibitory factor receptor); LIFR (Leukemia inhibitory factor receptor); LIG1 (Ligase I, DNA, ATP-dependent); LKN-1 (Chemokine CC-2,); LMAN1 (Lectin, mannose-binding, 1); LMO4 (LIM domain only 4); LNPEP (Leucyl/cystinyl aminopeptidase); LOX (Lysyl oxidase); LPA (Lipoprotein, Lp(a)); LPL (Lipoprotein lipase); LQT2 (Long (electrocardiographic) QT syndrome 2); LRP1 (Low density lipoprotein-related protein 1 (alpha-2-macroglobulin receptor)); LRP2 (Low density lipoprotein-related protein 2); LSL (Leptin, serum levels of); LSP1 (Lymphocyte-specific protein 1); LTA (Lymphotoxin alpha (TNF superfamily, member 1)); LTB4R (Leukotriene b4 receptor (chemokine receptor-like 1)); LTB4R (Leukotriene b4 receptor (chemokine receptor-like 1)); LTC4S (Leukotriene C4 synthase); LTF (Lactotransferrin); LTK (Leukocyte tyrosine kinase); LU (Lutheran blood group (Auberger b antigen included)); LY64 (Lymphocyte antigen 64 (mouse) homolog, radioprotective, 105kD); LY9 (Lymphocyte antigen 9); LYN (V-yes-1 Yamaguchi sarcoma viral related oncogene homolog); LYZ (Lysozyme (renal amyloidosis)); M1S1 (Membrane component, chromosome 1, surface marker 1 (40kD glycoprotein, identified by monoclonal antibody GA733)); MAB21L1 (Mab-21 (C. elegans)-like 1); MACAM1 (Mucosal addressin cell adhesion molecule-1); MADH1 (MAD (mothers against decapentaplegic, Drosophila) homolog 1); MADH2 (MAD (mothers against decapentaplegic, Drosophila) homolog 2); MADH4 (MAD (mothers against decapentaplegic, Drosophila) homolog 4); MAGEA1 (Melanoma antigen, family A, 1 (directs expression of antigen MZ2-E)); MAGEB1 (Melanoma antigen, family B, 1); MAL (Mal, T-cell differentiation protein); MALL (Mal, T-cell differentiation protein-like); MANB (Mannosidase, alpha B, lysosomal); MAP1B (Microtubule-associated protein 1B); MAPKAPK3 (Mitogen-activated protein kinase-activated protein kinase 3); MASP1 (Mannan-binding lectin serine protease 1 (C4/C2 activating component of Ra-reactive factor)); MAT2A (Methionine adenosyltransferase II, alpha); MATN1 (Matrilin 1, cartilage matrix protein); MATN3 (Matrilin 3); MBL2 (Mannose-binding lectin (protein C) 2, soluble (opsonic defect)); MC1R (Melanocortin 1 receptor (alpha melanocyte stimulating hormone receptor)); MC2R (Melanocortin 2 receptor (adrenocorticotropic hormone)); MCC (Mutated in colorectal cancers); MCF2 (MCF.2 cell line derived transforming sequence); MCP (Membrane cofactor protein (CD46, trophoblast-lymphocyte cross-reactive antigen)); MDF1 (Antigen identified by monoclonal antibody A-3A4); MDH2 (Malate dehydrogenase 2, NAD (mitochondrial)); MDU1 (Antigen identified by monoclonal antibodies 4F2, TRA1.10, TROP4, and T43); ME1 (Malic enzyme 1, soluble); ME2 (Malic enzyme 2, mitochondrial); MEKK1 (MAP/ERK kinase kinase 1); MEKK3 (MAP/ERK kinase kinase 3); MEMO1 (Methylation modifier for class I HLA); MEN1 (Multiple endocrine neoplasia I); MEP1A (Meprin A, alpha (PABA peptide hydrolase)); MER2 (Antigen identified by monoclonal antibodies 1D12, 2F7); MFAP2 (Microfibrillar-associated protein 2); MFAP4 (Microfibrillar-associated protein 4); MFTS (Migraine, familial typical, susceptibility to); MGCT ( MGI); MGP (Matrix Gla protein); MHC2TA (MHC class II transactivator); MIC2 (Antigen identified by monoclonal antibodies 12E7, F21 and 013); MIC5 (Antigen identified by monoclonal antibody R1); MIC7 (Antigen identified by monoclonal antibody 28.3.7); MICA (MHC class I polypeptide-related sequence A); MIF (Macrophage migration inhibitory factor (glycosylation-inhibiting factor)); MIG (Monokine induced by gamma interferon); MIR-10 (Leukocyte immunoglobulin-like receptor); MITF (Microphthalmia-associated transcription factor); MLLT2 (Myeloid/lymphoid or mixed-lineage leukemia (trithorax (Drosophila) homolog); translocated to, 2); MLN (Motilin); MLR (Mineralocorticoid receptor (aldosterone receptor)); MMP12 (Matrix metalloproteinase 12 (macrophage elastase)); MMP13 (Matrix metalloproteinase 13 (collagenase 3)); MMP14 (Matrix metalloproteinase 14 (membrane-inserted)); MMP15 (Matrix metalloproteinase 15 (membrane-inserted)); MMP16 (Matrix metalloproteinase 16 (membrane-inserted)); MMP17 (Matrix metalloproteinase 17 (membrane-inserted)); MMP18 (Matrix metalloproteinase 18); MMP19 (Matrix metalloproteinase 19); MMP2 (Matrix metalloproteinase 2 (gelatinase A, 72kD gelatinase, 72kD type IV collagenase)); MMP20 (Matrix metalloproteinase 20); MMP21 (Matrix metalloproteinase 21); MMP3 (Matrix metalloproteinase 3 (stromelysin 1, progelatinase)); MMP7 (Matrix metalloproteinase 7 (matrilysin, uterine)); MMP8 (Matrix metalloproteinase 8 (neutrophil collagenase)); MMP9 (Matrix metalloproteinase 9 (gelatinase B, 92kD gelatinase, 92kD type IV collagenase)); MNG1 (Multinodular goitre 1); MOG (Myelin oligodendrocyte glycoprotein); MPL (Myeloproliferative leukemia virus oncogene); MPO (Myeloperoxidase); MRBC (Monkey RBC receptor); MRC1 (Mannose receptor, C type 1); MRX20 (Mental retardation, X-linked 20); MSH3 (MutS (E. coli) homolog 3); MSLR1 (Macrophage scavenger receptor-like 1); MSR1 (Macrophage scavenger receptor 1); MSS (Marinesco-Sjogren syndrome); MSSE (Multiple self-healing squamous epithelioma); MST1 (Macrophage stimulating 1 (hepatocyte growth factor-like)); MST1R (Macrophage stimulating 1 receptor (c-met-related tyrosine kinase)); MSX2 (Msh (Drosophila) homeo box homolog 2); MTCO1 (Cytochrome c oxidase I); MTHFD (5,10-methylenetetrahydrofolate dehydrogenase, 5,10-methylenetetrahydrofolate cyclohydrolase, 10-formyltetrahydrofolate synthetase); MTHFR (5,10-methylenetetrahydrofolate reductase (NADPH)); MTND2 (NADH dehydrogenase 2); MTP (Microsomal triglyceride transfer protein (large polypeptide, 88kD)); MTR (5-methyltetrahydrofolate-homocysteine methyltransferase); MTRR (5-methyltetrahydrofolate-homocysteine methyltransferase reductase); MUC1 (Mucin 1, transmembrane); MUC2 (Mucin 2, intestinal/tracheal); MUC4 (Mucin 4, tracheobronchial); MUL (Mulibrey nanism); MUT (Methylmalonyl Coenzyme A mutase); MX1 (Myxovirus (influenza) resistance 1, homolog of murine (interferon-inducible protein p78)); MXI1 (MAX-interacting protein 1); MYB (V-myb avian myeloblastosis viral oncogene homolog); MYBPC3 (Myosin-binding protein C, cardiac); MYC (V-myc avian myelocytomatosis viral oncogene homolog); MYCL1 (V-myc avian myelocytomatosis viral oncogene homolog 1, lung carcinoma derived); MYD88 (Myeloid differentiation primary response gene (88)); MYF5 (Myogenic factor 5); MYF6 (Myogenic factor 6 (herculin)); MYO5A (Myosin VA (heavy polypeptide 12, myoxin)); MYO9B (Myosin IXB); NAB 1 (NGFI-A binding protein 1 (ERG1 binding protein 1)); NAGA (N-acetylgalactosaminidase, alpha-); NAIP (Neuronal apoptosis inhibitory protein); NAPA (N-ethylmaleimide-sensitive factor attachment protein, alpha); NAPB ); NAPG (N-ethylmaleimide-sensitive factor attachment protein, gamma); NAT1 (N-acetyltransferase 1 (arylamine N-acetyltransferase)); NAT2 (N-acetyltransferase 2 (arylamine N-acetyltransferase)); NB (Neuroblastoma (neuroblastoma suppressor)); NCAM1 (Neural cell adhesion molecule 1); NCF 1 (Neutrophil cytosolic factor 1 (47kD, chronic granulomatous disease, autosomal 1)); NCF2 (Neutrophil cytosolic factor 2 (65kD, chronic granulomatous disease, autosomal 2)); NCF4 (Neutrophil cytosolic factor 4 (40kD)); NDP (Norrie disease (pseudoglioma)); NDUFS2 (NADH dehydrogenase (ubiquinone) Fe-S protein 2 (49kD) (NADH-coenzyme Q reductase)); NEB (Nebulin); NEU (Neuraminidase); NF1 (Neurofibromin 1 (neurofibromatosis, von Recklinghausen disease, Watson disease)); NF2 (Neurofibromin 2 (bilateral acoustic neuroma)); NFATC1 (Nuclear factor of activated T-cells, cytoplasmic 1); NFATC3 (Nuclear factor of activated T-cells, cytoplasmic 3); NFATC4 (Nuclear factor of activated T-cells, cytoplasmic 4); NFE2 (Nuclear factor (erythroid-derived 2), 45kD); NFE2L2 (Nuclear factor (erythroid-derived 2)-like 2); NFIL3 (Nuclear factor, interleukin 3 regulated); NFKB1 (Nuclear factor of kappa light polypeptide gene enhancer in B-cells 1 (p105)); NFKB2 (Nuclear factor of kappa light polypeptide gene enhancer in B-cells 2 (p49/p100)); NFKBIA (Nuclear factor of kappa light polypeptide gene enhancer in B-cells inhibitor, alpha); NFRKB (Nuclear factor related to kappa B binding protein); NFYA (Nuclear transcription factor Y, alpha); NGFB (Nerve growth factor, beta polypeptide); NKS 1 (Natural killer cell susceptibility 1); NM (Neutrophil migration); NME1 (Non-metastatic cells 1, protein (NM23A) expressed in); NMOR2 (NAD(P)H menadione oxidoreductase 2, dioxin-inducible); NNMT (Nicotinamide N-methyltransferase); NOS 1 (Nitric oxide synthase 1 (neuronal)); NOS2A (Nitric oxide synthase 2A (inducible, hepatocytes)); NOS2B (Nitric oxide synthase 2B); NOS2C (Nitric oxide synthase 2C); NOS3 (Nitric oxide synthase 3 (endothelial cell)); NOTCH1 (Notch (Drosophila) homolog 1 (translocation-associated)); NOTCH4 (Notch (Drosophila) homolog 4); NP (Nucleoside phosphorylase); NPC1 (Niemann-Pick disease, type C1); NPHP1 (Nephronophthisis 1 (juvenile)); NPY1R (Neuropeptide Y receptor Y1); NRAMP1 (Natural resistance-associated macrophage protein 1 (might include Leishmaniasis)); NRAMP2 (Natural resistance-associated macrophage protein 2); NRAS (Neuroblastoma RAS viral (v-ras) oncogene homolog); NRL (Neural retina leucine zipper); NT5 (5' nucleotidase (CD73)); NTF3 (Neurotrophin 3); NUCB1 (Nucleobindin 1); NUMA1 (Nuclear mitotic apparatus protein 1); NURR1 (Nuclear receptor related 1 (transcriptionally inducible)); OA1 (Ocular albinism 1 (Nettleship-Falls)); OAS1 (2',5'-oligoadenylate synthetase 1); OAS2 (2'-5'oligoadenylate synthetase 2); OAT (Ornithine aminotransferase (gyrate atrophy)); OCRL (Oculocerebrorenal syndrome of Lowe); ODC 1 (Ornithine decarboxylase 1); OGG1 (8-oxoguanine DNA glycosylase); OLFR2 (Olfactory receptor 2); OMG (Oligodendrocyte myelin glycoprotein); OPA1 (Optic atrophy 1 (autosomal dominant)); OPA3 (Iraqi-Jewish optic atrophy plus (3-methylglutaconicaciduria type 3)); OPLL ); OPRM1 (Opioid receptor, mu 1); OPTA2 (Osteopetrosis, autosomal dominant, type II); OPTB1 (Osteopetrosis, autosomal recessive); OR1D2 (Olfactory receptor, family 1, subfamily D, member 2); ORCTL2 (Beckwith-Wiedemann syndrome chromosome region 1, candidate A; Organic cation transporter-like 2; Imprinted polyspecific membrane transporter 1); ORM1 (Orosomucoid 1); ORM2 (Orosomucoid 2); OSM (Oncostatin M); OTC (Ornithine carbamoyltransferase); OXT (Oxytocin, prepro- (neurophysin I)); P (P blood group globoside); P1 (P blood group (P one antigen)); P2RX1 (Purinergic receptor P2X, ligand-gated ion channel, 1); P2RY1 (Purinergic receptor P2Y, G-protein coupled, 1); PA2G4 (Proliferation-associated 2G4, 38kD); PAC1 (Prostate adenocarcinoma-1); PACE (Paired basic amino acid cleaving enzyme (furin, membrane associated receptor protein)); PAEP (Progestagen-associated endometrial protein (placental protein 14, pregnancy-associated endometrial alpha-2-globulin, alpha uterine protein)); PAFAH (Platelet-activating factor acetylhydrolase); PAFAH1B1 (Platelet-activating factor acetylhydrolase, isoform Ib, alpha subunit (45kD)); PAFAH1B2 (Platelet-activating factor acetylhydrolase, isoform Ib, beta subunit (30kD)); PAFAH2 (Platelet-activating factor acetylhydrolase 2 (40kD)); PAH (Phenylalanine hydroxylase); PAI1 (Plasminogen activator inhibitor, type I); PAPPA (Pregnancy-associated plasma protein A); PAR4 (Protease-activated receptor-4 PAWR); PRKC, apoptosis, WT1, regulator); PAX2 (Paired box gene 2); PAX3 (Paired box gene 3 (Waardenburg syndrome 1)); PAX8 (Paired box gene 8); PCBD (6-pyruvoyl-tetrahydropterin synthase/dimerization cofactor of hepatocyte nuclear factor 1 alpha (TCF1)); PCCA (Propionyl Coenzyme A carboxylase, alpha polypeptide); PCCB (Propionyl Coenzyme A carboxylase, beta polypeptide); PCI (Protein C inhibitor (plasminogen activator inhibitor III)); PCK1 (Phosphoenolpyruvate carboxykinase 1 (soluble)); PCM1 (Pericentriolar material 1); PCNT (Pericentrin); PCTK1 (PCTAIRE protein kinase 1); PCYT2 (Phosphate cytidylyltransferase 2, ethanolamine); PDB2); PDCD2 (Programmed cell death 2); PDE3B (Phosphodiesterase 3B, cGMP-inhibited); PDE4A (Phosphodiesterase 4A, cAMP-specific (dunce (Drosophila)-homolog phosphodiesterase E2)); PDE4B (Phosphodiesterase 4B, cAMP-specific (dunce (Drosophila)-homolog phosphodiesterase E4)); PDE7A (Phosphodiesterase 7A); PDES1B (Phosphodiesterase IB); PDGFA (Platelet-derived growth factor alpha polypeptide); PDGFB (Platelet-derived growth factor beta polypeptide (simian sarcoma viral (v-sis) oncogene homolog)); PDGFRA (Platelet-derived growth factor receptor, alpha polypeptide); PDGFRB (Platelet-derived growth factor receptor, beta polypeptide); PDHA1 (Pyruvate dehydrogenase (lipoamide) alpha 1); PDX1 (Pyruvate dehydrogenase complex, lipoyl-containing component X; E3-binding protein); PECAM1 (Platelet/endothelial cell adhesion molecule (CD31 antigen)); PEPC (Peptidase C); PEPD (Peptidase D); PEX10 (Peroxisome biogenesis factor 10); PF4 (Platelet factor 4); PF4V 1 (Platelet factor 4 variant 1); PFBI (Plasmodium falciparum blood infection levels); PFC (Properdin P factor, complement); PFKL (Phosphofructokinase, liver); PFKM (Phosphofructokinase, muscle); PFKP (Phosphofructokinase, platelet); PFN1 (Profilin 1); PGA3 (Pepsinogen 3, group I (pepsinogen A)); PGC (Progastricsin (pepsinogen C)); PGD (Phosphogluconate dehydrogenase); PGF (Placental growth factor, vascular endothelial growth factor-related protein); PGK1 (Phosphoglycerate kinase 1); PGK2 (Phosphoglycerate kinase 2); PGL1 (Paraganglioma or familial glomus tumors 1); PGM1 (Phosphoglucomutase 1); PGM3 (Phosphoglucomutase 3); PGP (Phosphoglycolate phosphatase); PGY1 (P glycoprotein 1/multiple drug resistance 1); PHAP1 (Putative human HLA class II associated protein I); PHB (Prohibitin); PI (Protease inhibitor 1 (anti-elastase), alpha-1-antitrypsin); PI3 (Protease inhibitor 3, skin-derived (SKALP)); PI6 (Protease inhibitor 6 (placental thrombin inhibitor)); PI8 (Protease inhibitor 8 (ovalbumin type)); PI9 (Protease inhibitor 9 (ovalbumin type)); PIGA (Phosphatidylinositol glycan, class A (paroxysmal nocturnal hemoglobinuria)); PIGF (Phosphatidylinositol glycan, class F); PIGR (Polymeric immunoglobulin receptor); PIK3CA (Phosphoinositide-3-kinase, catalytic, alpha polypeptide); PIK3CB (Phosphoinositide-3-kinase, catalytic, beta polypeptide); PIK3R1 (Phosphoinositide-3-kinase, regulatory subunit, polypeptide 1 (p85 alpha)); PIL (Protease inhibitor 1 (alpha-1-antitrypsin)-like); PIN (Dynein, cytoplasmic, light polypeptide); PKLR (Pyruvate kinase, liver and RBC); PLAGL1 (Pleomorphic adenoma gene-like 1); PLAT (Plasminogen activator, tissue); PLCD1 (Phospholipase C, delta 1); PLCG1 (Phospholipase C, gamma 1 (formerly subtype 148)); PLEK (Pleckstrin); PLG (Plasminogen); PLOD (Procollagen-lysine, 2-oxoglutarate 5-dioxygenase (lysine hydroxylase, Ehlers-Danlos syndrome type VI)); PLP (Proteolipid protein (Pelizaeus-Merzbacher disease, spastic paraplegia 2, uncomplicated)); PLT1 (Primed lymphocyte test-1); PML (Promyelocytic leukemia); PMP22 (Peripheral myelin protein 22); PMS2 (Postmeiotic segregation increased (S. cerevisiae) 2); PNMT (Phenylethanolamine N-methyltransferase); PNUTL1 (Peanut (Drosophila)-like 1); POLB (Polymerase (DNA directed), beta); POMC (Proopiomelanocortin (adrenocorticotropin/beta-lipotropin/ alpha-melanocyte stimulating hormone/beta-melanocyte stimulating hormone/ beta-endorphin)); PON1 (Paraoxonase 1); PON2 (Paraoxonase 2); PORC (Porphyria, acute; Chester type); POU2AF1 (POU domain, class 2, associating factor 1); POU5F1 (POU domain, class 5, transcription factor 1); PPBP (Pro-platelet basic protein (includes platelet basic protein, beta-thromboglobulin, connective tissue-activating peptide III, neutrophil-activating peptide-2)); PPCD (Posterior polymorphous corneal dystrophy); PPH1 (Primary pulmonary hypertension 1); PPIB (Peptidylprolyl isomerase B (cyclophilin B)); PPOX (Protoporphyrinogen oxidase); PPP1R8 (Protein phosphatase 1, regulatory (inhibitor) subunit 8); PRB1 (Proline-rich protein BstNI subfamily 1); PRB2 (Proline-rich protein BstNI subfamily 2); PRB3 (Proline-rich protein BstNI subfamily 3); PRB4 (Proline-rich protein BstNI subfamily 4); PREP (Prolyl endopeptidase); PRF1 (Perforin 1 (preforming protein)); PRG1 (Proteoglycan 1, secretory granule); PRH1 (Proline-rich protein HaeIII subfamily 1); PRH2 (Proline-rich protein HaeIII subfamily 2); PRKCQ (Protein kinase C, theta); PRKDC (Protein kinase, DNA-activated, catalytic polypeptide); PRKG1 (Protein kinase, cGMP-dependent, type I); PRKM10 (Protein kinase mitogen-activated 10 (MAP kinase)); PRKM9 (Protein kinase mitogen-activated 9 (MAP kinase)); PRL (Prolactin); PRLR (Prolactin receptor); PRNP (Prion protein (p27-30) (Creutzfeld-Jakob disease, Gerstmann-Strausler-Scheinker syndrome, fatal familial insomnia)); PROC (Protein C (inactivator of coagulation factors Va and VIIIa)); PROP1 (Prophet of Pit1, paired-like homeodomain transcription factor); PROS1 (Protein S (alpha)); PRPH (Peripherin); PRSS1 (Protease, serine, 1 (trypsin 1)); PRSS2 (Protease, serine, 2 (trypsin 2)); PRSS7 (Protease, serine, 7 (enterokinase)); PRSS8 (Protease, serine, 8 (prostasin)); PRTN3 (Proteinase 3 (serine proteinase, neutrophil, Wegener granulomatosis autoantigen)); PSAP (Prosaposin (variant Gaucher disease and variant metachromatic leukodystrophy)); PSD (Pleckstrin and Sec7 domain protein); PSMB8 (Proteasome (prosome, macropain) subunit, beta type, 8 (large multifunctional protease 7)); PSORS 1 (Psoriasis susceptibility 1); PSORS2 (Psoriasis susceptibility 2); PSORS3 (Psoriasis susceptibility 3); PTAFR (Platelet-activating factor receptor); PTC (Phenylthiocarbamide tasting); PTCH (Patched (Drosophila) homolog); PTEN (Phosphatase and tensin homolog (mutated in multiple advanced cancers 1)); PTGDS (Prostaglandin D2 synthase (21kD, brain)); PTGER3 (Prostaglandin E receptor 3 (subtype EP3)); PTGIR (Prostaglandin 12 (prostacyclin) receptor (IP)); PTGS 1 (Prostaglandin-endoperoxide synthase 1 (prostaglandin G/H synthase and cyclooxygenase)); PTGS2 (Prostaglandin-endoperoxide synthase 2 (prostaglandin G/H synthase and cyclooxygenase)); PTK2B (Protein tyrosine kinase 2 beta); PTN (Pleiotrophin (heparin binding growth factor 8, neurite growth-promoting factor 1)); PTPN13 (Protein tyrosine phosphatase, non-receptor type 13 (APO-1/CD95 (Fas)-associated phosphatase)); PTPN6 (Protein tyrosine phosphatase, non-receptor type 6); PTPRC (Protein tyrosine phosphatase, receptor type, c polypeptide); PTPRCAP (Protein tyrosine phosphatase, receptor type, c polypeptide-associated protein); PTPRD (Protein tyrosine phosphatase, receptor type, D); PTPRF (Protein tyrosine phosphatase, receptor type, F); PTPRG (Protein tyrosine phosphatase, receptor type, gamma polypeptide); PTX3 (Pentaxin-related gene, rapidly induced by IL-1 beta PUJO ); PVR (Poliovirus receptor); PVRL1 (Poliovirus receptor-like 1); PVRL2 (Poliovirus receptor-like 2); PXE (Pseudoxanthoma elasticum); PXMP1 (Peroxisomal membrane protein 1 (70kD, Zellweger syndrome)); PXN (Paxillin); PYCR1 (Pyrroline-5-carboxylate reductase 1); PYGM (Phosphorylase, glycogen; muscle (McArdle syndrome, glycogen storage disease type V)); QDPR (Quinoid dihydropteridine reductase); RAC2 (Ras-related C3 botulinum toxin substrate 2 (rho family, small GTP binding protein Rac2)); RAC3 (Ras-related C3 botulinum toxin substrate 3 (rho family, small GTP binding protein Rac3)); RAD17 (RAD17 (S. pombe) homolog); RAD51L3 (RAD51 (S. cerevisiae)-like 3); RAF1 (V-raf-1 murine leukemia viral oncogene homolog 1); RAG1 (Recombination activating gene 1); RAG2 (Recombination activating gene 2); RANBP3 (RAN binding protein 3); RAP1A (RAP1A, member of RAS oncogene family); RB1 (Retinoblastoma 1 (including osteosarcoma)); RBP4 (Retinol-binding protein 4, interstitial); RBS (Roberts syndrome); RCN2 (Reticulocalbin 2, EF-hand calcium binding domain); RCP (Red cone pigment (color blindness, protan)); RCV1 (Recoverin); RDBP (RD RNA-binding protein); RDS (Retinal degeneration, slow (retinitis pigmentosa 7)); RELA (V-rel avian reticuloendotheliosis viral oncogene homolog A (nuclear factor of kappa light polypeptide gene enhancer in B-cells 3 (p65))); REN (Renin); RENBP (Renin-binding protein); REQ (Requiem, apoptosis response zinc finger gene); RFX1 (Regulatory factor X, 1 (influences HLA class II expression)); RFX2 (Regulatory factor X, 2 (influences HLA class II expression)); RFX3 (Regulatory factor X, 3 (influences HLA class II expression)); RFX4 (Regulatory factor X, 4 (influences HLA class II expression)); RFX5 (Regulatory factor X, 5 (influences HLA class II expression)); RFXAP (Regulatory factor X-associated protein); RGS2 (Regulator of G-protein signalling 2, 24kD); RHCE (Rhesus blood group, CcEe antigens); RHD (Rhesus blood group, D antigen); RHO (Rhodopsin (retinitis pigmentosa 4, autosomal dominant)); RMSA1 (Regulator of mitotic spindle assembly 1); RN5S1@ (RNA, 5S cluster 1); RNR1 (RNA, ribosomal 1); RNU1A (RNA, U1A small nuclear); RNU2 (RNA, U2 small nuclear); ROM1 (Retinal outer segment membrane protein 1); RP2 (Retinitis pigmentosa 2 (X-linked recessive)); RPE65 (Retinal pigment epithelium-specific protein (65kD)); RPL7A (Ribosomal protein L7a); RPS4X (Ribosomal protein S4, X-linked); RRAD (Ras-related associated with diabetes); RRM1 (Ribonucleotide reductase M 1 polypeptide); RSN (Restin (Reed-Steinberg cell-expressed intermediate filament-associated protein)); RTS (Rothmund-Thomson syndrome); RXRB (Retinoid X receptor, beta); RYR1 (Ryanodine receptor 1 (skeletal)); S100A4 (S100 calcium-binding protein A4 (calcium protein, calvasculin, metastasin, murine placental homolog)); S100A7 (S100 calcium-binding protein A7 (psoriasin 1)); S100A8 (S100 calcium-binding protein A8 (calgranulin A)); SAA1 (Serum amyloid A1); SAG (S-antigen; retina and pineal gland (arrestin)); SAR1 (RasGAP-like with IQ motifs); SCLC1); SCN1B (Sodium channel, voltage-gated, type I, beta polypeptide); SCN4A (Sodium channel, voltage-gated, type IV, alpha polypeptide); SCN5A (Sodium channel, voltage-gated, type V, alpha polypeptide (long (electrocardiographic) QT syndrome 3)); SCNN1G (Sodium channel, nonvoltage-gated 1, gamma); SCYA1 (Small inducible cytokine A1 (I-309, homologous to mouse Tca-3)); SCYA11 (Small inducible cytokine subfamily A (Cys-Cys), member 11 (eotaxin)); SCYA13 (Small inducible cytokine subfamily A (Cys-Cys), member 13); SCYA14 (Small inducible cytokine subfamily A (Cys-Cys), member 14); SCYA15 (Small inducible cytokine subfamily A (Cys-Cys), member 15); SCYA16 (Small inducible cytokine subfamily A (Cys-Cys), member 16); SCYA17 (Small inducible cytokine subfamily A (Cys-Cys), member 17); SCYA18 (Small inducible cytokine subfamily A (Cys-Cys), member 18, pulmonary and activation-regulated); SCYA19 (Small inducible cytokine subfamily A (Cys-Cys), member 19); SCYA2 (Small inducible cytokine A2 (monocyte chemotactic protein 1, homologous to mouse Sig-je)); SCYA20 (Small inducible cytokine subfamily A (Cys-Cys), member 20); SCYA21 (Small inducible cytokine subfamily A (Cys-Cys), member 21); SCYA22 (Small inducible cytokine subfamily A (Cys-Cys), member 22); SCYA23 (Small inducible cytokine subfamily A (Cys-Cys), member 23); SCYA24 (Small inducible cytokine subfamily A (Cys-Cys), member 24); SCYA25 (Small inducible cytokine subfamily A (Cys-Cys), member 25); SCYA3 (Small inducible cytokine A3 (homologous to mouse Mip-1a)); SCYA3L1 (Small inducible cytokine A3-like 1); SCYA4 (Small inducible cytokine A4 (homologous to mouse Mip-1b)); SCYA5 (Small inducible cytokine A5 (RANTES)); SCYA7 (Small inducible cytokine A7 (monocyte chemotactic protein 3)); SCYA8 (Small inducible cytokine subfamily A (Cys-Cys), member 8 (monocyte chemotactic protein 2)); SCYB5 (Small inducible cytokine subfamily B (Cys-X-Cys), member 5 (epithelial-derived neutrophil-activating peptide 78)); SCYB6 (Small inducible cytokine subfamily B (Cys-X-Cys), member 6 (granulocyte chemotactic protein 2)); SCYC1 (Small inducible cytokine subfamily C, member 1 (lymphotactin)); SCYD1 (Small inducible cytokine subfamily D (Cys-X3-Cys), member 1 (fractalkine, neurotactin)); SDF1 (Stromal cell-derived factor 1); SDHC (Succinate dehydrogenase complex, subunit C, integral membrane protein, 15kD); SELE (Selectin E (endothelial adhesion molecule 1)); SELL (Selectin L (lymphocyte adhesion molecule 1)); SELP (Selectin P (granule membrane protein 140kD, antigen CD62)); SELPLG (Selectin P ligand); SERK1 (SAPK/Erk kinase 1); SF (Stoltzfus blood group); SFTPA1 (Surfactant, pulmonary-associated protein A1); SFTPA2 (Surfactant, pulmonary-associated protein A2); SFTPB (Surfactant, pulmonary-associated protein B); SFTPD (Surfactant, pulmonary-associated protein D); SGCB (Sarcoglycan, beta (43kD dystrophin-associated glycoprotein)); SGCD (Sarcoglycan, delta (35kD dystrophin-associated glycoprotein)); SGSH (N-sulfoglucosamine sulfohydrolase (sulfamidase)); SH2D1A (SH2 domain protein 1A, Duncan's disease (lymphoproliferative syndrome)); SHH (Sonic hedgehog (Drosophila) homolog); SHMT2 (Serine hydroxymethyltransferase 2 (mitochondrial)); SHOX (Short stature homeobox); SIAH1 (Seven in absentia (Drosophila) homolog 1); SIPA1 (Signal-induced proliferation-associated gene 1); SKIV2L (Superkiller viralicidic activity 2 (S. cerevisiae homolog)-like); SLC12A1 (Solute carrier family 12 (sodium/potassium/chloride transporters), member 1); SLC14A1 (Solute carrier family 14 (urea transporter), member 1 (Kidd blood group)); SLC18A2 (Solute carrier family 18 (vesicular monoamine), member 2); SLC1A5 (Solute carrier family 1 (neutral amino acid transporter), member 5); SLC20A1 (Solute carrier family 20 (phosphate transporter), member 1); SLC20A2 (Solute carrier family 20 (phosphate transporter), member 2); SLC2A1 (Solute carrier family 2 (facilitated glucose transporter), member 1); SLC2A2 (Solute carrier family 2 (facilitated glucose transporter), member 2); SLC2A4 (Solute carrier family 2 (facilitated glucose transporter), member 4); SLC3A1 (Solute carrier family 3 (cystine, dibasic and neutral amino acid transporters, activator of cystine, dibasic and neutral amino acid transport), member 1); SLC4A1 (Solute carrier family 4, anion exchanger, member 1 (erythrocyte membrane protein band 3, Diego blood group) ); SLC5A5 (Solute carrier family 5 (sodium iodide symporter), member 5); SLC6A2 (Solute carrier family 6 (neurotransmitter transporter, noradrenalin), member 2); SLC6A3 (Solute carrier family 6 (neurotransmitter transporter, dopamine), member 3); SLC6A4 (Solute carrier family 6 (neurotransmitter transporter, serotonin), member 4); SLC7A7 (Solute carrier family 7 (cationic amino acid transporter, y+ system), member 7); SLC9A1 (Solute carrier family 9 (sodium/hydrogen exchanger), isoform 1 (antiporter, Na+/H+, amiloride sensitive)); SLEB 1 (Systemic lupus erythematosus susceptibility 1); SLPI (Secretory leukocyte protease inhibitor (antileukoproteinase)); SM1 (Schistosoma mansoni, susceptibility/resistance to); SMN1 (Survival of motor neuron 1, telomeric); SNAP23 (Synaptosomal-associated protein, 23kD); SNCG (Synuclein, gamma (breast cancer-specific protein 1)); SNRP70 (Small nuclear ribonucleoprotein 70kD polypeptide (RNP antigen)); SNRPB (Small nuclear ribonucleoprotein polypeptides B and B1); SNRPN (Small nuclear ribonucleoprotein polypeptide N); SOAT1 (Sterol O-acyltransferase (acyl-Coenzyme A: cholesterol acyltransferase) 1); SOD1 (Superoxide dismutase 1, soluble (amyotrophic lateral sclerosis 1 (adult))); SOD2 (Superoxide dismutase 2, mitochondrial); SORL1 (Sortilin-related receptor, L(DLR class) A repeats-containing); SOX10 (SRY (sex-determining region Y)-box 10); SOX4 (SRY (sex determining region Y)-box 4); SPG3A (Spastic paraplegia 3A (autosomal dominant)); SPN (Sialophorin (gpL115, leukosialin, CD43)); SPN (Sialophorin (gpL115, leukosialin, CD43)); SPP1 (Secreted phosphoprotein 1 (osteopontin, bone sialoprotein I, early T-lymphocyte activation 1)); SPTA1 (Spectrin, alpha, erythrocytic 1 (elliptocytosis 2)); SRD5A2 (Steroid-5-alpha-reductase, alpha polypeptide 2 (3-oxo-5 alpha-steroid delta 4-dehydrogenase alpha 2)); SSA2 (Sjogren syndrome antigen A2 (60kD, ribonucleoprotein autoantigen SS-A/Ro)); SSB (Sjogren syndrome antigen B (autoantigen La)); SSTR1 (Somatostatin receptor 1); ST3 (Suppression of tumorigenicity 3); STAM (Signal transducing adaptor molecule (SH3 domain and ITAM motif) 1); STAT1 (Signal transducer and activator of transcription 1, 91kD); STAT2 (Signal transducer and activator of transcription 2, 113kD); STAT3 (Signal transducer and activator of transcription 3 (acute-phase response factor)); STAT4 (Signal transducer and activator of transcription 4); STAT5A (Signal transducer and activator of transcription 5A); STAT6 (Signal transducer and activator of transcription 6, interleukin-4 induced); STATH (Statherin); STATI2 (STAT induced STAT inhibitor-2); STX1B (Syntaxin 1B); SULT1A1 (Sulfotransferase family 1A, phenol-preferring, member 1); SULT1A3 (Sulfotransferase family 1A, phenol-preferring, member 3); SULT2A1 (Sulfotransferase family 2A, dehydroepiandrosterone (DHEA) -preferring, member 1); SUOX (Sulfite oxidase); SUR (Sulfonylurea receptor (hyperinsulinemia)); SURF1 (Surfeit 1); SW (Swann blood group); T (T brachyury (mouse) homolog); TAP1 (Transporter 1, ABC (ATP binding cassette)); TAP2 (Transporter 2, ABC (ATP binding cassette)); TAT (Tyrosine aminotransferase); TAZ (Tafazzin (cardiomyopathy, dilated 3A (X-linked), endocardial fibroelastosis 2; Barth syndrome)); TBG (Thyroxin-binding globulin); TBP (TATA box binding protein); TBX2 (T-box 2); TBXA2R (Thromboxane A2 receptor); TBXAS1 (Thromboxane A synthase 1 (platelet, cytochrome P450, subfamily V)); TCF1 (Transcription factor 1, hepatic; LF-B1, hepatic nuclear factor (HNF1), albumin proximal factor); TCF19 (Transcription factor 19 (SC1)); TCF3 (Transcription factor 3 (E2A immunoglobulin enhancer binding factors E12/E47)); TCF7 (Transcription factor 7 (T-cell specific, HMG-box)); TCF8 (Transcription factor 8 (represses interleukin 2 expression)); TCL1A (T-cell leukemia/lymphoma 1A); TCL4 (T-cell leukemia/lymphoma 4); TCN1 (Transcobalamin I (vitamin B12 binding protein, R binder family)); TCN2 (Transcobalamin II; macrocytic anemia); TCP1 (T-complex 1); TCRA (T-cell receptor, alpha (V,D,J,C)); TCRB (T-cell receptor, beta cluster); TCRB (T-cell receptor, beta cluster); TCRG (T-cell receptor, gamma cluster); TCTE1 (T-complex-associated-testis-expressed 1); TDO2 (Tryptophan 2,3-dioxygenase); TECTA (Tectorin alpha); TERF2 (Telomeric repeat binding factor 2); TF (Transferrin); TFF2 (Trefoil factor 2 (spasmolytic protein 1)); TFPI (Tissue factor pathway inhibitor (lipoprotein-associated coagulation inhibitor)); TFPI2 (Tissue factor pathway inhibitor-2); TFRC (Transferrin receptor (p90, CD71)); TG (Thyroglobulin); TGFB 1 (Transforming growth factor, beta 1); TGFB2 (Transforming growth factor, beta 2); TGFB3 (Transforming growth factor, beta 3); TGFBI (Transforming growth factor, beta-induced, 68kD); TGFBR3 (Transforming growth factor, beta receptor III (betaglycan, 300kD)); TGM1 (Transglutaminase 1 (K polypeptide epidermal type I, protein-glutamine-gamma-glutamyltransferase)); TGM2 (Transglutaminase 2 (C polypeptide, protein-glutamine-gamma-glutamyltransferase)); TH (Tyrosine hydroxylase); THBS1 (Thrombospondin 1); THBS2 (Thrombospondin 2); THPO (Thrombopoietin (myeloproliferative leukemia virus oncogene ligand, megakaryocyte growth and development factor)); THRA (Thyroid hormone receptor, alpha (avian erythroblastic leukemia viral (v-erb-a) oncogene homolog)); THRB (Thyroid hormone receptor, beta (avian erythroblastic leukemia viral (v-erb-a) oncogene homolog 2)); THY1 (Thy-1 cell surface antigen); TIEG (TGFB inducible early growth response); TIMP3 (Tissue inhibitor of metalloproteinase 3 (Sorsby fundus dystrophy, pseudoinflammatory)); TK1 (Thymidine kinase 1, soluble); TKT (Transketolase (Wernicke-Korsakoff syndrome)); TLR1 (Toll-like receptor 1); TLR2 (Toll-like receptor 2); TLR3 (Toll-like receptor 3); TLR4 (Toll-like receptor 4); TLR5 (Toll-like receptor 5); TM4SF7 (Transmembrane 4 superfamily member 7); TMEM 1 (Transmembrane protein 1); TNF (Tumor necrosis factor (TNF superfamily, member 2)); TNFAIP2 (Tumor necrosis factor, alpha-induced protein 2); TNFAIP6 (Tumor necrosis factor, alpha-induced protein 6); TNFRSF11B (Tumor necrosis factor receptor superfamily, member 11b (osteoprotegerin)); TNFRSF12 (Tumor necrosis factor receptor superfamily, member 12 (translocating chain-association membrane protein)); TNFRSF14 (Tumor necrosis factor receptor superfamily, member 14 (herpesvirus entry mediator)); TNFRSF17 (Tumor necrosis factor receptor superfamily, member 17); TNFRSF1A (Tumor necrosis factor receptor superfamily, member 1A); TNFRSF1B (Tumor necrosis factor receptor superfamily, member 1B); TNFRSF5 (Tumor necrosis factor receptor superfamily, member 5); TNFRSF6 (Tumor necrosis factor receptor superfamily, member 6); TNFRSF6B (Tumor necrosis factor receptor superfamily, member 6b, decoy); TNFRSF7 (Tumor necrosis factor receptor superfamily, member 7); TNFRSF9 (Tumor necrosis factor receptor superfamily, member 9); TNFSF11 (Tumor necrosis factor (ligand) superfamily, member 11); TNFSF12 (Tumor necrosis factor (ligand) superfamily, member 12); TNFSF14 (Tumor necrosis factor (ligand) superfamily, member 14); TNFSF5 (Tumor necrosis factor (ligand) superfamily, member 5); TNFSF6 (Tumor necrosis factor (ligand) superfamily, member 6); TNNT2 (Troponin T2, cardiac); TP53 (Tumor protein p53 (Li-Fraumeni syndrome)); TP73 (Tumor protein p73); TPH (Tryptophan hydroxylase (tryptophan 5-monooxygenase)); TPI1 (Triosephosphate isomerase 1); TPM1 (Tropomyosin 1 (alpha)); TPMT (Thiopurine S-methyltransferase); TPO (Thyroid peroxidase); TPT1 (Tumor protein, translationally-controlled 1); TRAF1 (TNF receptor-associated factor 1); TRAF1 (TNF receptor-associated factor 1); TRAF2 (TNF receptor-associated factor 2); TRAF3 (TNF receptor-associated factor 3); TRAF4 (TNF receptor-associated factor 4); TRAF5 (TNF receptor-associated factor 5); TRAF6 (TNF receptor-associated factor 6); TRP 1 (TRNA proline 1); TSHB (Thyroid stimulating hormone, beta); TSHR (Thyroid stimulating hormone receptor); TSSC3 (Tumor suppressing subtransferable candidate 3); TST (Thiosulfate sulfurtransferase (rhodanese)); TSTA3 (Tissue specific transplantation antigen P35B); TTIM1 (T-cell tumor invasion and metastasis 1); TTR (Transthyretin (prealbumin, amyloidosis type I)); TUB (Tubby (mouse) homolog); TUBA3 (Tubulin, alpha, brain-specific); TUBAL1 (Tubulin, alpha-like 1); TUBB (Tubulin, beta polypeptide); TWIST (Twist (Drosophila) homolog); TXN (Thioredoxin); U2AF1 (U2(RNU2) small nuclear RNA auxillary factor 1 (non-standard symbol)); UBC (Ubiquitin C); UBE1 (Ubiquitin-activating enzyme E1 (A1S9T and BN75 temperature sensitivity complementing)); UBE2B (Ubiquitin-conjugating enzyme E2B (RAD6 homolog)); UBE2I (Ubiquitin-conjugating enzyme E2I (homologous to yeast UBC9)); UBE2V2 (Ubiquitin-conjugating enzyme E2 variant 2); UBE3A (Ubiquitin protein ligase E3A (human papilloma virus E6-associated protein, Angelman syndrome)); UBL1 (Ubiquitin-like 1 (sentrin)); UCP2 (Uncoupling protein 2 (mitochondrial, proton carrier)); UCP3 (Uncoupling protein 3 (mitochondrial, proton carrier)); UFS (Urofacial syndrome); UGB (Uteroglobin); UGDH (UDP-glucose dehydrogenase); UGT1 (UDP glycosyltransferase 1); UMPK (Uridine monophosphate kinase); UMPS (Uridine monophosphate synthetase (orotate phosphoribosyl transferase and); orotidine-5'-decarboxylase)); UP (Uridine phosphorylase); UPK1B (Uroplakin 1B); UROD (Uroporphyrinogen decarboxylase); UROS (Uroporphyrinogen III synthase (congenital erythropoietic porphyria)); USH2A (Usher syndrome 2A (autosomal recessive, mild)); USP7 (Ubiquitin specific protease 7 (herpes virus-associated)); VASP (Vasodilator-stimulated phosphoprotein); VCAM1 (Vascular cell adhesion molecule 1); VDAC1 (Voltage-dependent anion channel 1); VDR (Vitamin D (1,25-dihydroxyvitamin D3) receptor); VHL (Von Hippel-Lindau syndrome); VMD2 (Vitelliform macular dystrophy (Best disease, bestrophin)); VPREB 1 (Pre-B lymphocyte gene 1 (non-standard provisional symbol)); VPREB2 (Pre-B lymphocyte-specific protein-2); VSD1 (Ventricular septal defect 1); VTN (Vitronectin (serum spreading factor, somatomedin B, complement S-protein)); VWF (Von Willebrand factor); WAS (Wiskott-Aldrich syndrome (ecezema-thrombocytopenia)); WEE1 (Wee1+ (S. pombe) homolog); WFS (Wolfram syndrome); WT1 (Wilms tumor 1); WT3 (Wilms tumor-3); WWS (Wieacker-Wolff syndrome); XBP1 (X-box binding protein 1); XG (Xg blood group (pseudoautosomal boundary-divided on the X chromosome)); XGR (Expression of XG and MIC2 on erythrocytes); XK (Kell blood group precursor (McLeod phenotype)); XPA (Xeroderma pigmentosum, complementation group A); XPC (Xeroderma pigmentosum, complementation group C); XPNPEPL (X-prolyl aminopeptidase (aminopeptidase P)-like); XRCC 1 (X-ray repair complementing defective repair in Chinese hamster cells 1); XRCC2 (X-ray repair complementing defective repair in Chinese hamster cells 2); XRCC3 (X-ray repair complementing defective repair in Chinese hamster cells 3); YB1 (Major histocompatibility complex, class II, Y box-binding protein I; DNA-binding protein B); ZFP161 (Zinc finger protein homologous to Zfp161 in mouse); ZFP36 (Zinc finger protein homologous to Zfp-36 in mouse); ZFY (Zinc finger protein, Y-linked); ZNF121 (Zinc finger protein 121 (clone ZHC32)); ZRK (Zona pellucida receptor tyrosine kinase, 95kD);

### Metabolism

ACAT1 (Acetyl-Coenzyme A acetyltransferase 1 (acetoacetyl Coenzyme A thiolase); ACAT2 (Acetyl-Coenzyme A acetyltransferase 2 (acetoacetyl Coenzyme A thiolase); ACATN (Acetyl-Coenzyme A transporter; BCAT1 (Branched chain aminotransferase 1, cytosolic; CRAT (Carnitine acetyltransferase; DIA4 (Diaphorase (NADH/NADPH) (cytochrome b-5 reductase); DUSP2 (Dual specificity phosphatase 2; EPHX1 (Epoxide hydrolase 1, microsomal (xenobiotic); EPHX2 (Epoxide hydrolase 2, cytoplasmic; GATM (Glycine amidinotransferase (L-arginine:glycine amidinotransferase); GJA4 (Gap junction protein, alpha 4, 37kD (connexin 37); HADHSC (L-3-hydroxyacyl-Coenzyme A dehydrogenase, short chain; HK1 (Hexokinase 1; HK3 (Hexokinase 3 (white cell); HMBS (Hydroxymethylbilane synthase; IARS (Isoleucine-tRNA synthetase; NAT1 (N-acetyltransferase 1 (arylamine N-acetyltransferase); OAT (Ornithine aminotransferase (gyrate atrophy); OTC (Ornithine carbamoyltransferase; PCCA (Propionyl Coenzyme A carboxylase, alpha polypeptide; PCCB (Propionyl Coenzyme A carboxylase, beta polypeptide; PDHA1 (Pyruvate dehydrogenase (lipoamide) alpha 1; QDPR (Quinoid dihydropteridine reductase; SGSH (N-sulfoglucosamine sulfohydrolase (sulfamidase); SHBG (Sex hormone-binding globulin; SHMT2 (Serine hydroxymethyltransferase 2 (mitochondrial); SLC7A1 (Solute carrier family 7 (cationic amino acid transporter, y+ system), member 1; SLC7A2 (Solute carrier family 7 (cationic amino acid transporter, y+ system), member 2; SLC7A4 (Solute carrier family 7 (cationic amino acid transporter, y+ system), member 4; SOAT1 (Sterol O-acyltransferase (acyl-Coenzyme A: cholesterol acyltransferase) 1; SOAT2 (Sterol O-acyltransferase 2; SORD (Sorbitol dehydrogenase; TPI1 (Triosephosphate isomerase 1; TYMS (Thymidylate synthetase; TYR (Tyrosinase (oculocutaneous albinism IA); TYRP1 (Tyrosinase-related protein 1; UGT2B17 (UDP glycosyltransferase 2 family, polypeptide B17; UGT2B7 (UDP glycosyltransferase 2 family, polypeptide B7; UGTREL1 (UDP-galactose transporter related;

### Metastasis

ACTG1 (Actin, gamma 1); ADD3 (Adducin 3 (gamma)); ALCAM (Activated leucocyte cell adhesion molecule); ANK1 (Ankyrin 1, erythrocytic); ANPEP (Alanyl (membrane) aminopeptidase (aminopeptidase N, aminopeptidase M, microsomal aminopeptidase, CD13, p150)); BTN (Butyrophilin); CD14 (CD14 antigen); CD19 (CD19 antigen); CD1D (CD1D antigen, d polypeptide); CD2 (CD2 antigen (p50), sheep red blood cell receptor); CD20 (CD20 antigen); CD22 (CD22 antigen); CD33 (CD33 antigen (gp67)); CD34 (CD34 antigen); CD37 (CD37 antigen); CD38 (CD38 antigen (p45)); CD39 (CD39 antigen); CD4 (CD4 antigen (p55)); CD44 (CD44 antigen (homing function and Indian blood group system)); CD47 (CD47 antigen (Rh-related antigen, integrin-associated signal transducer)); CD48 (CD48 antigen (B-cell membrane protein)); CD53 (CD53 antigen); CD58 (CD58 antigen, (lymphocyte function-associated antigen 3)); CD59 (CD59 antigen p18-20 (antigen identified by monoclonal antibodies 16.3A5, EJ16, EJ30, EL32 and G344)); CD63 (CD63 antigen (melanoma 1 antigen)); CD68 (CD68 antigen); CD7 (CD7 antigen (p41)); CD72 (CD72 antigen); CD8A (CD8 antigen, alpha polypeptide (p32)); CD9 (CD9 antigen (p24)); CD97 (CD97 antigen); CDH13 (Cadherin 13, H-cadherin (heart)); CDH17 (Cadherin 17, LI cadherin (liver-intestine)); CDH2 (Cadherin 2, N-cadherin (neuronal)); CDH4 (Cadherin 4, R-cadherin (retinal)); CDH5 (Cadherin 5, VE-cadherin (vascular epithelium)); CDW52 (CDW52 antigen (CAMPATH-1 antigen)); CLTB (Clathrin, light polypeptide (Lcb)); DAF (Decay accelerating factor for complement (CD55, Cromer blood group system)); DPP4 (Dipeptidylpeptidase IV (CD26, adenosine deaminase complexing protein 2)); ENG (Endoglin (Osler-Rendu-Weber syndrome 1)); F3 (Coagulation factor III (thromboplastin, tissue factor)); FAP (Fibroblast activation protein, alpha); FAT (FAT tumor suppressor (Drosophila) homolog); FLNA (Filamin A, alpha (actin-binding protein-280)); FN1 (Fibronectin 1); GJA4 (Gap junction protein, alpha 4, 37kD (connexin 37)); HMMR (Hyaluronan-mediated motility receptor (RHAMM)); ICAM1 (Intercellular adhesion molecule 1 (CD54), human rhinovirus receptor); ICAM2 (Intercellular adhesion molecule 2); ICAM3 (Intercellular adhesion molecule 3); ITGA2 (Integrin, alpha 2 (CD49B, alpha 2 subunit of VLA-2 receptor)); ITGA3 (Integrin, alpha 3 (antigen CD49C, alpha 3 subunit of VLA-3 receptor)); ITGA4 (Integrin, alpha 4 (antigen CD49D, alpha 4 subunit of VLA-4 receptor)); ITGA5 (Integrin, alpha 5 (fibronectin receptor, alpha polypeptide)); ITGA6 (Integrin, alpha 6); ITGA7 (Integrin, alpha 7); ITGA9 (Integrin, alpha 9); ITGAE (Integrin, alpha E (antigen CD103, human mucosal lymphocyte antigen 1; alpha polypeptide)); ITGAL (Integrin, alpha L (antigen CD11A (p180), lymphocyte function-associated antigen 1; alpha polypeptide)); ITGAM (Integrin, alpha M (complement component receptor 3, alpha; also known as CD11b (p170), macrophage antigen alpha polypeptide)); ITGAV (Integrin, alpha V (vitronectin receptor, alpha polypeptide, antigen CD51)); ITGAX (Integrin, alpha X (antigen CD11C (p150), alpha polypeptide)); ITGB1 (Integrin, beta 1 (fibronectin receptor, beta polypeptide, antigen CD29 includes MDF2, MSK12)); ITGB2 (Integrin, beta 2 (antigen CD18 (p95), lymphocyte function-associated antigen 1; macrophage antigen 1 (mac-1) beta subunit)); ITGB3 (Integrin, beta 3 (platelet glycoprotein IIIa, antigen CD61)); ITGB4 (Integrin, beta 4); ITGB5 (Integrin, beta 5); ITGB7 (Integrin, beta 7); ITGB8 (Integrin, beta 8); JAG1 (Jagged1 (Alagille syndrome)); JAG2 (Jagged 2); KAI1 (Kangai 1 (suppression of tumorigenicity 6, prostate; CD82 antigen (R2 leukocyte antigen, antigen detected by monoclonal and antibody IA4))); KPNB 1 (Karyopherin (importin) beta 1); LAG3 (Lymphocyte-activation gene 3); LY64 (Lymphocyte antigen 64 (mouse) homolog, radioprotective, 105kD); LYZ (Lysozyme (renal amyloidosis)); MAP1B (Microtubule-associated protein 1B); MDU1 (Antigen identified by monoclonal antibodies 4F2, TRA1.10, TROP4, and T43); MIC2 (Antigen identified by monoclonal antibodies 12E7, F21 and 013); MICA (MHC class I polypeptide-related sequence A); MME (Membrane metallo-endopeptidase (neutral endopeptidase, enkephalinase, CALLA, CD10)); MYL2 (Myosin, light polypeptide 2, regulatory, cardiac, slow); MYL3 (Myosin, light polypeptide 3, alkali; ventricular, skeletal, slow); NCAM1 (Neural cell adhesion molecule 1); NEO1 (Neogenin (chicken) homolog 1); NME3 (Non-metastatic cells 3, protein expressed in); PCDH1 (Protocadherin 1 (cadherin-like 1)); PDNP1 (Phosphodiesterase I/nucleotide pyrophosphatase 1 (homologous to mouse Ly-41 antigen)); PECAM1 (Platelet/endothelial cell adhesion molecule (CD31 antigen)); PKD1 (Polycystic kidney disease 1 (autosomal dominant)); PTCH (Patched (Drosophila) homolog); RSN (Restin (Reed-Steinberg cell-expressed intermediate filament-associated protein)); RTN1 (Reticulon 1); SDC1 (Syndecan 1); SDC2 (Syndecan 2 (heparan sulfate proteoglycan 1, cell surface-associated, fibroglycan)); SDC4 (Syndecan 4 (amphiglycan, ryudocan)); SELE (Selectin E (endothelial adhesion molecule 1)); SELL (Selectin L (lymphocyte adhesion molecule 1)); SELP (Selectin P (granule membrane protein 140kD, antigen CD62)); SELPLG (Selectin P ligand); SIAT1 (Sialyltransferase 1 (beta-galactoside alpha-2,6-sialytransferase)); SLAM (Signaling lymphocytic activation molecule); SPTA1 (Spectrin, alpha, erythrocytic 1 (elliptocytosis 2)); SPTB (Spectrin, beta, erythrocytic (includes sperocytosis, clinical type I)); ST2 (Suppression of tumorigenicity 2); TBCC (Tubulin-specific chaperone c); THBS1 (Thrombospondin 1); TM4SF2 (Transmembrane 4 superfamily member 2); TM4SF3 (Transmembrane 4 superfamily member 3); TNFSF4 (Tumor necrosis factor (ligand) superfamily, member 4 (tax-transcriptionally activated glycoprotein 1, 34kD)); TNFSF5 (Tumor necrosis factor (ligand) superfamily, member 5); TNFSF7 (Tumor necrosis factor (ligand) superfamily, member 7); TTN (Titin); TUBA1 (Tubulin, alpha 1 (testis specific)); TUBG (Tubulin, gamma polypeptide); VCAM1 (Vascular cell adhesion molecule 1); VCL (Vinculin); VIM (Vimentin);

### Miscellaneous

AREG (Amphiregulin (schwannoma-derived growth factor)); BCGF1 (B-cell growth factor 1 (12kD)); CTGF (Connective tissue growth factor); CTGF-L (Connective tissue growth factor-like protein); ECGF1 (Endothelial cell growth factor 1 (platelet-derived)); EGF (Epidermal growth factor); EPS 15 (Epidermal growth factor receptor pathway substrate 15); EPS8 (Epidermal growth factor receptor pathway substrate 8); FGF1 (Fibroblast growth factor 1 (acidic)); FGF10 (Fibroblast growth factor 10); FGF11 (Fibroblast growth factor 11); FGF12B (Fibroblast growth factor 12B); FGF13 (Fibroblast growth factor 13); FGF14 (Fibroblast growth factor 14); FGF16 (Fibroblast growth factor 16); FGF6 (Fibroblast growth factor 6); FGF7 (Fibroblast growth factor 7 (keratinocyte growth factor)); FGF8 (Fibroblast growth factor 8 (androgen-induced)); FGF9 (Fibroblast growth factor 9 (glia-activating factor)); FGFR1 (Fibroblast growth factor receptor 1 (fins-related tyrosine kinase 2, Pfeiffer syndrome)); FGFR2 (Fibroblast growth factor receptor 2 (bacteria-expressed kinase, keratinocyte growth factor receptor, craniofacial dysostosis 1, Crouzon syndrome, Pfeiffer syndrome, Jackson-Weiss syndrome)); FGFR3 (Fibroblast growth factor receptor 3 (achondroplasia, thanatophoric dwarfism)); FGFR4 (Fibroblast growth factor receptor 4); FIBP (Fibroblast growth factor (acidic) intracellular binding protein); FIGF (C-fos induced growth factor (vascular endothelial growth factor D)); HDGF (Hepatoma-derived growth factor (high-mobility group protein 1-like)); IGF1 (Insulin-like growth factor 1 (somatomedin C)); IGF2 (Insulin-like growth factor 2 (somatomedin A)); IGFALS (Insulin-like growth factor binding protein, acid labile subunit); NGFB (Nerve growth factor, beta polypeptide); PDGFA (Platelet-derived growth factor alpha polypeptide); PDGFRB (Platelet-derived growth factor receptor, beta polypeptide); PGF (Placental growth factor, vascular endothelial growth factor-related protein); PTN (Pleiotrophin (heparin binding growth factor 8, neurite growth-promoting factor 1)); QSCN6 (Quiescin Q6); TAK1 (Transforming growth factor beta-activated kinase 1); TIEG (TGFB inducible early growth response); TIEG2 (TGFB inducible early growth response 2); VEGF (Vascular endothelial growth factor); VEGFB (Vascular endothelial growth factor B); VGF (VGF nerve growth factor inducible); VTN (Vitronectin (serum spreading factor, somatomedin B, complement S-protein)); A2M (Alpha-2-macroglobulin); ADAM17 (A disintegrin and metalloproteinase domain 17 (tumor necrosis factor, alpha, converting enzyme)); ADSL (Adenylosuccinate lyase); AOAH (Acyloxyacyl hydrolase (neutrophil)); AOX1 (Aldehyde oxidase 1); ATP5J (ATP synthase, H+ transporting, mitochondrial F0 complex, subunit F6); BCAT2 (Branched chain aminotransferase 2, mitochondrial); BCKDHA (Branched chain keto acid dehydrogenase E1, alpha polypeptide (maple syrup urine disease)); BCL6 (B-cell CLL/lymphoma 6 (zinc finger protein 51)); BCL7 (B-cell CLL/lymphoma 7); BHMT (Betaine-homocysteine methyltransferase); BSG (Basigin); BTG1 (B-cell translocation gene 1, anti-proliferative); C5 (Complement component 5); CAD (Carbamoyl-phosphate synthetase 2, aspartate transcarbamylase, and dihydroorotase); CATR1 (CATR tumorigenicity conversion 1); CDR1 (Cerebellar degeneration-related protein (34kD)); CHGB (Chromogranin B (secretogranin 1)); CHIT1 (Chitinase 1); COL9A2 (Collagen, type IX, alpha 2); COX10 (Cytochrome c oxidase subunit X (heme A: farnesyltransferase)); COX11 (Cytochrome c oxidase subunit 11); COX15 (Cytochrome c oxidase subunit 15); COX17 (Human homolog of yeast mitochondrial copper recruitment gene); COX5A (Cytochrome c oxidase subunit Va); COX5B (Cytochrome c oxidase subunit Vb); COX6A1 (Cytochrome c oxidase subunit VIa polypeptide 1); COX6A2 (Cytochrome c oxidase subunit VIa polypeptide 2); COX6B (Cytochrome c oxidase subunit VIb); COX6C (Cytochrome c oxidase subunit VIc); COX7C (Cytochrome c oxidase subunit VIIc); COX7RP (Cytochrome c oxidase subunit VII-related protein); COX8 (Cytochrome c oxidase subunit VIII); CPA1 (Carboxypeptidase A1 (pancreatic)); CRHBP (Corticotropin releasing hormone-binding protein); CRIP2 (Cysteine-rich protein 2); CTH (Cystathionase (cystathionine gamma-lyase)); CTSB (Cathepsin B); CTSL (Cathepsin L); CYP (Clk-associating RS-cyclophilin); DARS (Aspartyl-tRNA synthetase); DEFA1 (Defensin, alpha 1, myeloid-related sequence); DSCAM (Down syndrome cell adhesion molecule); DUSP2 (Dual specificity phosphatase 2); DYT1 (Dystonia 1, torsion (autosomal dominant)); EDN1 (Endothelin 1); EGFL2 (EGF-like-domain, multiple 2); F5 (Coagulation factor V (proaccelerin, labile factor)); FMO1 (Flavin containing monooxygenase 1); FUS (Fusion, derived from t(12;16) malignant liposarcoma); FVT1 (Follicular lymphoma variant translocation 1); GARS (Glycyl-tRNA synthetase); GZMB (Granzyme B (granzyme 2, cytotoxic T-lymphocyte-associated serine esterase 1)); GZMK (Granzyme K (serine protease, granzyme 3; tryptase II)); HGFAC (HGF activator); HRMT1L2 (HMT1 (hnRNP methyltransferase, S. cerevisiae)-like 2); HSJ1 (Heat shock protein, neuronal DNAJ-like 1); HSJ2 (Heat shock protein, DNAJ-like 2); IDUA (Iduronidase, alpha-L-); IGFBP 10 (Insulin-like growth factor binding protein 10); LDHA (Lactate dehydrogenase A); LDHB (Lactate dehydrogenase B); LGALS 1 (Lectin, galactoside-binding, soluble, 1 (galectin 1)); LMO1 (LIM domain only 1 (rhombotin 1)); LNPEP (Leucyl/cystinyl aminopeptidase); LPP (LIM domain-containing preferred translocation partner in lipoma); MANA2 (Mannosidase, alpha type II); MAOA (Monoamine oxidase A); MFNG (Manic fringe (Drosophila) homolog); MMP-20 (Enamelysin); MMP10 (Matrix metalloproteinase 10 (stromelysin 2)); MMP19 (Matrix metalloproteinase 19); MMP8 (Matrix metalloproteinase 8 (neutrophil collagenase)); MT7SDNA (7S DNA); NB (Neuroblastoma (neuroblastoma suppressor)); NB4S (Neuroblastoma stage 4S gene); NPAT (Nuclear protein, ataxia-telangiectasia locus); OAS2 (2'-5'oligoadenylate synthetase 2); ORCTL3 (Organic cationic transporter-like 3); PDHA1 (Pyruvate dehydrogenase (lipoamide) alpha 1); PGAM1 (Phosphoglycerate mutase 1 (brain)); PITX2 (Paired-like homeodomain transcription factor 2); PRNP (Prion protein (p27-30) (Creutzfeld-Jakob disease, Gerstmann-Strausler-Scheinker syndrome, fatal familial insomnia)); PRSM1 (Protease, metallo, 1, 33kD); PTGS2 (Prostaglandin-endoperoxide synthase 2 (prostaglandin G/H synthase and cyclooxygenase)); RAD50 (RAD50 (S. cerevisiae) homolog); RAD51C (RAD51 (S. cerevisiae) homolog C); RCN2 (Reticulocalbin 2, EF-hand calcium binding domain); SET (SET translocation (myeloid leukemia-associated)); SIAT4A (Sialyltransferase 4A (beta-galactosidase alpha-2,3-sialytransferase)); SPARC (Secreted protein, acidic, cysteine-rich (osteonectin)); TAL1 (T-cell acute lymphocytic leukemia 1); TBG (Thyroxin-binding globulin); TFF1 (Trefoil factor 1 (breast cancer, estrogen-inducible sequence expressed in)); TIMP4 (Tissue inhibitor of metalloproteinase 4); TSC1 (Tuberous sclerosis 1); TSC2 (Tuberous sclerosis 2); UCP2 (Uncoupling protein 2 (mitochondrial, proton carrier)); UROS (Uroporphyrinogen III synthase (congenital erythropoietic porphyria)); VWF (Von Willebrand factor); ANT2 (Adenine nucleotide translocator 2 (fibroblast)); BSEP (Bile salt export pump (ABC member 16, MDR/TAP subfamily)); GJB1 (Gap junction protein, beta 1, 32kD (connexin 32, Charcot-Marie-Tooth neuropathy, X-linked)); KCNN3 (Potassium intermediate/small conductance calcium-activated channel, subfamily N, member 3); OCLN (Occludin); PGY3 (P glycoprotein 3/multiple drug resistance 3); SLC2A3 (Solute carrier family 2 (facilitated glucose transporter), member 3); SLC2A5 (Solute carrier family 2 (facilitated glucose transporter), member 5); TAP1 (Transporter 1, ABC (ATP binding cassette)); TAPBP (TAP binding protein (tapasin)); ARHGDIB (Rho GDP dissociation inhibitor (GDI) beta); ATRX (Alpha thalassemia/mental retardation syndrome X-linked); BMZF2 (Zinc finger 2, bone marrow); BMZF3 (Bone marrow zinc finger 3); BPI (Bactericidal/permeability-increasing protein); CD14 (CD14 antigen); EPB41 (Erythrocyte membrane protein band 4.1 (elliptocytosis 1, RH-linked)); EPB42 (Erythrocyte membrane protein band 4.2); GATA1 (GATA-binding protein 1 (globin transcription factor 1)); GATA2 (GATA-binding protein 2); GATA3 (GATA-binding protein 3); GATA4 (GATA-binding protein 4); GATA6 (GATA-binding protein 6); GZMA (Granzyme A (granzyme 1, cytotoxic T-lymphocyte-associated serine esterase 3)); HBA1 (Hemoglobin, alpha 1); HBB (Hemoglobin, beta); HBG1 (Hemoglobin, gamma A); HBZ (Hemoglobin, zeta); HCF2 (Heparin cofactor II); HHEX (Hematopoietically expressed homeobox); HK2 (Hexokinase 2); HP (Haptoglobin); HPS (Hermansky-Pudlak syndrome); HPX (Hemopexin); LAF4 (Lymphoid nuclear protein related to AF4); LCP 1 (Lymphocyte cytosolic protein 1 (L-plastin)); LRMP (Lymphoid-restricted membrane protein); MAL (Mal, T-cell differentiation protein); MLL (Myeloid/lymphoid or mixed-lineage leukemia (trithorax (Drosophila) homolog)); MLL2 (Myeloid/lymphoid or mixed-lineage leukemia 2); MLLT1 (Myeloid/lymphoid or mixed-lineage leukemia (trithorax (Drosophila) homolog); translocated to, 1); MLLT2 (Myeloid/lymphoid or mixed-lineage leukemia (trithorax (Drosophila) homolog); translocated to, 2); MLLT3 (Myeloid/lymphoid or mixed-lineage leukemia (trithorax (Drosophila) homolog); translocated to, 3); MLLT4 (Myeloid/lymphoid or mixed-lineage leukemia (trithorax (Drosophila) homolog); translocated to, 4); MLLT6 (Myeloid/lymphoid or mixed-lineage leukemia (trithorax (Drosophila) homolog); translocated to, 6); MLLT7 (Myeloid/lymphoid or mixed-lineage leukemia (trithorax (Drosophila) homolog); translocated to, 7); MPO (Myeloperoxidase); MYD88 (Myeloid differentiation primary response gene (88)); POU2F1 (POU domain, class 2, transcription factor 1); POU2F2 (POU domain, class 2, transcription factor 2); POU3F1 (POU domain, class 3, transcription factor 1); SCYC1 (Small inducible cytokine subfamily C, member 1 (lymphotactin)); TNFRSF17 (Tumor necrosis factor receptor superfamily, member 17); VWF (Von Willebrand factor);

### Pharmacology

ALDH1 (Aldehyde dehydrogenase 1, soluble); ALDH10 (Aldehyde dehydrogenase 10 (fatty aldehyde dehydrogenase)); ALDH2 (Aldehyde dehydrogenase 2, mitochondrial); ALDH3 (Aldehyde dehydrogenase 3); ALDH6 (Aldehyde dehydrogenase 6); ALDH7 (Aldehyde dehydrogenase 7); ALDH8 (Aldehyde dehydrogenase 8); ANT2 (Adenine nucleotide translocator 2 (fibroblast)); APEX (APEX nuclease (multifunctional DNA repair enzyme)); BCHE (Butyrylcholinesterase); BLMH (Bleomycin hydrolase); CAT (Catalase); CDA (Cytidine deaminase); CYP11A (Cytochrome P450, subfamily XIA (cholesterol side chain cleavage)); CYP11B1 (Cytochrome P450, subfamily XIB (steroid 11-beta-hydroxylase), polypeptide 1); CYP11B2 (Cytochrome P450, subfamily XIB (steroid 11-beta-hydroxylase), polypeptide 2); CYP17 (Cytochrome P450, subfamily XVII (steroid 17-alpha-hydroxylase), adrenal hyperplasia); CYP19 (Cytochrome P450, subfamily XIX (aromatization of androgens)); CYP1A1 (Cytochrome P450, subfamily I (aromatic compound-inducible), polypeptide 1); CYP1A2 (Cytochrome P450, subfamily I (aromatic compound-inducible), polypeptide 2); CYP1B1 (Cytochrome P450, subfamily I (dioxin-inducible), polypeptide 1 (glaucoma 3, primary infantile)); CYP21 (Cytochrome P450, subfamily XXI (steroid 21-hydroxylase, congenital adrenal hyperplasia)); CYP27A1 (Cytochrome P450, subfamily XXVIIA (steroid 27-hydroxylase, cerebrotendinous xanthomatosis), polypeptide 1); CYP2A6 (Cytochrome P450, subfamily IIA (phenobarbital-inducible), polypeptide 6); CYP2B (Cytochrome P450, subfamily IIB (phenobarbital-inducible)); CYP2B6 (Cytochrome P450, subfamily IIB (phenobarbital-inducible), polypeptide 6); CYP2C18 (Cytochrome P450, subfamily IIC (mephenytoin 4-hydroxylase), polypeptide 18); CYP2C19 (Cytochrome P450, subfamily IIC (mephenytoin 4-hydroxylase)); CYP2C9 (Cytochrome P450, subfamily IIC (mephenytoin 4-hydroxylase), polypeptide 9); CYP2D6 (Cytochrome P450, subfamily IID (debrisoquine, sparteine, etc., -metabolizing), polypeptide 6); CYP2E (Cytochrome P450, subfamily IIE (ethanol-inducible)); CYP2F1 (Cytochrome P450, subfamily IIF, polypeptide 1); CYP2J2 (Cytochrome P450, subfamily IIJ (arachidonic acid epoxygenase) polypeptide 2); CYP3A3 (Cytochrome P450, subfamily IIIA (niphedipine oxidase), polypeptide 3); CYP3A5 (Cytochrome P450, subfamily IIIA (niphedipine oxidase), polypeptide 5); CYP3A7 (Cytochrome P450, subfamily IIIA, polypeptide 7); CYP4A11 (Cytochrome P450, subfamily IVA, polypeptide 11); CYP4F3 (Cytochrome P450, subfamily IVF, polypeptide 3 (leukotriene B4 omega hydroxylase)); CYP51 (Cytochrome P450, 51 (lanosterol 14-alpha-demethylase)); DCK (Deoxycytidine kinase); DGUOK (Deoxyguanosine kinase); DHFR (Dihydrofolate reductase); DIA4 (Diaphorase (NADH/NADPH) (cytochrome b-5 reductase)); DPYD (Dihydropyrimidine dehydrogenase); EPHX1 (Epoxide hydrolase 1, microsomal (xenobiotic)); EPHX2 (Epoxide hydrolase 2, cytoplasmic); GJB1 (Gap junction protein, beta 1, 32kD (connexin 32, Charcot-Marie-Tooth neuropathy, X-linked)); GLRX (Glutaredoxin (thioltransferase)); GPX1 (Glutathione peroxidase 1); GPX2 (Glutathione peroxidase 2 (gastrointestinal)); GPX3 (Glutathione peroxidase 3 (plasma)); GPX4 (Glutathione peroxidase 4 (phospholipid hydroperoxidase)); GSR (Glutathione reductase); GSS (Glutathione synthetase); GSTA2 (Glutathione S-transferase A2); GSTA3 (Glutathione S-transferase A3); GSTM1 (Glutathione S-transferase M1); GSTM2 (Glutathione S-transferase M2 (muscle)); GSTM3 (Glutathione S-transferase M3 (brain)); GSTM4 (Glutathione S-transferase M4); GSTM5 (Glutathione S-transferase M5); GSTP1 (Glutathione S-transferase pi); GSTT1 (Glutathione S-transferase theta 1); GSTT2 (Glutathione S-transferase theta 2); GSTTLp28 (Glutathione-S-transferase like); GSTZ1 (Glutathione S-transferase Zeta 1 (maleylacetoacetate isomerase)); HPRT1 (Hypoxanthine phosphoribosyltransferase 1 (Lesch-Nyhan syndrome)); IMPDH1 (IMP (inosine monophosphate) dehydrogenase 1); IMPDH2 (IMP (inosine monophosphate) dehydrogenase 2); KCNN3 (Potassium intermediate/small conductance calcium-activated channel, subfamily N, member 3); MGST1 (Microsomal glutathione S-transferase 1); MGST2 (Microsomal glutathione S-transferase 2); MGST3 (Microsomal glutathione S-transferase 3); MRP1 (Multiple drug resistance protein 1); NAT1 (N-acetyltransferase 1 (arylamine N-acetyltransferase)); NMOR2 (NAD(P)H menadione oxidoreductase 2, dioxin-inducible); OCLN (Occludin); PGY1 (P glycoprotein 1/multiple drug resistance 1); PGY3 (P glycoprotein 3/multiple drug resistance 3); RRM1 (Ribonucleotide reductase M1 polypeptide); RRM2 (Ribonucleotide reductase M2 polypeptide); SLC2A3 (Solute carrier family 2 (facilitated glucose transporter), member 3); SLC2A5 (Solute carrier family 2 (facilitated glucose transporter), member 5); SOD1 (Superoxide dismutase 1, soluble (amyotrophic lateral sclerosis 1 (adult))); SOD2 (Superoxide dismutase 2, mitochondrial); SOD3 (Superoxide dismutase 3, extracellular); TAP1 (Transporter 1, ABC (ATP binding cassette)); TAPBP (TAP binding protein (tapasin)); TK1 (Thymidine kinase 1, soluble); TPMT (Thiopurine S-methyltransferase); TXNRD1 (Thioredoxin reductase 1); TYMS (Thymidylate synthetase); UGALT (UDP-galactose translocator); UGT2B 10 (UDP glycosyltransferase 2 family, polypeptide B10); UGT2B 15 (UDP glycosyltransferase 2 family, polypeptide B15); UGT2B4 (UDP glycosyltransferase 2 family, polypeptide B4); UGT2B7 (UDP glycosyltransferase 2 family, polypeptide B7); UGT8 (UDP glycosyltransferase 8 (UDP-galactose ceramide galactosyltransferase)); UMPS (Uridine monophosphate synthetase (orotate phosphoribosyl transferase and orotidine-5'-decarboxylase)); XDH (Xanthene dehydrogenase);

### Signal transduction

ABL1 (V-abl Abelson murine leukemia viral oncogene homolog 1); ABL2 (V-abl Abelson murine leukemia viral oncogene homolog 2 (arg, Abelson-related gene)); ABR (Active BCR-related gene); ACVR1B (Activin A receptor, type IB); ADK (Adenosine kinase); ADRBK1 (Adrenergic, beta, receptor kinase 1); AK1 (Adenylate kinase 1); AK2 (Adenylate kinase 2); AK3 (Adenylate kinase 3); AKT1 (V-akt murine thymoma viral oncogene homolog 1); AKT2 (V-akt murine thymoma viral oncogene homolog 2); ALOX12 (Arachidonate 12-lipoxygenase); ALOX5 (Arachidonate 5-lipoxygenase); ARAF1 (V-raf murine sarcoma 3611 viral oncogene homolog 1); ARHA (Ras homolog gene family, member A); ARHC (Ras homolog gene family, member C); ARHGDIB (Rho GDP dissociation inhibitor (GDI) beta); BLK (B lymphoid tyrosine kinase); BMPR2 (Bone morphogenetic protein receptor, type II (serine/threonine kinase)); BMX (BMX non-receptor tyrosine kinase); BRAF (V-raf murine sarcoma viral oncogene homolog B1); BTK (Bruton agammaglobulinemia tyrosine kinase); CAK (Cell adhesion kinase); CALM1 (Calmodulin 1 (phosphorylase kinase, delta)); CAMK4 (Calcium/calmodulin-dependent protein kinase IV); CBLB (Cas-Br-M (murine) ectropic retroviral transforming sequence b); CCNH (Cyclin H); CDC2L1 (Cell division cycle 2-like 1 (PITSLRE proteins)); CDC42 (Cell division cycle 42 (GTP-binding protein, 25kD)); CDC42 (Cell division cycle 42 (GTP-binding protein, 25kD)); CDC7L1 (CDC7 (cell division cycle 7, S. cerevisiae, homolog)-like 1); CDK2 (Cyclin-dependent kinase 2); CDK5 (Cyclin-dependent kinase 5); CDK6 (Cyclin-dependent kinase 6); CDK7 (Cyclin-dependent kinase 7 (homolog of Xenopus MO15 cdk-activating kinase)); CDK8 (Cyclin-dependent kinase 8); CDKN2B (Cyclin-dependent kinase inhibitor 2B (p15, inhibits CDK4)); CHEK1 (CHK1 (checkpoint, S.pombe) homolog); CHN1 (Chimerin (chimaerin) 1); CHN2 (Chimerin (chimaerin) 2); CHUK (Conserved helix-loop-helix ubiquitous kinase); CIS4 (STAT induced STAT inhibitor-4); CKS1 (CDC28 protein kinase 1); CKS2 (CDC28 protein kinase 2); CLGN (Calmegin); CLK2 (CDC-like kinase 2); CLK3 (CDC-like kinase 3); CNK (Cytokine-inducible kinase); COT (Cot (cancer Osaka thyroid) oncogene); CSK (C-src tyrosine kinase); CSNK1A1 (Casein kinase 1, alpha 1); CSNK1D (Casein kinase 1, delta); CSNK1E (Casein kinase 1, epsilon); CSNK2A2 (Casein kinase 2, alpha prime polypeptide); CSNK2B (Casein kinase 2, beta polypeptide); CTNNA1 (Catenin (cadherin-associated protein), alpha 1 (102kD)); CTNNB1 (Catenin (cadherin-associated protein), beta 1 (88kD)); CTNND2 (Catenin (cadherin-associated protein), delta 2 (neural plakophilin-related arm-repeat protein)); DGKA (Diacylglycerol kinase, alpha (80kD)); DGKG (Diacylglycerol kinase, gamma (90kD)); DGKQ (Diacylglycerol kinase, theta (110kD)); DMPK (Dystrophia myotonica-protein kinase); DRG2 (Developmentally regulated GTP-binding protein 2); DVL3 (Dishevelled 3 (homologous to Drosophila dsh)); DYRK1 (Dual-specificity tyrosine-(Y)-phosphorylation regulated kinase 1); DYRK1B (Dual-specificity tyrosine-(Y)-phosphorylation regulated kinase 1B); DYRK2 (Dual-specificity tyrosine-(Y)-phosphorylation regulated kinase 2); DYRK3 (Dual-specificity tyrosine-(Y)-phosphorylation regulated kinase 3); DYRK4 (Dual-specificity tyrosine-(Y)-phosphorylation regulated kinase 4); EFNA1 (Ephrin-A1); EFNA5 (Ephrin-A5); EFNB1 (Ephrin-B1); EFNB2 (Ephrin-B2); EGFR (Epidermal growth factor receptor (avian erythroblastic leukemia viral (v-erb-b) oncogene homolog)); EPHA2 (EphA2); EPHA4 (EphA4); EPHA5 (EphA5); EPHA7 (EphA7); EPHB3 (EphB3); EPHB4 (EphB4); EPHB6 (EphB6); ERBB2 (V-erb-b2 avian erythroblastic leukemia viral oncogene homolog 2 (neuro/glioblastoma derived oncogene homolog)); ERBB3 (V-erb-b2 avian erythroblastic leukemia viral oncogene homolog 3); ERBB4 (V-erb-a avian erythroblastic leukemia viral oncogene homolog-like 4); FBL (Fibrillarin); FER (Fer (fps/fes related) tyrosine kinase (phosphoprotein NCP94)); FES (Feline sarcoma (Snyder-Theilen) viral (v-fes)/Fujinami avian sarcoma (PRCII) viral (v-fps) oncogene homolog); FGFR1 (Fibroblast growth factor receptor 1 (fins-related tyrosine kinase 2, Pfeiffer syndrome)); FGFR2 (Fibroblast growth factor receptor 2 (bacteria-expressed kinase, keratinocyte growth factor receptor, craniofacial dysostosis 1, Crouzon syndrome, Pfeiffer syndrome, Jackson-Weiss syndrome)); FGR (Gardner-Rasheed feline sarcoma viral (v-fgr) oncogene homolog); FLT1 (Fms-related tyrosine kinase 1 (vascular endothelial growth factor/vascular permeability factor receptor)); FLT3LG (Fms-related tyrosine kinase 3 ligand); FLT4 (Fms-related tyrosine kinase 4); FRK (Fyn-related kinase); FYB (FYN-binding protein (FYB-120/130)); FYN (FYN oncogene related to SRC, FGR, YES); GLA (Galactosidase, alpha); GNAI1 (Guanine nucleotide binding protein (G protein), alpha inhibiting activity polypeptide 1); GNG10 (Guanine nucleotide binding protein 10); GPRK6 (G protein-coupled receptor kinase 6); GRB2 (Growth factor receptor-bound protein 2); GUCY1B3 (Guanylate cyclase 1, soluble, beta 3); HCK (Hemopoietic cell kinase); HIPK3 (Homeodomain-interacting protein kinase 3); HRK (Harakiri, BCL2-interacting protein (contains only BH3 domain)); ILK (Integrin-linked kinase); IRAK1 (Interleukin-1 receptor-associated kinase 1); IRAK2 (Interleukin-1 receptor-associated kinase 2); ITPKB (Inositol 1,4,5-trisphosphate 3-kinase B); JAK1 (Janus kinase 1 (a protein tyrosine kinase)); JAK2 (Janus kinase 2 (a protein tyrosine kinase)); JAK3 (Janus kinase 3 (a protein tyrosine kinase, leukocyte)); KDR (Kinase insert domain receptor (a type III receptor tyrosine kinase)); KIAA0641 (Apoptosis-associated tyrosine kinase); LCAT (Lecithin-cholesterol acyltransferase); LCK (Lymphocyte-specific protein tyrosine kinase); LIMK1 (LIM domain kinase 1); LIMK2 (LIM domain kinase 2); LTK (Leukocyte tyrosine kinase); LYN (V-yes-1 Yamaguchi sarcoma viral related oncogene homolog); MACS (Myristoylated alanine-rich protein kinase C substrate (MARCKS, 80K-L)); MADH1 (MAD (mothers against decapentaplegic, Drosophila) homolog 1); MADH2 (MAD (mothers against decapentaplegic, Drosophila) homolog 2); MADH3 (MAD (mothers against decapentaplegic, Drosophila) homolog 3); MAPKAPK2 (Mitogen-activated protein kinase-activated protein kinase 2); MAPKAPK5 (Mitogen-activated protein kinase-activated protein kinase 5); MATK (Megakaryocyte-associated tyrosine kinase); MEKK3 (MAP/ERK kinase kinase 3); MEKK4 (MAP/ERK kinase kinase 4); MEKK5 (MAP/ERK kinase kinase 5); MST1R (Macrophage stimulating 1 receptor (c-met-related tyrosine kinase)); NCK1 (NCK adaptor protein 1); NEK3 (NIMA (never in mitosis gene a)-related kinase); NME1 (Non-metastatic cells 1, protein (NM23A) expressed in); NME2 (Non-metastatic cells 2, protein (NM23B) expressed in); NMI (N-myc (and STAT) interactor); NPM1 (Nucleophosmin (nucleolar phosphoprotein B23, numatrin)); NTRK1 (Neurotrophic tyrosine kinase, receptor, type 1); NTRK2 (Neurotrophic tyrosine kinase, receptor, type 2); NTRK3 (Neurotrophic tyrosine kinase, receptor, type 3); NTRKR1 (Neurotrophic tyrosine kinase, receptor-related 1); NTRKR2 (Neurotrophic tyrosine kinase, receptor-related 2); NTRKR3 (Neurotrophic tyrosine kinase, receptor-related 3); PCTK3 (PCTAIRE protein kinase 3); PDE4B (Phosphodiesterase 4B, cAMP-specific (dunce (Drosophila)-homolog phosphodiesterase E4)); PDGFA (Platelet-derived growth factor alpha polypeptide); PDGFB (Platelet-derived growth factor beta polypeptide (simian sarcoma viral (v-sis) oncogene homolog)); PDGFRA (Platelet-derived growth factor receptor, alpha polypeptide); PDGFRB (Platelet-derived growth factor receptor, beta polypeptide); PDGFRL (Platelet-derived growth factor receptor-like); PDK2 (Pyruvate dehydrogenase kinase, isoenzyme 2); PDK4 (Pyruvate dehydrogenase kinase, isoenzyme 4); PDPK1 (3-phosphoinositide dependent protein kinase-1); PHKA2 (Phosphorylase kinase, alpha 2 (liver), glycogen storage disease IX); PHKG2 (Phosphorylase kinase, gamma 2 (testis)); PIASX-BETA (Protein inhibitor of activated STAT X); PIK3C3 (Phosphoinositide-3-kinase, class 3); PIK3CA (Phosphoinositide-3-kinase, catalytic, alpha polypeptide); PIK3CA (Phosphoinositide-3-kinase, catalytic, alpha polypeptide); PIK3CG (Phosphoinositide-3-kinase, catalytic, gamma polypeptide); PIM1 (Pim-1 oncogene); PLA2G2A (Phospholipase A2, group IIA (platelets, synovial fluid)); PLCB4 (Phospholipase C, beta 4); PLCE (Phospholipase C, epsilon); PLCG2 (Phospholipase C, gamma 2 (phosphatidylinositol-specific)); PPP2R5A (Protein phosphatase 2, regulatory subunit B (B56), alpha isoform); PPP2R5B (Protein phosphatase 2, regulatory subunit B (B56), beta isoform); PPP2R5C (Protein phosphatase 2, regulatory subunit B (B56), gamma isoform); PPP2R5D (Protein phosphatase 2, regulatory subunit B (B56), delta isoform); PPP2R5E (Protein phosphatase 2, regulatory subunit B (B56), epsilon isoform); PRKAA2 (Protein kinase, AMP-activated, alpha 2 catalytic subunit); PRKACA (Protein kinase, cAMP-dependent, catalytic, alpha); PRKACB (Protein kinase, cAMP-dependent, catalytic, beta); PRKACG (Protein kinase, cAMP-dependent, catalytic, gamma); PRKAG1 (Protein kinase, AMP-activated, gamma 1 non-catalytic subunit); PRKAR1A (Protein kinase, cAMP-dependent, regulatory, type I, alpha (tissue specific extinguisher 1)); PRKAR1B (Protein kinase, cAMP-dependent, regulatory, type I, beta); PRKAR2B (Protein kinase, cAMP-dependent, regulatory, type II, beta); PRKCA (Protein kinase C, alpha); PRKCB 1 (Protein kinase C, beta 1); PRKCG (Protein kinase C, gamma); PRKCI (Protein kinase C, iota); PRKCL1 (Protein kinase C-like 1); PRKCL2 (Protein kinase C-like 2); PRKCM (Protein kinase C, mu); PRKCQ (Protein kinase C, theta); PRKCZ (Protein kinase C, zeta); PRKG1 (Protein kinase, cGMP-dependent, type I); PRKG2 (Protein kinase, cGMP-dependent, type II); PRKM1 (protein kinase, mitogen-activated 1 (MAP kinase 1; p40, p41)); PRKM 10 (Protein kinase mitogen-activated 10 (MAP kinase)); PRKM 11 (Protein kinase mitogen-activated 11); PRKM13 (Protein kinase mitogen- activated 13); PRKM3 (Protein kinase, mitogen-activated 3 (MAP kinase 3; p44)); PRKM4 (Protein kinase, mitogen-activated 4 (MAP kinase 4; p63)); PRKM6 (Protein kinase, mitogen-activated 6 (extracellular signal-regulated kinase, p97)); PRKM7 (Protein kinase mitogen-activated 7 (MAP kinase)); PRKM8 (Protein kinase mitogen-activated 8 (MAP kinase)); PRKM9 (Protein kinase mitogen-activated 9 (MAP kinase)); PRKMK1 (Protein kinase, mitogen-activated, kinase 1 (MAP kinase kinase 1)); PRKMK1 (Protein kinase, mitogen-activated, kinase 1 (MAP kinase kinase 1)); PRKMK2 (Protein kinase, mitogen-activated, kinase 2, p45 (MAP kinase kinase 2)); PRKMK3 (Protein kinase, mitogen-activated, kinase 3 (MAP kinase kinase 3)); PRKMK5 (Protein kinase, mitogen-activated, kinase 5 (MAP kinase kinase 5)); PRKMK7 (Protein kinase, mitogen-activated, kinase 7 (MAP kinase kinase 7)); PRKR (Protein kinase, interferon-inducible double stranded RNA dependent); PTK2 (PTK2 protein tyrosine kinase 2); PTK2B (Protein tyrosine kinase 2 beta); PTK7 (PTK7 protein tyrosine kinase 7); PTK9 (Protein tyrosine kinase 9); RAB8IP (Rab8 interacting protein (GC kinase)); RASA1 (RAS p21 protein activator (GTPase activating protein) 1); RET (Ret proto-oncogene (multiple endocrine neoplasia MEN2A, MEN2B and medullary thyroid carcinoma 1, Hirschsprung disease)); RGS1 (Regulator of G-protein signalling 1); RGS16 (Regulator of G-protein signalling 16); RGS7 (Regulator of G-protein signalling 7); RHO7 (GTP-binding protein Rho7); RHOK (Rhodopsin kinase); ROCK1 (Rho-associated, coiled-coil containing protein kinase 1); RPS6KA2 (Ribosomal protein S6 kinase, 90kD, polypeptide 2); RPS6KB1 (Ribosomal protein S6 kinase, 70kD, polypeptide 1); RYK (RYK receptor-like tyrosine kinase); SAPK3 (Stress-activated protein kinase 3); SERK1 (SAPK/Erk kinase 1); SFN (Stratifin); SH2D1A (SH2 domain protein 1A, Duncan's disease (lymphoproliferative syndrome)); SH3D1B (SH3 domain protein 1B); SRC (V-src avian sarcoma (Schmidt-Ruppin A-2) viral oncogene homolog); SRPK2 (SFRS protein kinase 2); SSI-1 (JAK binding protein); SSI-3 (STAT induced STAT inhibitor 3); STAT1 (Signal transducer and activator of transcription 1, 91kD); STAT2 (Signal transducer and activator of transcription 2, 113kD); STAT3 (Signal transducer and activator of transcription 3 (acute-phase response factor)); STAT4 (Signal transducer and activator of transcription 4); STAT5A (Signal transducer and activator of transcription 5A); STAT6 (Signal transducer and activator of transcription 6, interleukin-4 induced); STATI2 (STAT induced STAT inhibitor-2); STK11 (Serine/threonine kinase 11 (Peutz-Jeghers syndrome)); STK15 (Serine/threonine kinase 15); STK17A (Serine/threonine kinase 17a (apoptosis-inducing)); STK17B (Serine/threonine kinase 17b (apoptosis-inducing)); STK2 (Serine/threonine kinase 2); STK3 (Serine/threonine kinase 3 (Ste20, yeast homolog)); STK4 (Serine/threonine kinase 4); SUB 1.5 (Guanine nucleotide exchange factor; 115-kD; mouse Lsc homolog); SYK (Spleen tyrosine kinase); TESK1 (Testis-specific kinase 1); TIAM1 (T-cell lymphoma invasion and metastasis 1); TK2 (Thymidine kinase 2, mitochondrial); TNFAIP1 (Tumor necrosis factor, alpha-induced protein 1 (endothelial)); TRK (Oncogene TRK); TRK1 (TRNA lysine 1); TTK (TTK protein kinase); TXK (TXK tyrosine kinase); TYK2 (Tyrosine kinase 2); TYRO3 (TYRO3 protein tyrosine kinase); TYROBP (TYRO protein tyrosine kinase binding protein); UBE1L (Ubiquitin-activating enzyme E1, like); UBE2A (Ubiquitin-conjugating enzyme E2A (RAD6 homolog)); UBE2B (Ubiquitin-conjugating enzyme E2B (RAD6 homolog)); VASP (Vasodilator-stimulated phosphoprotein); VAV2 (Vav 2 oncogene); VRK2 (Vaccinia related kinase 2); WAS (Wiskott-Aldrich syndrome (ecezema-thrombocytopenia)); YES 1 (V-yes-1 Yamaguchi sarcoma viral oncogene homolog 1); YESP (V-yes-1 Yamaguchi sarcoma viral oncogene homolog pseudogene); ZAP70 (Zeta-chain (TCR) associated protein kinase (70 kD)); ZPK (Zipper (leucine) protein kinase);

### Transcription

ADA (Adenosine deaminase); ADPRT (ADP-ribosyltransferase (NAD+; poly (ADP-ribose) polymerase)); ADSS (Adenylosuccinate synthase); AHR (Aryl hydrocarbon receptor); ATBF1 (AT-binding transcription factor 1); ATF1 (Activating transcription factor 1); ATF3 (Activating transcription factor 3); ATF4 (Activating transcription factor 4 (tax-responsive enhancer element B67)); BARD1 (BRCA1 associated RING domain 1); BCL6 (B-cell CLL/lymphoma 6 (zinc finger protein 51)); BMZF2 (Zinc finger 2, bone marrow); BMZF3 (Bone marrow zinc finger 3); CBF2 (CCAAT-box-binding transcription factor); CBFA2 (Core-binding factor, runt domain, alpha subunit 2 (acute myeloid leukemia 1; aml1 oncogene)); CBFA3 (Core-binding factor, runt domain, alpha subunit 3); CBFB (Core-binding factor, beta subunit); CEBPA (CCAAT/enhancer binding protein (C/EBP), alpha); CEBPB (CCAAT/enhancer binding protein (C/EBP), beta); CEBPD (CCAAT/enhancer binding protein (C/EBP), delta); CEBPE (CCAAT/enhancer binding protein (C/EBP), epsilon); CEBPG (CCAAT/enhancer binding protein (C/EBP), gamma); CENPE (Centromere protein E (312kD)); CHD 1 (Chromodomain helicase DNA binding protein 1); CHD2 (Chromodomain helicase DNA binding protein 2); CHD3 (Chromodomain helicase DNA binding protein 3); CHD4 (Chromodomain helicase DNA binding protein 4); CREB2 (CAMP responsive element binding protein 2); CREBBP (CREB binding protein (Rubinstein-Taybi syndrome)); CSE1L (Chromosome segregation 1 (yeast homolog)-like); CSTF3 (Cleavage stimulation factor, 3' pre-RNA, subunit 3, 77kD); CTPS (CTP synthase); DCK (Deoxycytidine kinase); DCTD (DCMP deaminase); DDIT3 (DNA-damage-inducible transcript 3); DGUOK (Deoxyguanosine kinase); DR1 (Down-regulator of transcription 1, TBP-binding (negative cofactor 2)); DUT (DUTP pyrophosphatase); E2F1 (E2F transcription factor 1); E2F2 (E2F transcription factor 2); E2F5 (E2F transcription factor 5, p130-binding); EGR1 (Early growth response 1); EIF3S6 (Eukaryotic translation initiation factor 3, subunit 6 (48kD)); ELF 1 (E74-like factor 1 (ets domain transcription factor)); ELF3 (E74-like factor 3 (ets domain transcription factor)); ELK4 (ELK4, ETS-domain protein (SRF accessory protein 1) NOTE: Symbol and name provisional); EP300 (E1A binding protein p300); ERV3 (Endogenous retroviral sequence 3 (includes zinc finger protein H-plk/HPF9)); ESR1 (Estrogen receptor 1); ETS2 (V-ets avian erythroblastosis virus E26 oncogene homolog 2); ETV1 (Ets variant gene 1); ETV3 (Ets variant gene 3); ETV4 (Ets variant gene 4 (E1A enhancer-binding protein, E1AF)); ETV5 (Ets variant gene 5 (ets-related molecule)); EZF (Endothelial Kruppel-like zinc finger protein); FKHR (Forkhead (Drosophila) homolog 1 (rhabdomyosarcoma)); FLI1 (Friend leukemia virus integration 1); FSRG1 (Female sterile homeotic-related gene 1 (mouse homolog)); GART (Phosphoribosylglycinamide formyltransferase, phosphoribosylglycinamide synthetase, phosphoribosylaminoimidazole synthetase); GATA1 (GATA-binding protein 1 (globin transcription factor 1)); GATA2 (GATA-binding protein 2); GATA3 (GATA-binding protein 3); GATA4 (GATA-binding protein 4); GLI (Glioma-associated oncogene homolog (zinc finger protein)); GRSF1 (G-rich RNA sequence binding factor 1); GTF2H2 (General transcription factor IIH, polypeptide 2 (44kD subunit)); H4FI (H4 histone family, member I); HHEX (Hematopoietically expressed homeobox); HIF1A (Hypoxia-inducible factor 1, alpha subunit (basic helix-loop-helix transcription factor)); HIVEP1 (Human immunodeficiency virus type I enhancer-binding protein 1); HLF (Hepatic leukemia factor); HMG17 (High-mobility group (nonhistone chromosomal) protein 17); HMG2 (High-mobility group (nonhistone chromosomal) protein 2); HNRPK (Heterogeneous nuclear ribonucleoprotein K); HZF2 (Zinc finger (C2H2)); ICSBP1 (Interferon consensus sequence binding protein 1); ID1 (Inhibitor of DNA binding 1, dominant negative helix-loop-helix protein); ID2 (Inhibitor of DNA binding 2, dominant negative helix-loop-helix protein); ID3 (Inhibitor of DNA binding 3, dominant negative helix-loop-helix protein); ID4 (Inhibitor of DNA binding 4, dominant negative helix-loop-helix protein); IRF1 (Interferon regulatory factor 1); IRF2 (Interferon regulatory factor 2); IRF4 (Interferon regulatory factor 4); IRF5 (Interferon regulatory factor 5); IRF7 (Interferon regulatory factor 7); JUN (V-jun avian sarcoma virus 17 oncogene homolog); JUND (Jun D proto-oncogene); KIAA0646 (C3HC4-type zinc finger protein); LAF4 (Lymphoid nuclear protein related to AF4); LKLF (Lung Kruppel-like zinc finger transcription factor); LYF1 (Zinc finger protein, subfamily 1A, 1 (Ikaros)); LYL1 (Lymphoblastic leukemia derived sequence 1); MAFG (V-maf musculoaponeurotic fibrosarcoma (avian) oncogene family, protein G); MAX (MAX protein); MAZ (MYC-associated zinc finger protein (purine-binding transcription factor)); MBLL (C3H-type zinc finger protein; similar to D. melanogaster muscleblind B protein); MDM2 (Mouse double minute 2, human homolog of; p53-binding protein); MHC2TA (MHC class II transactivator); MKI67 (Antigen identified by monoclonal antibody Ki-67); MNDA (Myeloid cell nuclear differentiation antigen); MSX1 (Msh (Drosophila) homeo box homolog 1 (formerly homeo box 7)); MTHFD (5,10-methylenetetrahydrofolate dehydrogenase, 5,10-methylenetetrahydrofolate cyclohydrolase, 10-formyltetrahydrofolate synthetase); MYC (V-myc avian myelocytomatosis viral oncogene homolog); NCBP (Nuclear cap binding protein, 80kD); NCBP (Nuclear cap binding protein, 80kD); NDP52 (Nuclear domain 10 protein); NFE2 (Nuclear factor (erythroid-derived 2), 45kD); NFKB1 (Nuclear factor of kappa light polypeptide gene enhancer in B-cells 1 (p105)); NFKB2 (Nuclear factor of kappa light polypeptide gene enhancer in B-cells 2 (p49/p100)); NFYA (Nuclear transcription factor Y, alpha); NP (Nucleoside phosphorylase); NURR1 (Nuclear receptor related 1 (transcriptionally inducible)); ODC 1 (Ornithine decarboxylase 1); PAX3 (Paired box gene 3 (Waardenburg syndrome 1)); PBX1 (Pre-B-cell leukemia transcription factor 1); PBX3 (Pre-B-cell leukemia transcription factor 3); PCNA (Proliferating cell nuclear antigen); PEX6 (Peroxisomal biogenesis factor 6); PITX2 (Paired-like homeodomain transcription factor 2); PML (Promyelocytic leukemia); POU1F1 (POU domain, class 1, transcription factor 1 (Pit1, growth hormone factor 1)); POU2AF1 (POU domain, class 2, associating factor 1); POU2F1 (POU domain, class 2, transcription factor 1); POU2F2 (POU domain, class 2, transcription factor 2); POU3F1 (POU domain, class 3, transcription factor 1); POU3F2 (POU domain, class 3, transcription factor 2); POU5F1 (POU domain, class 5, transcription factor 1); PPAT (Phosphoribosyl pyrophosphate amidotransferase); PROP1 (Prophet of Pit1, paired-like homeodomain transcription factor); PRPS1 (Phosphoribosyl pyrophosphate synthetase 1); PTB (Polypyrimidine tract binding protein (heterogeneous nuclear ribonucleoprotein I)); RANBP2 (RAN binding protein 2); RARB (Retinoic acid receptor, beta); RARG (Retinoic acid receptor, gamma); RECQL (RecQ protein-like (DNA helicase Q1-like)); RENBP (Renin-binding protein); RFX1 (Regulatory factor X, 1 (influences HLA class II expression)); RFX2 (Regulatory factor X, 2 (influences HLA class II expression)); RFX3 (Regulatory factor X, 3 (influences HLA class II expression)); RFX4 (Regulatory factor X, 4 (influences HLA class II expression)); RFX5 (Regulatory factor X, 5 (influences HLA class II expression)); RFXAP (Regulatory factor X-associated protein); RXRA (Retinoid X receptor, alpha); SATB 1 (Special AT-rich sequence binding protein 1 (binds to nuclear matrix/scaffold-associating DNA's)); SFRS7 (Splicing factor, arginine/serine-rich 7 (35kD)); SKIL (SKI-like); SLUG (Slug (chicken homolog), zinc finger protein); SMARCA2 (SWI/SNF related, matrix associated, actin dependent regulator of chromatin, subfamily a, member 2); SON (SON DNA binding protein); SOX4 (SRY (sex determining region Y)-box 4); SP100 (Nuclear antigen Sp100); SPIB (Spi-B transcription factor (Spi-1/PU.1 related)); SRF (Serum response factor (c-fos serum response element-binding transcription factor)); STAT3 (Signal transducer and activator of transcription 3 (acute-phase response factor)); STAT4 (Signal transducer and activator of transcription 4); STAT5A (Signal transducer and activator of transcription 5A); TAF2C2 (TATA box binding protein (TBP)-associated factor, RNA polymerase II, C2, 105kD); TAL2 (T-cell acute lymphocytic leukemia 2); TCEB1L (Transcription elongation factor B (SIII), polypeptide 1-like); TCF1 (Transcription factor 1, hepatic; LF-B1, hepatic nuclear factor (HNF1), albumin proximal factor); TCF12 (Transcription factor 12 (HTF4, helix-loop-helix transcription factors 4)); TCF3 (Transcription factor 3 (E2A immunoglobulin enhancer binding factors E12/E47)); TCF7 (Transcription factor 7 (T-cell specific, HMG-box)); TCF8 (Transcription factor 8 (represses interleukin 2 expression)); TCF9 (Transcription factor 9 (binds GC-rich sequences)); TEGT (Testis enhanced gene transcript); TFAP2B (Transcription factor AP-2 beta (activating enhancer-binding protein 2 beta)); TFAP4 (Transcription factor AP-4 (activating enhancer-binding protein 4)); TFDP2 (Transcription factor Dp-2 (E2F dimerization partner 2)); THRA (Thyroid hormone receptor, alpha (avian erythroblastic leukemia viral (v-erb-a) oncogene homolog)); TIAL1 (TIA1 cytotoxic granule-associated RNA-binding protein-like 1); TK1 (Thymidine kinase 1, soluble); TOP1 (Topoisomerase (DNA) I); TOP2B (Topoisomerase (DNA) II beta (180kD)); TP53 (Tumor protein p53 (Li-Fraumeni syndrome)); UBL1 (Ubiquitin-like 1 (sentrin)); WT1 (Wilms tumor 1); WT1 (Wilms tumor 1); XPO1 (Exportin 1 (CRM1, yeast, homolog)); ZFP103 (Zinc finger protein homologous to Zfp103 in mouse); ZFP36 (Zinc finger protein homologous to Zfp-36 in mouse); ZFP37 (Zinc finger protein homologous to Zfp37 in mouse); ZFP93 (Zinc finger protein); ZFX (Zinc finger protein, X-linked); ZFY (Zinc finger protein, Y-linked); ZK1 (Kruppel-type zinc finger (C2H2)); ZNF10 (Zinc finger protein 10 (KOX 1)); ZNF121 (Zinc finger protein 121 (clone ZHC32)); ZNF124 (Zinc finger protein 124 (HZF-16)); ZNF127 (Zinc finger protein 127); ZNF131 (Zinc finger protein 131 (clone pHZ-10)); ZNF132 (Zinc finger protein 132 (clone pHZ-12)); ZNF133 (Zinc finger protein 133 (clone pHZ-13)); ZNF134 (Zinc finger protein 134 (clone pHZ-15)); ZNF135 (Zinc finger protein 135 (clone pHZ-17)); ZNF136 (Zinc finger protein 136 (clone pHZ-20)); ZNF137 (Zinc finger protein 137 (clone pHZ-30)); ZNF139 (Zinc finger protein 139 (clone pHZ-37)); ZNF140 (Zinc finger protein 140 (clone pHZ-39)); ZNF141 (Zinc finger protein 141 (clone pHZ-44)); ZNF142 (Zinc finger protein 142 (clone pHZ-49)); ZNF143 (Zinc finger protein 143 (clone pHZ-1)); ZNF144 (Zinc finger protein 144 (Mel-18)); ZNF145 (Zinc finger protein 145 (Kruppel-like, expressed in promyelocytic leukemia)); ZNF147 (Zinc finger protein 147 (estrogen-responsive finger protein)); ZNF148 (Zinc finger protein 148 (pHZ-52)); ZNF151 (Zinc finger protein 151 (pHZ-67)); ZNF 154 (Zinc finger protein 154 (pHZ-92)); ZNF155 (Zinc finger protein 155 (pHZ-96)); ZNF157 (Zinc finger protein 157 (HZF22)); ZNF162 (Zinc finger protein 162); ZNF165 (Zinc finger protein 165); ZNF169 (Zinc finger protein 169); ZNF173 (Zinc finger protein 173); ZNF177 (Zinc finger protein 177); ZNF189 (Zinc finger protein 189); ZNF198 (Zinc finger protein 198); ZNF2 (Zinc finger protein 2); ZNF20 (Zinc finger protein, C2H2, rapidly turned over); ZNF200 (Zinc finger protein 200); ZNF202 (Zinc finger protein 202); ZNF204 (Zinc finger protein 204); ZNF205 (Zinc finger protein 205); ZNF206 (Zinc finger protein 206); ZNF207 (Zinc finger protein 207); ZNF239 (Zinc finger protein 239); ZNF259 (Zinc finger protein 259); ZNF261 (Zinc finger protein 261); ZNF262 (Zinc finger protein 262); ZNF263 (Zinc finger protein 263); ZNF264 (Zinc finger protein 264); ZNF3 (Zinc finger protein 3 (A8-51)); ZNF35 (Zinc finger protein 35 (clone HF.10)); ZNF37A (Zinc finger protein 37a (KOX 21)); ZNF42 (Zinc finger protein 42 (myeloid-specific retinoic acid- responsive)); ZNF44 (Zinc finger protein 44 (KOX 7)); ZNF45 (Zinc finger protein 45 (a Kruppel-associated box (KRAB) domain polypeptide)); ZNF6 (Zinc finger protein 6 (CMPX1)); ZNF7 (Zinc finger protein 7 (KOX 4, clone HF.16)); ZNF74 (Zinc finger protein 74 (Cos52)); ZNF76 (Zinc finger protein 76 (expressed in testis)); ZNF8 (Zinc finger protein 8 (clone HF.18)); ZNF84 (Zinc finger protein 84 (HPF2)); ZNF85 (Zinc finger protein 85); ZNF9 (Zinc finger protein 9 (a cellular retroviral nucleic acid binding protein)); ZNF91 (Zinc finger protein 91 (HPF7, HTF10));

### Tumor Suppressor/Oncogenes

ABL1 (V-abl Abelson murine leukemia viral oncogene homolog 1); ABL2 (V-abl Abelson murine leukemia viral oncogene homolog 2 (arg, Abelson-related gene)); AKT1 (V-akt murine thymoma viral oncogene homolog 1); AKT2 (V-akt murine thymoma viral oncogene homolog 2); APC (Adenomatosis polyposis coli); ARAF1 (V-raf murine sarcoma 3611 viral oncogene homolog 1); ARAF2 (V-raf murine sarcoma 3611 viral oncogene homolog 2); ARHA (Ras homolog gene family, member A); ARHB (Ras homolog gene family, member B); ARHC (Ras homolog gene family, member C); AXL (AXL receptor tyrosine kinase); BCL2 (B-cell CLL/lymphoma 2); BCL3 (B-cell CLL/lymphoma 3); BCR (Breakpoint cluster region); BLYM (Avian lymphoma virus-derived transforming sequence); BMI1 (Murine leukemia viral (bmi-1) oncogene homolog); BRAF (V-raf murine sarcoma viral oncogene homolog B1); BRAFP (V-raf murine sarcoma viral oncogene homolog B1 pseudogene); CBFA2 (Core-binding factor, runt domain, alpha subunit 2 (acute myeloid leukemia 1; aml1 oncogene)); CBL (Cas-Br-M (murine) ecotropic retroviral transforming sequence); CCND1 (Cyclin D1 (PRAD1: parathyroid adenomatosis 1)); CDH1 (Cadherin 1, E-cadherin (epithelial)); CDK4 (Cyclin-dependent kinase 4); CDKN1A (Cyclin-dependent kinase inhibitor 1A (p21, Cip1)); CDKN1C (Cyclin-dependent kinase inhibitor 1C (p57, Kip2)); CDKN2A (Cyclin-dependent kinase inhibitor 2A (melanoma, p16, inhibits CDK4)); CHES1 (Checkpoint suppressor 1); COT (Cot (cancer Osaka thyroid) oncogene); CRK (V-crk avian sarcoma virus CT10 oncogene homolog); CRKL (V-crk avian sarcoma virus CT10 oncogene homolog-like); CSF1R (Colony stimulating factor 1 receptor, formerly McDonough feline sarcoma viral (v-fms) oncogene homolog); D10S170 (DNA segment, single copy, probe pH4 (transforming sequence, thyroid-1,); DCC (Deleted in colorectal carcinoma); DDX6 (DEAD/H (Asp-Glu-Ala-Asp/His) box polypeptide 6 (RNA helicase, 54kD)); E2F1 (E2F transcription factor 1); ECT2 (Epithelial cell transforming sequence 2 oncogene); EGFR (Epidermal growth factor receptor (avian erythroblastic leukemia viral (v-erb-b) oncogene homolog)); EIF3S6 (Eukaryotic translation initiation factor 3, subunit 6 (48kD)); ELE1 (RET-activating gene ELE1); ELK1 (ELK1, member of ETS oncogene family); ELK2P1 (ELK2, member of ETS oncogene family, pseudogene 1); ELK3 (ELK3, ETS-domain protein (SRF accessory protein 2) NOTE: Symbol and name provisional.); EMP1 (Epithelial membrane protein 1); EMS 1 (Ems 1 sequence (mammary tumor and squamous cell carcinoma-associated (p80/85 src substrate)); EPHA1 (EphA1); EPHA3 (EphA3); ERBA2L (V-erb-a avian erythroblastic leukemia viral oncogene homolog 2-like); ERBAL2 (V-erb-a avian erythroblastic leukemia viral oncogene homolog-like 2); ERBB2 (V-erb-b2 avian erythroblastic leukemia viral oncogene homolog 2 (neuro/glioblastoma derived oncogene homolog)); ERBB3 (V-erb-b2 avian erythroblastic leukemia viral oncogene homolog 3); ERBB4 (V-erb-a avian erythroblastic leukemia viral oncogene homolog-like 4); ERG (V-ets avian erythroblastosis virus E26 oncogene related); ETS1 (V-ets avian erythroblastosis virus E26 oncogene homolog 1); ETS2 (V-ets avian erythroblastosis virus E26 oncogene homolog 2); ETV1 (Ets variant gene 1); ETV3 (Ets variant gene 3); ETV6 (Ets variant gene 6 (TEL oncogene)); EVI1 (Ecotropic viral integration site 1); EWSR1 (Ewing sarcoma breakpoint region 1); FAT (FAT tumor suppressor (Drosophila) homolog); FER (Fer (fps/fes related) tyrosine kinase (phosphoprotein NCP94)); FES (Feline sarcoma (Snyder-Theilen) viral (v-fes)/Fujinami avian sarcoma (PRCII) viral (v-fps) oncogene homolog); FGF3 (Fibroblast growth factor 3 (murine mammary tumor virus integration site (v-int-2) oncogene homolog)); FGF4 (Fibroblast growth factor 4 (heparin secretory transforming protein 1, Kaposi sarcoma oncogene)); FGF6 (Fibroblast growth factor 6); FGR (Gardner-Rasheed feline sarcoma viral (v-fgr) oncogene homolog); FKHL1 (Forkhead (Drosophila)-like 1); FLI1 (Friend leukemia virus integration 1); FLT 1 (Fms-related tyrosine kinase 1 (vascular endothelial growth factor/vascular permeability factor receptor)); FOS (V-fos FBJ murine osteosarcoma viral oncogene homolog); FOSB (FBJ murine osteosarcoma viral oncogene homolog B); FOSL1 (FOS-like antigen-1); FOSL2 (FOS-like antigen 2); FYN (FYN oncogene related to SRC, FGR, YES); GLI (Glioma-associated oncogene homolog (zinc finger protein)); GLI2 (GLI-Kruppel family member GLI2); GLI3 (GLI-Kruppel family member GLI3 (Greig cephalopolysyndactyly syndrome)); GRF2 (Guanine nucleotide-releasing factor 2 (specific for crk proto-oncogene)); GRO1 (GRO1 oncogene (melanoma growth stimulating activity, alpha)); GRO2 (GRO2 oncogene); GRO3 (GRO3 oncogene); HCK (Hemopoietic cell kinase); HKR3 (GLI-Kruppel family member HKR3); HRAS (V-Ha-ras Harvey rat sarcoma viral oncogene homolog); HRASP (V-Ha-ras Harvey rat sarcoma viral oncogene homolog pseudogene); INT6P1 (Murine mammary tumor integration site 6 (oncogene homolog) pseudogene 1); IRF4 (Interferon regulatory factor 4); JUN (V-jun avian sarcoma virus 17 oncogene homolog); JUNB (Jun B proto-oncogene); JUND (Jun D proto-oncogene); KAI1 (Kangai 1 (suppression of tumorigenicity 6, prostate; CD82 antigen (R2 leukocyte antigen, antigen detected by monoclonal and antibody IA4))); KIT (V-kit Hardy-Zuckerman 4 feline sarcoma viral oncogene homolog); KRAS1P (V-Ki-ras1 Kirsten rat sarcoma 1 viral oncogene homolog, processed pseudogene); KRAS2 (V-Ki-ras2 Kirsten rat sarcoma 2 viral oncogene homolog); LBC (Lymphoid blast crisis oncogene); LCK (Lymphocyte-specific protein tyrosine kinase); LCN2 (Lipocalin 2 (oncogene 24p3)); LCO (Liver cancer oncogene); LPSA (Oncogene liposarcoma (DNA segment, single copy, expressed, probes); LTA (Lymphotoxin alpha (TNF superfamily, member 1)); LTB (Lymphotoxin beta (TNF superfamily, member 3)); LYN (V-yes-1 Yamaguchi sarcoma viral related oncogene homolog); M1S1 (Membrane component, chromosome 1, surface marker 1 (40kD glycoprotein, identified by monoclonal antibody GA733)); M4S 1 (Membrane component, chromosomal 4, surface marker (35kD glycoprotein)); MADH4 (MAD (mothers against decapentaplegic, Drosophila) homolog 4); MAF (V-maf musculoaponeurotic fibrosarcoma (avian) oncogene homolog); MAFG (V-maf musculoaponeurotic fibrosarcoma (avian) oncogene family, protein G); MAFK (V-maf avian musculoaponeurotic fibrosarcoma oncogene family, protein K); MAS1 (MAS1 oncogene); MAX (MAX protein); MCC (Mutated in colorectal cancers); MCF2 (MCF.2 cell line derived transforming sequence); MDM2 (Mouse double minute 2, human homolog of; p53-binding protein); MEL (Mel transforming oncogene (derived from cell line NK14)- RAB8 homolog); MELL1 (Mel transforming oncogene-like 1); MET (Met proto-oncogene (hepatocyte growth factor receptor)); MLH1 (MutL (E. coli) homolog 1 (colon cancer, nonpolyposis type 2)); MOS (V-mos Moloney murine sarcoma viral oncogene homolog); MPL (Myeloproliferative leukemia virus oncogene); MSH2 (MutS (E. coli) homolog 2 (colon cancer, nonpolyposis type 1)); MUM1 (Multiple myeloma oncogene 1); MYB (V-myb avian myeloblastosis viral oncogene homolog); MYBL1 (V-myb avian myeloblastosis viral oncogene homolog-like 1); MYBL2 (V-myb avian myeloblastosis viral oncogene homolog-like 2); MYC (V-myc avian myelocytomatosis viral oncogene homolog); MYCL1 (V-myc avian myelocytomatosis viral oncogene homolog 1, lung carcinoma derived); MYCL2 (V-myc avian myelocytomatosis viral oncogene homolog 2); MYCLK1 (V-myc avian myelocytomatosis viral oncogene homolog-like 1); MYCN (V-myc avian myelocytomatosis viral related oncogene, neuroblastoma derived); MYCP (V-myc avian myelocytomatosis viral oncogene homolog pseudogene); NBL1 (Neuroblastoma candidate region, suppression of tumorigenicity 1); NF1 (Neurofibromin 1 (neurofibromatosis, von Recklinghausen disease, Watson disease)); NF2 (Neurofibromin 2 (bilateral acoustic neuroma)); NFKB2 (Nuclear factor of kappa light polypeptide gene enhancer in B-cells 2 (p49/p100)); NKTR (Natural killer-tumor recognition sequence); NOTCH4 (Notch (Drosophila) homolog 4); NOV (Nephroblastoma overexpressed gene); NRAS (Neuroblastoma RAS viral (v-ras) oncogene homolog); NRASL1 (Neuroblastoma RAS viral (v-ras) oncogene homolog-like 1); NRASL2 (Neuroblastoma RAS viral (v-ras) oncogene homolog-like 2); NRASL3 (Neuroblastoma RAS viral (v-ras) oncogene homolog-like 3); NTRK1 (Neurotrophic tyrosine kinase, receptor, type 1); OVC (Oncogene OVC (ovarian adenocarcinoma oncogene)); PACE (Paired basic amino acid cleaving enzyme (furin, membrane associated receptor protein)); PDGFB (Platelet-derived growth factor beta polypeptide (simian sarcoma viral (v-sis) oncogene homolog)); PIM1 (Pim-1 oncogene); PVT1 (Pvt-1 (murine) oncogene homolog, MYC activator); RAB1 (RAB 1, member RAS oncogene family); RAB11A (RAB 11A, member oncogene family); RAB 11 A (RAB11A, member RAS oncogene family); RAB 13 (RAB 13, member RAS oncogene family); RAB2 (RAB2, member RAS oncogene family); RAB27A (RAB27A, member RAS oncogene family); RAB27B (RAB27B, member RAS oncogene family); RAB2L (RAB2, member RAS oncogene family-like); RAB3A (RAB3A, member RAS oncogene family); RAB3B (RAB3B, member RAS oncogene family); RAB4 (RAB4, member RAS oncogene family); RAB5A (RAB5A, member RAS oncogene family); RAB5B (RAB5B, member RAS oncogene family); RAB6 (RAB6, member RAS oncogene family); RAB7L1 (RAB7, member RAS oncogene family-like 1); RABL (RAB, member of RAS oncogene family-like); RAF1 (V-raf-1 murine leukemia viral oncogene homolog 1); RAF1P1 (V-raf-1 murine leukemia viral oncogene homolog 1 pseudogene 1); RALA (V-ral simian leukemia viral oncogene homolog A (ras related)); RALB (V-ral simian leukemia viral oncogene homolog B (ras related; GTP binding protein)); RAN (RAN, member RAS oncogene family); RAP1A (RAP1A, member of RAS oncogene family); RAP1AP (RAP1A, member of RAS oncogene family pseudogene); RAP1B (RAP1B, member of RAS oncogene family); RAP2A (RAP2A, member of RAS oncogene family); RAP2B (RAP2B, member of RAS oncogene family); RB1 (Retinoblastoma 1 (including osteosarcoma)); REL (V-rel avian reticuloendotheliosis viral oncogene homolog); RELA (V-rel avian reticuloendotheliosis viral oncogene homolog A (nuclear factor of kappa light polypeptide gene enhancer in B-cells 3 (p65))); RELB (V-rel avian reticuloendotheliosis viral oncogene homolog B (nuclear factor of kappa light polypeptide gene enhancer in B-cells 3)); RET (Ret proto-oncogene (multiple endocrine neoplasia MEN2A, MEN2B and medullary thyroid carcinoma 1, Hirschsprung disease)); ROS 1 (V-ros avian UR2 sarcoma virus oncogene homolog 1); RRAS (Related RAS viral (r-ras) oncogene homolog); SEA (S13 avian erythroblastosis oncogene homolog); SKI (V-ski avian sarcoma viral oncogene homolog); SMARCB1 (SWI/SNF related, matrix associated, actin dependent regulator of chromatin, subfamily b, member 1); SPI1 (Spleen focus forming virus (SFFV) proviral integration oncogene spi1); SPINK1 (Serine protease inhibitor, Kazal type 1); SRC (V-src avian sarcoma (Schmidt-Ruppin A-2) viral oncogene homolog); ST5 (Suppression of tumorigenicity 5); SUPT3H (Suppressor of Ty (S.cerevisiae) 3 homolog); SUPT5H (Suppressor of Ty (S.cerevisiae) 5 homolog); SUPT6H (Suppressor of Ty (S.cerevisiae) 6 homolog); TAL1 (T-cell acute lymphocytic leukemia 1); TGFBR2 (Transforming growth factor, beta receptor II (70-80kD)); THPO (Thrombopoietin (myeloproliferative leukemia virus oncogene ligand, megakaryocyte growth and development factor)); THRA (Thyroid hormone receptor, alpha (avian erythroblastic leukemia viral (v-erb-a) oncogene homolog)); THRB (Thyroid hormone receptor, beta (avian erythroblastic leukemia viral (v-erb-a) oncogene homolog 2)); TIAM1 (T-cell lymphoma invasion and metastasis 1); TIM (Oncogene TIM); TM4SF1 (Transmembrane 4 superfamily member 1); TNF (Tumor necrosis factor (TNF superfamily, member 2)); TP53BP2 (Tumor protein p53-binding protein, 2); TP73 (Tumor protein p73); TPR (Translocated promoter region (to activated MET oncogene)); TRE17 (Tre-2 oncogene); USP4 (Ubiquitin specific protease 4 (proto-oncogene)); USP6 (Ubiquitin specific protease 6 (Tre-2 oncogene)); VAV1 (Vav 1 oncogene); VAV2 (Vav 2 oncogene); VHL (Von Hippel-Lindau syndrome); WNT1 (Wingless-type MMTV integration site family, member 1); WNT2 (Wingless-type MMTV integration site family member 2); WNT5A (Wingless-type MMTV integration site family, member 5A); WT1 (Wilms tumor 1); YES1 (V-yes-1 Yamaguchi sarcoma viral oncogene homolog 1); YESP (V-yes-1 Yamaguchi sarcoma viral oncogene homolog pseudogene); AMPHL (Amphiphysin-like); APC (Adenomatosis polyposis coli); ARHA (Ras homolog gene family, member A); ARHB (Ras homolog gene family, member B); ARHC (Ras homolog gene family, member C); AXL (AXL receptor tyrosine kinase); BCL2 (B-cell CLL/lymphoma 2); BCL3 (B-cell CLL/lymphoma 3); BCR (Breakpoint cluster region); BLYM (Avian lymphoma virus-derived transforming sequence); BRCA1 (Breast cancer 1, early onset); BRCA2 (Breast cancer 2, early onset); CBL (Cas-Br-M (murine) ecotropic retroviral transforming sequence); CCND 1 (Cyclin D 1 (PRAD1: parathyroid adenomatosis 1)); CDH1 (Cadherin 1, E-cadherin (epithelial)); CDK4 (Cyclin-dependent kinase 4); CDKN1A (Cyclin-dependent kinase inhibitor 1A (p21, Cip1)); CDKN1C (Cyclin-dependent kinase inhibitor 1C (p57, Kip2)); CDKN2A (Cyclin-dependent kinase inhibitor 2A (melanoma, p16, inhibits CDK4)); CDKN2B (Cyclin-dependent kinase inhibitor 2B (p15, inhibits CDK4)); CHES 1 (Checkpoint suppressor 1); D10S170 (DNA segment, single copy, probe pH4 (transforming sequence, thyroid-1,); DCC (Deleted in colorectal carcinoma); DDX6 (DEAD/H (Asp-Glu-Ala-Asp/His) box polypeptide 6 (RNA helicase, 54kD)); E2F1 (E2F transcription factor 1); EIF3S6 (Eukaryotic translation initiation factor 3, subunit 6 (48kD)); ELE1 (RET-activating gene ELE1); ELK3 (ELK3, ETS-domain protein (SRF accessory protein 2) NOTE: Symbol and name provisional.); EMP 1 (Epithelial membrane protein 1); EMS 1 (Ems1 sequence (mammary tumor and squamous cell carcinoma-associated (p80/85 src substrate)); EPHA1 (EphA1); EPHA3 (EphA3); ETV3 (Ets variant gene 3); EVI1 (Ecotropic viral integration site 1); EWSR1 (Ewing sarcoma breakpoint region 1); FAT (FAT tumor suppressor (Drosophila) homolog); FER (Fer (fps/fes related) tyrosine kinase (phosphoprotein NCP94)); FHIT (Fragile histidine triad gene); FKHL1 (Forkhead (Drosophila)-like 1); FLT1 (Fms-related tyrosine kinase 1 (vascular endothelial growth factor/vascular permeability factor receptor)); FOSL1 (FOS-like antigen-1); FOSL2 (FOS-like antigen 2); GLI2 (GLI-Kruppel family member GLI2); GLI3 (GLI-Kruppel family member GLI3 (Greig cephalopolysyndactyly syndrome)); HCK (Hemopoietic cell kinase); HKR3 (GLI-Kruppel family member HKR3); ING1 (Inhibitor of growth 1); IRF4 (Interferon regulatory factor 4); KAI1 (Kangai 1 (suppression of tumorigenicity 6, prostate; CD82 antigen (R2 leukocyte antigen, antigen detected by monoclonal and antibody IA4))); LCK (Lymphocyte-specific protein tyrosine kinase); LTA (Lymphotoxin alpha (TNF superfamily, member 1)); LTB (Lymphotoxin beta (TNF superfamily, member 3)); M1S1 (Membrane component, chromosome 1, surface marker 1 (40kD glycoprotein, identified by monoclonal antibody GA733)); M4S 1 (Membrane component, chromosomal 4, surface marker (35kD glycoprotein)); MADH4 (MAD (mothers against decapentaplegic, Drosophila) homolog 4); MAX (MAX protein); MCC (Mutated in colorectal cancers); MEN1 (Multiple endocrine neoplasia I); MLH1 (MutL (E. coli) homolog 1 (colon cancer, nonpolyposis type 2)); MSH2 (MutS (E. coli) homolog 2 (colon cancer, nonpolyposis type 1)); NBL1 (Neuroblastoma candidate region, suppression of tumorigenicity 1); NF1 (Neurofibromin 1 (neurofibromatosis, von Recklinghausen disease, Watson disease)); NF2 (Neurofibromin 2 (bilateral acoustic neuroma)); NFKB2 (Nuclear factor of kappa light polypeptide gene enhancer in B-cells 2 (p49/p100)); NKTR (Natural killer-tumor recognition sequence); NME1 (Non-metastatic cells 1, protein (NM23A) expressed in); NOV (Nephroblastoma overexpressed gene); NTRK1 (Neurotrophic tyrosine kinase, receptor, type 1); PDGFRL (Platelet-derived growth factor receptor-like); PLA2G2A (Phospholipase A2, group IIA (platelets, synovial fluid)); PTCH (Patched (Drosophila) homolog); PTEN (Phosphatase and tensin homolog (mutated in multiple advanced cancers 1)); RB1 (Retinoblastoma 1 (including osteosarcoma)); SMARCB1 (SWI/SNF related, matrix associated, actin dependent regulator of chromatin, subfamily b, member 1); SPINK1 (Serine protease inhibitor, Kazal type 1); ST5 (Suppression of tumorigenicity 5); SUPT3H (Suppressor of Ty (S.cerevisiae) 3 homolog); SUPT5H (Suppressor of Ty (S.cerevisiae) 5 homolog); SUPT6H (Suppressor of Ty (S.cerevisiae) 6 homolog); TAL1 (T-cell acute lymphocytic leukemia 1); TGFBR2 (Transforming growth factor, beta receptor II (70-80kD)); TIAM1 (T-cell lymphoma invasion and metastasis 1); TIM (Oncogene TIM); TM4SF1 (Transmembrane 4 superfamily member 1); TNF (Tumor necrosis factor (TNF superfamily, member 2)); TP53 (Tumor protein p53 (Li-Fraumeni syndrome)); TP53BP2 (Tumor protein p53-binding protein, 2); TP73 (Tumor protein p73); VHL (Von Hippel-Lindau syndrome); WNT1 (Wingless-type MMTV integration site family, member 1); WNT2 (Wingless-type MMTV integration site family member 2); WNT5A (Wingless-type MMTV integration site family, member 5A); WT1 (Wilms tumor 1)

## Claims

1. A method for determining the biological effect and/or activity of at least one drug, chemical substance and/or pharmaceutical composition, comprising the steps of:
(a) providing a biological sample A containing DNA from at least one individual, tissue, cell or other biological material containing DNA, which was exposed to said at least one drug, chemical substance and/or pharmaceutical composition;
(b) providing a biological sample B containing DNA from at least one individual, tissue, cell or other biological material containing DNA, which was not exposed to said at least one drug, chemical substance or pharmaceutical composition;
(c) analysing the level of cytosine methylation at chosen sites of the DNA contained in the samples A and B;
(d) selecting the sites which are differentially methylated between the DNA in samples A and B,
whereby a knowledge base is generated; and
(e) concluding from the said knowledge base on the biological effect and/or activity of said at least one drug, chemical substance or pharmaceutical composition.

2. Method according to claim 1, comprising that the biological sample is provided by means of a biopsy, by means of an operation on an individual, by means of a dissection, derived from a preserved biological sample, collected from body fluid(s) and/or collected directly from the environment.

3. Method according to claim 1 or 2, **characterised in that** the biological sample comprises a eukaryotic and/or prokaryotic cell line, a biopsy sample, blood, sputum, faeces, urine, cerebral liquid, tissue embedded in paraffin, tissue derived from eyes, intestine, brain, heart, prostate, kidney, lung, breast or liver, histological samples or a combination thereof.

4. Method according to any of claims 1 to 3, **characterised in that** said biological sample is provided from biological material of healthy and/or diseased individuals.

5. Method according to any of claims 1 to 4, **characterised in that** the biological samples A and B are provided from the identical individual, tissue, cell or other biological material.

6. Method according claim 5, **characterised in that** the biological samples A and B that are provided have been taken before, during and/or after onset of a treatment with said drug, chemical substance or pharmaceutical composition.

7. Method according to any of claims 1 to 6, further comprising the step of isolating DNA from the said samples before analysing the level of cytosine methylation at chosen sites in said isolated DNA.

8. Method according to claim 7, **characterised in that** the isolation of said DNA contained in said biological sample comprises isolating subcellular compartments, organelles, macromolecular structures and multiprotein complexes, partial or complete preparation of the DNA and/or mRNA, reverse transcription or partial digestion of the material with an enzyme selected from proteases, RNAses and/or DNAses or combinations thereof.

9. Method according to any of claims 1 to 8, **characterised in that** the analysis of the level of cytosine methylation comprises chemical treatment with bisulphite, hydrogen sulphite or disulphite, polymerase chain reaction (PCR), hybridisation analyses, sequencing, mass spectrometry and fluorescent, enzymatic, radioactive, dye and/or antibody labelling.

10. Method according to any of claims 1 to 9, **characterised in that** all potential methylation sites of the DNA are analysed.

11. Method according to any of claims 1 to 10, **characterised in that** the level of at least two cytosine methylation sites is analysed in parallel.

12. Method according to claim 11, **characterised in that** the level of at least 100 cytosine methylation sites is analysed in parallel.

13. Method according to any of claims 1 to 12, **characterised in that** the methylation sites are located in methylation relevant regions of the DNA comprising complete genes and/or promoters, introns, first exons and/or enhancers.

14. Method according to any of claims 1 to 13, **characterised in that** the methylation sites are located in methylation relevant regions of genes related with unwanted side effects of medicaments, cancers, dysfunctions, damages or diseases of the central nervous system (CNS), aggressive symptoms or behavioural disorders, clinical, psychological and social consequences of brain injuries, psychotic disorders and disorders of the personality, dementia and/or associates syndromes, cardiovascular diseases, malfunctions or damages, diseases, malfunctions or damages of the gastrointestine, diseases, malfunctions or damages of the respiratory system, injury, inflammation, infection, immunity and/or reconvalescence, diseases, malfunctions or damages as consequences of modifications in the developmental process, diseases, malfunctions or damages of the skin, muscles, connective tissue or bones, endocrine or metabolic diseases, malfunctions or damages, headache, and sexual malfunctions or combinations thereof.

15. Method according to claim 14, **characterised in that** the methylation sites are located in methylation relevant regions of genes related with leukaemia, head and neck cancer, Hodgkin's disease, gastric cancer, prostate cancer, renal cancer, bladder cancer, breast cancer, Burkitt's lymphoma, Wilms tumour, Prader-Willi/Angelman syndrome, ICF syndrome, dermatofibroma, hypertension, paediatric neurobiological diseases, autism, ulcerative colitis, fragile X syndrome, and Huntington's disease.

16. Method according to any of claims 1 to 15, wherein said analysed methylation sites are disease specific and/or personalised.

17. Method according to any of claims 1 to 16, **characterised in that** the selection is based on the result of at least two individual rows of analyses.

18. Method according to any of claims 1 to 17, **characterised in that** the selection is at least partially performed automatically by means of a suited automate, such as a computer device.

19. Method according to any of claims 1 to 18, **characterised in that** at least two sites are selected in parallel.

20. Method according to claim 19, **characterised in that** at least 100 sites are selected in parallel.

21. Method according to any of claims 1 to 20, **characterised in that** all or a part of the sites of the knowledge base are used for the conclusion.

22. Method according to any of claims 1 to 21, **characterised in that** additional information about the biological sample is used for the conclusion.

23. Method according to any of claims 1 to 22, **characterised in that** the conclusion is based on the result of at least two individual rows of analyses.

24. The method according to any of claims 1 to 23, **characterized in that** the conclusion is performed by a computer system.

25. Method according to any of claims 1 to 24, **characterised in that** steps a) to d) are repeated.

26. Method according to any of claims 1 to 25, **characterised in that** the identical biological sample, different biological samples or a combination thereof is used in steps a) and/or b).

27. Method according to any of claims 1 to 24, **characterised in that** steps c) to d) are repeated.

28. Method according to any of claims 1 to 27, **characterised in that** said method is repeated for at least 5 to 50 times.

29. Method according to any of claims 1 to 28, **characterised in that** said method is at least partially performed by means of a suited automate, for example a robot and/or a computer system.

30. Use of a method according to any of claims 1 to 29 for determining at least one drug, chemical substance and/or pharmaceutical composition that is biologically effective and/or active.

31. Use according to claim 30, wherein said at least one drug, chemical substance and/or pharmaceutical composition is biologically effective and/or active in the treatment of unwanted side effects of medicaments, cancers, dysfunctions, damages or diseases of the central nervous system (CNS), aggressive symptoms or behavioural disorders, clinical, psychological and social consequences of brain injuries, psychotic disorders and disorders of the personality, dementia and/or associates syndromes, cardiovascular diseases, malfunctions or damages, diseases, malfunctions or damages of the gastrointestine, diseases, malfunctions or damages of the respiratory system, injury, inflammation, infection, immunity and/or reconvalescence, diseases, malfunctions or damages as consequences of modifications in the developmental process, diseases, malfunctions or damages of the skin, muscles, connective tissue or bones, endocrine or metabolic diseases, malfunctions or damages, headache, and sexual malfunctions or combinations thereof.

32. Use according to claim 31, wherein said at least one drug, chemical substance and/or pharmaceutical composition is biologically effective and/or active in the treatment of leukaemia, head and neck cancer, Hodgkin's disease, gastric cancer, prostate cancer, renal cancer, bladder cancer, breast cancer, Burkitt's lymphoma, Wilms tumour, Prader-Willi/Angelman syndrome, ICF syndrome, dermatofibroma, hypertension, paediatric neurobiological diseases, autism, ulcerative colitis, fragile X syndrome, and Huntington's disease.
